# EUROPEAN PATENT APPLICATION

(11) **EP 4 653 462 A2**
(43) Date of publication of application: **26.11.2025**
(21) Application number: 25192429.6
(22) Date of filing: 21.08.2017
(51) Int. Cl.: C07K 16/28

(54) **ANTI-PD-1 ANTIBODIES, OR FRAGMENTS THEREOF, FOR TREATING HEPATITIS B**

(30) Priority: 22.08.2016 US 201662377953 P
(62) Divisional of application: 17844217.4
(71) Applicant: Arbutus Biopharma Corporation, Burnaby, BC V5J 5J8 (CA)
(72) Inventor: MOORE, Christopher Brooks, Burnaby, British Columbia V5J 5J8 (CA); PARK, Jang-June, Burnaby, British Columbia V5J 5J8 (CA)
(74) Representative: Müller-Boré & Partner Patentanwälte PartG mbB

(57) **Abstract**

Certain embodiments of the invention provide a method for treating a Hepatitis B virus infection and/or ameliorating one or more symptoms associated with a Hepatitis B virus infection in a mammal, the method comprising the step of administering to the mammal a therapeutically effective amount of an anti-PD-1 antibody, or fragment thereof.

## Description

### CROSS-REFERENCE TO RELATED APPLICATION

This patent application claims the benefit of priority of U.S. application serial No. 62/377,953, filed August 22, 2016, which application is herein incorporated by reference.

### BACKGROUND OF THE INVENTION

Hepatitis B virus (abbreviated as "HBV") is a member of the Hepadnavirus family. The virus particle (sometimes referred to as a virion) includes an outer lipid envelope and an icosahedral nucleocapsid core composed of protein. The nucleocapsid encloses the viral DNA and a DNA polymerase that has reverse transcriptase activity. The outer envelope contains embedded proteins that are involved in viral binding of, and entry into, susceptible cells, typically liver hepatocytes. In addition to the infectious viral particles, filamentous and spherical bodies lacking a core can be found in the serum of infected individuals. These particles are not infectious and are composed of the lipid and protein that forms part of the surface of the virion, which is called the surface antigen (HBsAg), and is produced in excess during the life cycle of the virus.

The genome of HBV is made of circular DNA, but it is unusual because the DNA is not fully double-stranded. One end of the full length strand is linked to the viral DNA polymerase. The genome is 3020-3320 nucleotides long (for the full-length strand) and 1700-2800 nucleotides long (for the shorter strand). The negative-sense (non-coding) is complementary to the viral mRNA. The viral DNA is found in the nucleus soon after infection of the cell. There are four known genes encoded by the genome, called C, X, P, and S. The core protein is coded for by gene C (HBcAg), and its start codon is preceded by an upstream in-frame AUG start codon from which the pre-core protein is produced. HBeAg is produced by proteolytic processing of the pre-core protein. The DNA polymerase is encoded by gene P. Gene S is the gene that codes for the surface antigen (HBsAg). The HBsAg gene is one long open reading frame but contains three in frame "start" (ATG) codons that divide the gene into three sections, pre-S 1, pre-S2, and S. Because of the multiple start codons, polypeptides of three different sizes called large, middle, and small are produced. The function of the protein coded for by gene X is not fully understood but it is associated with the development of liver cancer. Replication of HBV is a complex process. Although replication takes place in the liver, the virus spreads to the blood where viral proteins and antibodies against them are found in infected people. The structure, replication and biology of HBV is reviewed in D. Glebe and C.M.Bremer, Seminars in Liver Disease, Vol. 33, No. 2, pages 103-112 (2013).

Infection of humans with HBV can cause an infectious inflammatory illness of the liver. Infected individuals may not exhibit symptoms for many years. It is estimated that about a third of the world population has been infected at one point in their lives, including 350 million who are chronic carriers.

The virus is transmitted by exposure to infectious blood or body fluids. Perinatal infection can also be a major route of infection. The acute illness causes liver inflammation, vomiting, jaundice, and possibly death. Chronic hepatitis B may eventually cause cirrhosis and liver cancer.

Although most people who are infected with HBV clear the infection through the action of their immune system, some infected people suffer an aggressive course of infection (fulminant hepatitis); while others are chronically infected thereby increasing their chance of liver disease. Several medications are currently approved for treatment of HBV infection, but infected individuals respond with various degrees of success to these medications, and none of these medications clear the virus from the infected person.

Thus, there is a continuing need for compositions and methods for the treatment of HBV infection in animals (e.g. humans).

### SUMMARY OF THE INVENTION

Certain embodiments of the present invention provide a method for treating a Hepatitis B virus infection in a mammal (e.g., a human) in need thereof, comprising administering to the mammal a therapeutically effective amount of an anti-PD-1 antibody, or a fragment thereof.

The invention also provides an anti-PD-1 antibody for the prophylactic or therapeutic treatment of a Hepatitis B virus infection.

The invention provides the use of an anti-PD-1 antibody to prepare a medicament for the treatment of a Hepatitis B virus infection.

The invention provides a pharmaceutical composition for use in the treatment of a Hepatitis B virus infection, comprising an anti-PD-1 antibody and a pharmaceutically acceptable carrier.

The invention provides the use of a pharmaceutical composition comprising an anti-PD-1 antibody, or a fragment thereof, and a pharmaceutically acceptable carrier to prepare a medicament for the treatment of a Hepatitis B virus infection.

The invention provides an anti-PD-1 antibody, or fragment thereof, for the prophylactic or therapeutic treatment of a Hepatitis B virus infection, in combination with at least one additional therapeutic agent.

The invention provides the use of an anti-PD-1 antibody, or fragment thereof, to prepare a medicament for the treatment of a Hepatitis B virus infection, in combination with at least one additional therapeutic agent.

The invention provides a method for ameliorating one or more symptoms associated with Hepatitis B virus infection in a mammal, the method comprising the step of administering to the mammal a therapeutically effective amount of an anti-PD-1 antibody, or fragment thereof

The invention provides an anti-PD-1 antibody, or fragment thereof, for ameliorating one or more symptoms associated with Hepatitis B virus infection in a mammal.

The invention provides the use of an anti-PD-1 antibody, or a fragment thereof, to prepare a medicament for ameliorating one or more symptoms associated with Hepatitis B virus infection in a mammal.

The invention provides a pharmaceutical composition for use in ameliorating one or more symptoms associated with Hepatitis B virus infection in a mammal, comprising an anti-PD-1 antibody, or a fragment thereof, and a pharmaceutically acceptable carrier.

The invention provides the use of a pharmaceutical composition comprising an anti-PD-1 antibody, or a fragment thereof, and a pharmaceutically acceptable carrier to prepare a medicament for ameliorating one or more symptoms associated with Hepatitis B virus infection in a mammal.

The invention also provides an anti-PD-1 antibody, or fragment thereof, for ameliorating one or more symptoms associated with Hepatitis B virus infection in a mammal, in combination with at least one additional therapeutic agent.

The invention provides the use of an anti-PD-1 antibody, or a fragment thereof, to prepare a medicament for ameliorating one or more symptoms associated with Hepatitis B virus infection in a mammal, in combination with at least one additional therapeutic agent.

The invention also provides a kit comprising:
(a) a pharmaceutical composition comprising an anti-PD-1 antibody, or a fragment thereof, and a pharmaceutically acceptable diluent or carrier;
(b) a pharmaceutical composition comprising at least one additional therapeutic agent and a pharmaceutically acceptable diluent or carrier; and
(c) instructions for the simultaneous, sequential or separate administration of the an anti-PD-1 antibody, or a fragment thereof, and the at least one additional therapeutic agent.

### BRIEF DESCRIPTION OF THE FIGURES

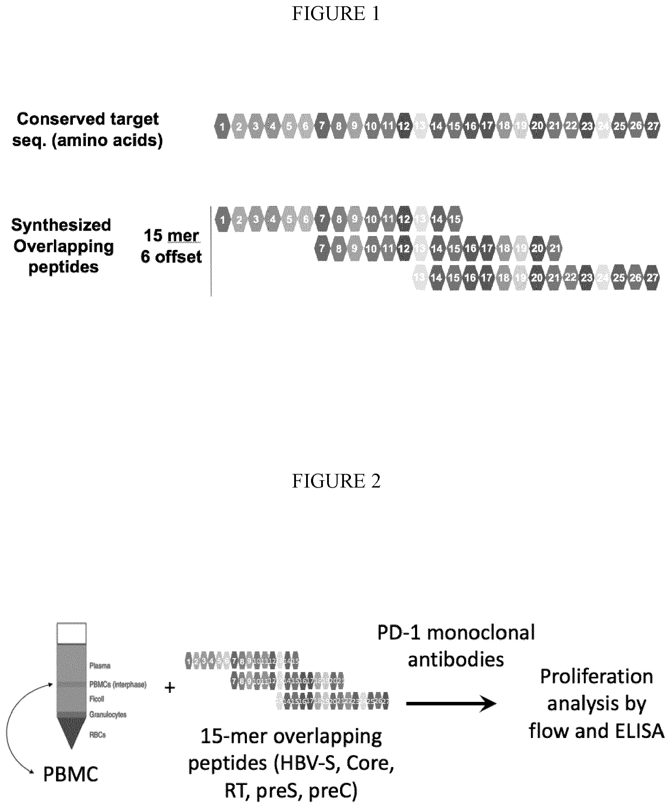

**Figure 1****.** The schematic design of 15-mer overlapping peptide pools from a conserved sequence.
**Figure 2****.** The schematic diagram of T cell proliferation in response to HBV-specific 15-mer overlapping peptides.
**Figure 3****.** T cell proliferation in allogeneic co-culture subtracted from background in the presence of anti-PD-1 mAb clones (n=3).
**Figure 4****.** α-PD-1 mAb enhances general T cell proliferation from chronic HBV patient.
**Figure 5****.** Anti-PD-1 antibody enhances the general T cell divisions from chronic HBV patient following anti-CD3 stimulation.
**Figure 6****.** Anti-PD-1 clones enhances IFN-γ production (average of 3 replicates) from general T cells in chronic HBV patients.
**Figure 7****.** Anti-PD1 mAb stimulates HBV-specific T cell proliferation (%) to HBV-specific 15-mer overlapping peptides and positive control Influenza 15-mer overlapping peptides.

### DETAILED DESCRIPTION

The present invention provides methods for treating, preventing, reducing the risk or likelihood of developing (e.g., reducing the susceptibility to), delaying the onset of, and/or ameliorating one or more symptoms associated with Hepatitis B virus (HBV) infection in a mammal (e.g., human) in need thereof, the method comprising administering to the mammal a therapeutically effective amount of of an anti-PD-1 antibody, or a fragment thereof. Examples of symptoms associated with HBV and/or HDV infection in a human include fever, abdominal pain, dark urine, joint pain, loss of appetite, nausea, vomiting, weakness, fatigue and yellowing of the skin (jaundice).

The term "Hepatitis B virus" (abbreviated as HBV) refers to a virus species of the genus Orthohepadnavirus, which is a part of the Hepadnaviridae family of viruses, and that is capable of causing liver inflammation in humans.

As used herein, "treatment" (and grammatical variations thereof such as "treat" or "treating") refers to clinical intervention to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis.

By the phrase "therapeutically effective amount" is meant an amount that produces the desired effect for which it is administered. The exact amount will depend on the purpose of the treatment, and will be ascertainable by one skilled in the art using known techniques (see, for example, Lloyd (1999) The Art, Science and Technology of Pharmaceutical Compounding).

The term "mammal" refers to any mammalian species such as a human, mouse, rat, dog, cat, hamster, guinea pig, rabbit, livestock, and the like.

The term "virus particle load", as used herein, refers to a measure of the number of virus particles present in a bodily fluid, such as blood. For example, particle load may be expressed as the number of virus particles per milliliter of, e.g., blood. Particle load testing may be performed using nucleic acid amplification based tests, as well as non-nucleic acid-based tests (*see, e.g.,* Puren et al., The Journal of Infectious Diseases, 201:S27-36 (2010)). In some embodiments, an anti-PD-1 antibody, or fragment thereof, is useful in decreasing viral titers in the host and/or rescuing exhausted T-cells. In certain embodiments, the Hepatitis B virus particle load in the mammal is reduced by at least about 50% relative to Hepatitis B virus particle load in the absence of the anti-PD-1 antibody, or fragment thereof.

### Anti-PD-1 Antibodies

The term "PD-1" refers to the programmed death-1 protein, a T-cell co-inhibitor, also known as CD279. A representative amino acid sequence of full-length PD-1 is provided in GenBank as accession number NP_005009.2. The term "PD-1" includes protein variants and recombinant PD-1 or a fragment thereof. Unless specified as being from a non-human species, the term "PD-1" means human PD-1.

PD-1 is a 288 amino acid protein receptor expressed on activated T-cells and B-cells, natural killer cells and monocytes. PD-1 is a member of the CD28/CTLA-4 (cytotoxic T lymphocyte antigen)/ICOS (inducible co-stimulator) family of T-cell co-inhibitory receptors (Chen et al. 2013, Nat. Rev. Immunol. 13: 227-242). The protein has an extracellular N-terminal domain which is IgV-like, a transmembrane domain and an intracellular domain containing an immunoreceptor tyrosine-based inhibitory (ITIM) motif and an immunoreceptor tyrosine-based switch (ITSM) motif (Chattopadhyay et al 2009, Immunol. Rev.). The primary function of PD-1 is to attenuate the immune response (Riley 2009, lmmunol. Rev. 229:114-125). PD-1 has two ligands, PD-Ligand1 (PD-L1) and PD-L2. PD-L1 (CD274, B7H 1) is expressed widely on both lymphoid and non-lymphoid tissues such as CD4 and CD8 T-cells, macrophage lineage cells, peripheral tissues as well as on tumor cells, virally-infected cells and autoimmune tissue cells. PD-L2 (CD273, B7-DC) has a more restricted expression than PD-L1, being expressed on activated dendritic cells and macrophages (Dong et al 1999, Nature Med.). PD-1 binding to its ligands results in decreased T-cell proliferation and cytokine secretion, compromising humoral and cellular immune responses.

T-cell co-stimulatory and co-inhibitory molecules (collectively named co-signaling molecules) play a crucial role in regulating T-cell activation, subset differentiation, effector function and survival (Chen et al2013, Nature Rev. lmmunol. 13: 227-242). Following recognition of cognate peptide-MHC complexes on antigen-presenting cells by the T-cell receptor, co-signaling receptors co-localize with T-cell receptors at the immune synapse, where they synergize with TCR signaling to promote or inhibit T-cell activation and function (Flies et al. 2011, Yale J. Bioi. Med. 84: 409-421). The ultimate immune response is regulated by a balance between co-stimulatory and co-inhibitory signals ("immune checkpoints") (Pardoll2012, Nature 12: 252-264). While not wishing to be bound by theory, it is currently believed that PD-1 functions as one such 'immune checkpoint' in mediating peripheral T-cell tolerance and in avoiding autoimmunity. PD-1 binds to PD-L1 or PD-L2 and inhibits T-cell activation. The ability of PD1 to inhibit T-cell activation is exploited by chronic viral infections and tumors to evade immune response. In chronic viral infections, PD-1 is highly expressed on virus-specific T-cells and these T-cells become "exhausted" with loss of effector functions and proliferative capacity (Freeman 2008, PNAS 105: 10275-1 0276). The PD-1:PD-L1 system also plays an important role in induced T-regulatory (Treg) cell development and in sustaining Treg function (Francisco et al2010, lmmunol. Rev. 236:219-242).

Anti-PD-1 antibodies, and fragments thereof, are known in the art. For example, anti-PD-1 antibodies include, but are limited to, Nivolumab (MDX-1106), Pembrolizumab (MK-3475, lambrolizumab, BMS-936558), TSR-042 (Tesaro, Inc.), REGN2810 (Regeneron Pharmaceuticals) and EH12.2H7 (BioLegend, catalog no. 329902). Antibodies to PD-1 have also been described, e.g., in US Patent/Publication Nos. 8008449, 8168757, 9217034, 20110008369, 20130017199, 20130022595, and in WO/2003/099196, WO/2004/004771, WO/2004/072286, WO/2004/056875, WO/2006/121168, WO/2007/005874, WO/2008/083174, WO/2009/014708, WO/2009/073533, WO/2009/1154335, WO/2012/145493, WO/2013/014668, WO/2014/179664, WO/2015/112800, WO/2016/015685, WO/2009/101611, EP2262837, EP2504028, and Berger et al., Clin. Cancer Res., Vol. 14, pp. 30443051 (2008), which are incorporated by reference herein.

Accordingly, in certain embodiments, the anti-PD-1 antibody, or fragment thereof, is described in US 8008449, US 8168757, US 9217034, US 20110008369, US 20130017199, US 20130022595, WO/2003/099196, WO/2004/004771, WO/2004/072286, WO/2004/056875, WO/2006/121168, WO/2007/005874, WO/2008/083174, WO/2009/014708, WO/2009/073533, WO/2009/1154335, WO/2012/145493, WO/2013/014668, WO/2014/179664, WO/2015/112800, WO/2016/015685, WO/2009/101611, EP2262837, EP2504028, or Berger et al., Clin. Cancer Res., Vol. 14, pp. 30443051 (2008).

In certain embodiments, the anti-PD-1 antibody, or fragment thereof, is Nivolumab (MDX-1106), Pembrolizumab (MK-3475, lambrolizumab, BMS-936558), TSR-042 (Tesaro, Inc.), REGN2810 (Regeneron Pharmaceuticals) or EH12.2H7 (BioLegend, catalog no. 329902).

In certain embodiments, the anti-PD-1 antibody, or fragment thereof, is EH12.2H7 (BioLegend, catalog no. 329902).

In one embodiment, the anti-PD-1 antibody, or fragment thereof, specifically binds to PD-1 and modulates its activity. In certain embodiments, the anti-PD-1 antibody is a full-length antibody (for example, an IgG1 or IgG4 antibody). In certain embodiments, a fragment of an anti-PD-1 antibody is used in the methods of the invention, and comprises only an antigen-binding portion (for example, a Fab, F(ab')₂ or scFv fragment). In certain embodiments, the anti-PD-1 antibody, or fragment thereof, is modified to affect functionality, e.g., to eliminate residual effector functions (Reddy et al., 2000, J. lmmunol. 164:1925-1933).

In certain embodiments, the anti-PD-1 antibody is a monoclonal antibody, or fragment thereof. In certain embodiments, the anti-PD-1 antibody is an isolated recombinant monoclonal antibody, or fragment thereof, that binds specifically to PD-1. In certain embodiments, the anti-PD-1 antibody, or a fragment thereof, is a human antibody, or a fragment thereof. In certain embodiments, the antibodies are fully human. In certain embodiments, the anti-PD-1 antibody, or a fragment thereof, is a blocking antibody, or a fragment thereof, or a neutralizing antibody, or a fragment thereof.

In certain embodiments, the antibodies or antigen-binding fragments are bispecific comprising a first binding specificity to PD-1 and a second binding specificity for a second target epitope. The second target epitope may be another epitope on PD-1 or on a different protein.

As used herein, the term "Fc receptor" refers to the surface receptor protein found on immune cells including B lymphocytes, natural killer cells, macrophages, basophils, neutrophils, and mast cells, which has a binding specificity for the Fc region of an antibody. The term "Fc receptor" includes, but is not limited to, a Fey receptor [e.g., FcyRI (CD64), FcyRI IA (CD32), FcyRII B (CD32), FcyRI IIA (CD16a), and FcyRIII B (CD16b)], Fca receptor (e.g., FcaRI or CD89) and Fes receptor [e.g., FcsRI , and FcsRII (CD23)].

The term "antibody", as used herein, is intended to refer to immunoglobulin molecules comprised of four polypeptide chains, two heavy (H) chains and two light (L) chains interconnected by disulfide bonds (i.e., "full antibody molecules"), as well as multimers thereof (e.g. IgM) or antigen-binding fragments thereof. Each heavy chain is comprised of a heavy chain variable region ("HCVR" or "V_{H}") and a heavy chain constant region (comprised of domains CH₁, CH₂ and CH₃). Each light chain is comprised of a light chain variable region ("LCVR or "V_{L}") and a light chain constant region (C_{L}). The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The FRs of the antibody (or antigen binding fragment thereof) may be identical to the human germline sequences, or may be naturally or artificially modified. An amino acid consensus sequence may be defined based on a side-by-side analysis of two or more CDRs. Substitution of one or more CDR residues or omission of one or more CDRs is also possible. Antibodies have been described in the scientific literature in which one or two CDRs can be dispensed with for binding. Padlan et al. (1995 FASEB J. 9:133-139) analyzed the contact regions between antibodies and their antigens, based on published crystal structures, and concluded that only about one fifth to one third of CDR residues actually contact the antigen. Padlan also found many antibodies in which one or two CDRs had no amino acids in contact with an antigen (see also, Vajdos et al. 2002 J Mol Biol 320:415-428). CDR residues not contacting antigen can be identified based on previous studies (for example residues H60-H65 in CDRH2 are often not required), from regions of Kabat CDRs lying outside Chothia CDRs, by molecular modeling and/or empirically. If a CDR or residue(s) thereof is omitted, it is usually substituted with an amino acid occupying the corresponding position in another human antibody sequence or a consensus of such sequences. Positions for substitution within CDRs and amino acids to substitute can also be selected empirically.

Unless specifically indicated otherwise, the term "antibody," as used herein, shall be understood to encompass antibody molecules comprising two immunoglobulin heavy chains and two immunoglobulin light chains (i.e., "full antibody molecules"), as well as antigen-binding fragments thereof. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. The terms "antigen-binding fragment" of an antibody, or "antibody fragment", as used herein, refers to one or more fragments of an antibody that retain the ability to specifically bind to PD-1.

An antibody fragment may include a Fab fragment, a F(ab')₂ fragment, a Fv fragment, a dAb fragment, a fragment containing a CDR, or an isolated CDR. In certain embodiments, the term "antigen-binding fragment" refers to a polypeptide fragment of a multi-specific antigen-binding molecule. In such embodiments, the term" antigen-binding fragment" includes, e.g., an extracellular domain of PD-L1 which binds specifically to PD-1. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and (optionally) constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g. , an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR, which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H} - V_{H}, V_{H} - V_{L} or V_{L} - V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}1 ; (ii) V_{H}-C_{H}2; (iii) V_{H}-C_{H}3; (iv) V_{H}-C_{H}1-C_{H}2; (V) V_{H}-C_{H}1-C_{H}2-C_{H}3; (vi) V_{H}-C_{H}2-C_{H}3; (vii) V_{H}-C_{L}; (viii) V_{L}-C_{H}1 ; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids, which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in non-covalent association with one another and/or with one or more monomeric V_{H} or V_{L} domain (e.g., by disulfide bond(s)).

As with full antibody molecules, antigen-binding fragments may be mono-specific or multi-specific (e.g., bi-specific). A multi-specific antigen-binding fragment of an antibody will typically comprise at least two different variable domains, wherein each variable domain is capable of specifically binding to a separate antigen or to a different epitope on the same antigen. Any multi-specific antibody format, including the exemplary bi-specific antibody formats disclosed herein, may be adapted for use in the context of an antigen-binding fragment of an antibody of the present invention using routine techniques available in the art.

The term "human antibody", as used herein, is intended to include antibodies having variable and constant regions derived from human germline immunoglobulin sequences. The human mAbs may include amino acid residues not encoded by human germline immunoglobulin sequences (e.g., mutations introduced by random or site-specific mutagenesis in vitro or by somatic mutation in vivo), for example in the CDRs and in particular CDR3.

However, the term "human antibody", as used herein, is not intended to include mAbs in which CDR sequences derived from the germline of another mammalian species (e.g., mouse), have been grafted onto human FR sequences. The term includes antibodies recombinantly produced in a non-human mammal, or in cells of a non-human mammal.

The term "recombinant", as used herein, refers to antibodies or antigen-binding fragments thereof created, expressed, isolated or obtained by technologies or methods known in the art as recombinant DNA technology which include, e.g., DNA splicing and transgenic expression. The term refers to antibodies expressed in a non-human mammal (including transgenic non-human mammals, e.g., transgenic mice), or a cell (e.g., CHO cells) expression system or isolated from a recombinant combinatorial human antibody library.

The term "multi-specific antigen-binding molecules", as used herein refers to bispecific, tri-specific or multi-specific antigen-binding molecules, and antigen-binding fragments thereof. Multi-specific antigen-binding molecules may be specific for different epitopes of one target polypeptide or may contain antigen-binding domains specific for epitopes of more than one target polypeptide. A multi-specific antigen-binding molecule can be a single multifunctional polypeptide, or it can be a multimeric complex of two or more polypeptides that are covalently or non-covalently associated with one another. The term "multi-specific antigen-binding molecules" includes antibodies that may be linked to or co-expressed with another functional molecule, e.g., another peptide or protein. For example, an antibody or fragment thereof can be functionally linked (e.g., by chemical coupling, genetic fusion, non-covalent association or otherwise) to one or more other molecular entities, such as a protein or fragment thereof to produce a bi-specific or a multi-specific antigen-binding molecule with a second binding specificity. According to the present invention, the term "multi-specific antigen-binding molecules" also includes bi-specific, tri-specific or multi-specific antibodies or antigen-binding fragments thereof. In certain embodiments, an antibody may be functionally linked to another antibody or antigen-binding fragment thereof to produce a bispecific antibody with a second binding specificity.

The term "specifically binds," or "binds specifically to", or the like, means that an antibody or antigen-binding fragment thereof forms a complex with an antigen that is relatively stable under physiologic conditions. Specific binding can be characterized by an equilibrium dissociation constant of at least about 1x10⁻⁸ M or less (e.g., a smaller K_{D} denotes a tighter binding). Methods for determining whether two molecules specifically bind are well known in the art and include, for example, equilibrium dialysis, surface plasmon resonance (e.g., BIACORE^{™}), and the like. Moreover, multi-specific antibodies that bind to one domain in PD-1 and one or more additional antigens or a bi-specific that binds to two different regions of PD-1 are nonetheless considered antibodies that "specifically bind", as used herein.

The term "high affinity" antibody refers to those mAbs having a binding affinity to PD-1, expressed as K_{D}, of at least 10⁻⁷ M; preferably 10⁻⁸ M; more preferably 10⁻⁹M, even more preferably 10⁻¹⁰ M, even more preferably 10⁻¹¹ M, as measured by surface plasmon resonance, e.g., BIACORE^{™} or solution-affinity ELISA.

By the term "slow off rate", "Koff' or "kd" is meant an antibody that dissociates from PD-1, with a rate constant of 1 x 10⁻³ s⁻¹ or less, preferably 1 x 10⁻⁴ s⁻¹ or less, as determined by surface plasmon resonance, e.g., BIACORE^{™}.

Anti-PD-1 antibodies, or fragments thereof, may be conjugated to a moiety such a ligand or a therapeutic moiety ("immunoconjugate"), such as a second anti-PD-1 antibody, or an antibody to another antigen, such a virally-infected cell antigen, a Fc receptor, a T-cell receptor, or a T-cell co-inhibitor, or any other therapeutic moiety useful for treating a HBV.

An "isolated antibody", as used herein, is intended to refer to an antibody that is substantially free of other antibodies (Abs) having different antigenic specificities (e.g., an isolated antibody that specifically binds PD-1, or a fragment thereof, is substantially free of Abs that specifically bind antigens other than PD-1.

A "blocking antibody" or a "neutralizing antibody", as used herein (or an "antibody that neutralizes PD-1 activity" or "antagonist antibody"), is intended to refer to an antibody whose binding to PD-1 results in inhibition of at least one biological activity of PD-1. For example, an antibody of the invention may prevent or block PD-1 binding to PD-L1.

The term "surface plasmon resonance", as used herein, refers to an optical phenomenon that allows for the analysis of real-time biomolecular interactions by detection of alterations in protein concentrations within a biosensor matrix, for example using the BIACORE^{™} system (Pharmacia Biosensor AB, Uppsala, Sweden and Piscataway, N.J.).

The term "K_{D} ", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. The term "epitope" also refers to a site on an antigen to which B and/or T cells respond. It also refers to a region of an antigen that is bound by an antibody. Epitopes may be defined as structural or functional. Functional epitopes are generally a subset of the structural epitopes and have those residues that directly contribute to the affinity of the interaction. Epitopes may also be conformational, that is, composed of nonlinear amino acids. In certain embodiments, epitopes may include determinants that are chemically active surface groupings of molecules such as amino acids, sugar side chains, phosphoryl groups, or sulfonyl groups, and, in certain embodiments, may have specific three-dimensional structural characteristics, and/or specific charge characteristics.

As applied to polypeptides, the term "substantial similarity" or "substantially similar" means that two peptide sequences, when optimally aligned, such as by the programs GAP or BESTFIT using default gap weights, share at least 90% sequence identity, even more preferably at least 95%, 98% or 99% sequence identity. Preferably, residue positions, which are not identical, differ by conservative amino acid substitutions. A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art. See, e.g., Pearson (1994) Methods Mol. Biol. 24: SOT-SSI, which is herein incorporated by reference. Examples of groups of amino acids that have side chains with similar chemical properties include 1 ) aliphatic side chains: glycine, alanine, valine, leucine and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate, and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256:1443 45, herein incorporated by reference. A "moderately conservative" replacement is any change having a nonnegative value in the PAM250 log-likelihood matrix.

Sequence similarity for polypeptides is typically measured using sequence analysis software. Protein analysis software matches similar sequences using measures of similarity assigned to various substitutions, deletions and other modifications, including conservative amino acid substitutions. For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA with default or recommended parameters; a program in GCG Version 6.1. FASTA (e.g., FASTA2 and FAST A3) provides alignments and percent sequence identity of the regions of the best overlap between the query and search sequences (Pearson (2000) supra). Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215: 403-410 and (1997) Nucleic Acids Res. 25:3389-3402, each of which is herein incorporated by reference.

The antibodies and antigen-binding fragments, which may be used in the present invention, specifically bind to PD-1. In certain embodiments, an anti-PD-1 antibody, or fragment thereof, may modulate the interaction of PD-1 with PD-L1. The anti-PD-1 antibodies may bind to PD-1 with high affinity or with low affinity. In certain embodiments, the antibodies or fragments thereof, which may be used in the present invention, may be blocking antibodies wherein the antibodies may bind to PD-1 and block the interaction of PD-1 with PD-L1. They may be used alone or as adjunct therapy with other therapeutic moieties or modalities known in the art (i.e., at least one additional therapeutic agent) for treating an HBV infection.

In certain embodiments, the anti-PD-1 antibodies may be multi-specific antigen-binding molecules, wherein they comprise a first binding specificity to PD-1 and a second binding specificity to an antigen selected from the group consisting of another T-cell co-inhibitor, T-cell receptor, Fc receptor, T-cell receptor, PD-L1, and a different epitope of PD-1.

Certain anti-PD-1 antibodies may be able to bind to and neutralize the activity of PD-1, as determined by in vitro or in vivo assays. The ability of the antibodies to bind to and neutralize the activity of PD-1 may be measured using any standard method known to those skilled in the art, including binding assays or activity assays.

The antibodies specific for PD-1 may contain no additional labels or moieties, or they may contain an N-terminal or C-terminal label or moiety. In one embodiment, the label may be a radionuclide or a fluorescent dye. In certain embodiments, such labeled antibodies may be used in diagnostic assays.

### Preparation of Human Antibodies

Methods for generating human antibodies in transgenic mice are known in the art. Any such known methods can be used in the context of the present invention to make human antibodies that specifically bind to PD-1.

An immunogen comprising any one of the following can be used to generate antibodies to PD-1, or fragments thereof. For example, the primary immunogen may be a full length PD-1 (See GenBank accession number NP_005009.2) or a recombinant form of PD-1 or modified human PD-1 fragments or modified cynomolgus PD-1 fragments. In certain embodiments, the primary immunogen may be followed by immunization with a secondary immunogen, or with an immunogenically active fragment of PD-1. Alternatively, PD-1 or a fragment thereof may be produced using standard biochemical techniques and modified and used as immunogen. The immunogen may be a biologically active and/or immunogenic fragment of PD-1 or DNA encoding the active fragment thereof.

In certain embodiments, the immunogen may be a peptide from the N terminal or C terminal end of PD-1. In one embodiment, the immunogen is the extracellular domain or the IgV-like domain of PD-1. In some embodiments, the immunogen may be a recombinant PD-1 peptide expressed in E. coli or in any other eukaryotic or mammalian cells such as Chinese hamster ovary (CHO) cells. The peptides may be modified to include addition or substitution of certain residues for tagging or for purposes of conjugation to carrier molecules, such as, KLH. For example, a cysteine may be added at either the N terminal or C terminal end of a peptide, or a linker sequence may be added to prepare the peptide for conjugation to, for example, KLH for immunization.

VELOCIMMUNE^{®} technology (see, for example, US 6,596,541, Regeneron Pharmaceuticals, VELOCIMMUNE^{®}) or any other known method for generating monoclonal antibodies may be used to isolate high affinity chimeric antibodies to PD-1 having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the full human antibody.

### Anti-PD-1 Antibodies Comprising Fc Variants

Anti-PD-1 antibodies may comprise an Fc domain comprising one or more mutations, which enhance or diminish antibody binding to the FcRn receptor, e.g., at acidic pH as compared to neutral pH. For example, anti-PD-1 antibodies may comprise a mutation in the C_{H}2 or a C_{H}3 region of the Fc domain, wherein the mutation(s) increases the affinity of the Fc domain to FcRn in an acidic environment (e.g., in an endosome where pH ranges from about 5.5 to about 6.0). Such mutations may result in an increase in serum half-life of the antibody when administered to an animal.

Anti-PD-1 antibodies may also comprise a chimeric heavy chain constant (C_{H}) region, wherein the chimeric C_{H} region comprises segments derived from the C_{H} regions of more than one immunoglobulin isotype. For example, the antibodies useful in the present invention may comprise a chimeric C_{H} region comprising part or all of a C_{H}2 domain derived from a human IgG1, human IgG2 or human IgG4 molecule, combined with part or all of a C_{H}3 domain derived from a human IgG1, human IgG2 or human IgG4 molecule. According to certain embodiments, the antibodies may comprise a chimeric C_{H} region having a chimeric hinge region. For example, a chimeric hinge may comprise an "upper hinge" amino acid sequence derived from a human IgG1, a human IgG2 or a human IgG4 hinge region, combined with a "lower hinge" sequence derived from a human IgG1, a human IgG2 or a human IgG4 hinge region. According to certain embodiments, the chimeric hinge region comprises amino acid residues derived from a human IgG1 or a human IgG4 upper hinge and amino acid residues derived from a human IgG2 lower hinge. An antibody comprising a chimeric CH region as described herein may, in certain embodiments, exhibit modified Fc effector functions without adversely affecting the therapeutic or pharmacokinetic properties of the antibody.

### Biological Characteristics of Anti-PD-1 Antibodies, or Fragments Thereof

In general, the antibodies useful for the present invention function by binding to PD-1, and include anti-PD-1 antibodies and antigen-binding fragments thereof that bind soluble monomeric or dimeric PD-1 molecules with high affinity. For example, antibodies and antigen-binding fragments of antibodies that bind monomeric PD-1 (e.g., at 25°C or at 37°C) with a K_{D} of less than about 50nM as measured by surface plasmon resonance may be used in the present invention. In certain embodiments, the antibodies or antigen-binding fragments thereof bind monomeric PD-1 with a K_{D} of less than about 40nM, less than about 30nM, less than about 20nM, less than about 10nM less than about 5nM, less than about 2nM or less than about 1 nM, as measured by surface plasmon resonance or a substantially similar assay. The present invention also includes the use of antibodies and antigen-binding fragments thereof that bind dimeric PD-1 (e.g., at 25°C or at 37°C) with a K_{D} of less than about 400 pM as measured by surface plasmon resonance. In certain embodiments, the antibodies or antigen-binding fragments thereof bind dimeric PD-1 with a K_{D} of less than about 300 pM, less than about 250 pM, less than about 200 pM, less than about 100 pM, or less than about 50 pM, as measured by surface plasmon resonance, or a substantially similar assay. The present invention also includes the use of antibodies or antigen-binding fragments thereof that bind cynomolgus (Macaca fascicularis) PD-1 (e.g., at 25°C or at 37°C) with a K_{D} of less than about 35 nM as measured by surface plasmon resonance. In certain embodiments, the antibodies or antigen-binding fragments thereof bind cynomolgus PD-1 with a K_{D} of less than about 30 nM, less than about 20 nM, less than about 15 nM, less than about 10 nM, or less than about 5 nM, as measured by surface plasmon resonance or a substantially similar assay.

The present invention also includes the use of antibodies and antigen-binding fragments thereof that bind PD-1 with a dissociative half-life (t½) of greater than about 1.1 minutes as measured by surface plasmon resonance at 25°C or 37°C, or a substantially similar assay. In certain embodiments, the antibodies or antigen-binding fragments bind PD-1 with a t½ of greater than about 5 minutes, greater than about 10 minutes, greater than about 30 minutes, greater than about 50 minutes, greater than about 60 minutes, greater than about 70 minutes, greater than about 80 minutes, greater than about 90 minutes, greater than about 100 minutes, greater than about 200 minutes, greater than about 300 minutes, greater than about 400 minutes, greater than about 500 minutes, greater than about 600 minutes, greater than about 700 minutes, greater than about 800 minutes, greater than about 900 minutes, greater than about 1000 minutes, or greater than about 1200 minutes, as measured by surface plasmon resonance at 25°C or 37°C (e.g., mAb-capture or antigen-capture format), or a substantially similar assay.

In some embodiments, the antibodies may bind to the extracellular domain of PD-1 or to a fragment of the domain. In some embodiments, the antibodies for use in the present invention may bind to more than one domain (cross-reactive antibodies). In certain embodiments, the antibodies for use in the present invention may bind to an epitope located in the extracellular domain comprising amino acid residues 21 - 171 of PD-1.

In certain embodiments, the antibodies may function by blocking or inhibiting the PD-L1 -binding activity associated with PD-1 by binding to any region or fragment of the full length protein. In certain embodiments, the antibodies may attenuate or modulate the interaction between PD-1 and PD-L1.

In certain embodiments, antibodies for use in the present invention may be bi-specific antibodies. The bi-specific antibodies may bind one epitope in one domain and may also bind a second epitope in a different domain of PD-1. In certain embodiments, the bi-specific antibodies may bind two different epitopes in the same domain.

In one embodiment, the invention provides for the use of an isolated fully human monoclonal antibody or antigen-binding fragment thereof that binds to PD-1.

### Species Selectivity and Species Cross-Reactivity

According to certain embodiments, an anti-PD-1 antibody for use in the present invention may bind to human PD-1 but not to PD-1 from other species. Alternatively, an anti-PD-1 antibody may bind to human PD-1 and to PD-1 from one or more non-human species. For example, an anti-PD-1 antibody may bind to human PD-1 and may bind or not bind, as the case may be, to one or more of mouse, rat, guinea pig, hamster, gerbil, pig, cat, dog, rabbit, goat, sheep, cow, horse, camel, cynomolgus, marmoset, rhesus or chimpanzee PD-1. In certain embodiments, an anti-PD-1 antibody may bind to human and cynomolgus PD-1 with the same affinities or with different affinities, but do not bind to rat and mouse PD-1.

### Epitope Mapping and Related Technologies

Anti-PD-1 antibodies that may be used in the present invention may interact with one or more amino acids found within one or more domains of the PD-1 molecule including, e.g., extracellular (IgV-like) domain, a transmembrane domain, and an intracellular domain containing the immunoreceptor tyrosine-based inhibition motif (ITIM) and immunoreceptor tyrosine-based switch motif (ITSM). The epitope to which the antibodies bind may consist of a single contiguous sequence of 3 or more (e.g., 3, 4, 5, 6, 7, 8, 9, 10, 11 , 12, 13, 14, 15, 16, 17, 18, 19, 20 or more) amino acids located within any of the aforementioned domains of the PD-1 molecule (e.g. a linear epitope in a domain). Alternatively, the epitope may consist of a plurality of noncontiguous amino acids (or amino acid sequences) located within either or both of the aforementioned domains of the PD-1 molecule (e.g. a conformational epitope).

Various techniques known to persons of ordinary skill in the art can be used to determine whether an antibody "interacts with one or more amino acids" within a polypeptide or protein. Exemplary techniques include, for example, routine cross-blocking assays, such as that described in Antibodies, Harlow and Lane (Cold Spring Harbor Press, Cold Spring Harbor, NY). Other methods include alanine scanning mutational analysis, peptide blot analysis (Reineke (2004) Methods Mol. Biol. 248: 443-63), peptide cleavage analysis crystallographic studies and NMR analysis. In addition, methods such as epitope excision, epitope extraction and chemical modification of antigens can be employed (Tomer (2000) Prot. Sci. 9: 487-496). Another method that can be used to identify the amino acids within a polypeptide with which an antibody interacts is hydrogen/deuterium exchange detected by mass spectrometry. In general terms, the hydrogen/deuterium exchange method involves deuterium-labeling the protein of interest, followed by binding the antibody to the deuterium-labeled protein. Next, the protein/antibody complex is transferred to water and exchangeable protons within amino acids that are protected by the antibody complex undergo deuterium-to-hydrogen back-exchange at a slower rate than exchangeable protons within amino acids that are not part of the interface. As a result, amino acids that form part of the protein/antibody interface may retain deuterium and therefore exhibit relatively higher mass compared to amino acids not included in the interface. After dissociation of the antibody, the target protein is subjected to protease cleavage and mass spectrometry analysis, thereby revealing the deuterium-labeled residues which correspond to the specific amino acids with which the antibody interacts. See, e.g., Ehring (1999) Analytical Biochemistry 267: 252-259; Engen and Smith (2001) Anal. Chem. 73: 256A-265A.

The term "epitope" may refer to a site on an antigen to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5 or 8-10 amino acids in a unique spatial conformation.

Modification-Assisted Profiling (MAP), also known as Antigen Structure-based Antibody Profiling (ASAP) is a method that categorizes large numbers of monoclonal antibodies (mAbs) directed against the same antigen according to the similarities of the binding profile of each antibody to chemically or enzymatically modified antigen surfaces (see US 2004/0101920, herein specifically incorporated by reference in its entirety). Each category may reflect a unique epitope either distinctly different from or partially overlapping with epitope represented by another category. This technology allows rapid filtering of genetically identical antibodies, such that characterization can be focused on genetically distinct antibodies. When applied to hybridoma screening, MAP may facilitate identification of rare hybridoma clones that produce mAbs having the desired characteristics. MAP may be used to sort the antibodies, which may be used in the present invention into groups of antibodies binding different epitopes.

Anti-PD-1 antibodies or antigen-binding fragments thereof, which may be used in the present invention, may bind an epitope within any one or more of the regions exemplified in PD-1, either in natural form, or recombinantly produced, or to a fragment thereof.

### Multi-specific Antibodies

As discussed herein, the antibodies that may be used in the present invention may be mono-specific, bi-specific, or multi-specific. Multi-specific antibodies may be specific for different epitopes of one target polypeptide or may contain antigen-binding domains specific for more than one target polypeptide. See, e.g., Tutt et al., 1991, J. Immunol. 147:60-69; Kufer et al., 2004, Trends Biotechnol. 22:238-244.

In one aspect, multi-specific antigen-binding molecules or antigen-binding fragments thereof may be used in the present invention, wherein one specificity of an immunoglobulin is specific for the extracellular domain of PD-1, or a fragment thereof, and the other specificity of the immunoglobulin is specific for binding outside the extracellular domain of PD-1 or for a second therapeutic target. A multi-specific antigen-binding molecule, or variant thereof, may be constructed using standard molecular biological techniques (e.g., recombinant DNA and protein expression technology), as will be known to a person of ordinary skill in the art.

In some embodiments, PD-1-specific antibodies may be generated in a bi-specific format (a "bi-specific") in which variable regions binding to distinct domains of PD-1 are linked together to confer dual-domain specificity within a single binding molecule. Appropriately designed bi-specifics may enhance overall PD-1 inhibitory efficacy through increasing both specificity and binding avidity. Variable regions with specificity for individual domains, (e.g., segments of the N-terminal domain), or that can bind to different regions within one domain, are paired on a structural scaffold that allows each region to bind simultaneously to the separate epitopes, or to different regions within one domain. In one example for a bi-specific, heavy chain variable regions (V_{H}) from a binder with specificity for one domain are recombined with light chain variable regions (V_{L}) from a series of binders with specificity for a second domain to identify non-cognate V_{L} partners that can be paired with an original V_{H} without disrupting the original specificity for that V_{H}. In this way, a single V_{L} segment (e.g., V_{L}1) can be combined with two different V_{H} domains (e.g., V_{H}1 and V_{H}2) to generate a bi-specific comprised of two binding "arms" (V_{H}1 - V_{L}1 and V_{H}2- V_{L}1). Use of a single V_{L} segment reduces the complexity of the system and thereby simplifies and increases efficiency in cloning, expression, and purification processes used to generate the bi-specific (See, for example, USSN 13/022759 and US2010/0331527).

Alternatively, antibodies that bind more than one domain and a second target, such as, but not limited to, for example, a second different anti-PD-1 antibody, may be prepared in a bi-specific format using techniques described herein, or other techniques known to those skilled in the art. Antibody variable regions binding to distinct regions may be linked together with variable regions that bind to relevant sites on, for example, the extracellular domain of PD-1, to confer dual-antigen specificity within a single binding molecule. Appropriately designed bi-specifics of this nature serve a dual function. Variable regions with specificity for the extracellular domain are combined with a variable region with specificity for outside the extracellular domain and are paired on a structural scaffold that allows each variable region to bind to the separate antigens.

Exemplary bispecific formats that can be used in the context of the present invention include, without limitation, e.g., scFv-based or diabody bispecific formats, IgG-scFv fusions, dual variable domain (DVD)-Ig, Quadroma, knobs-into-holes, common light chain (e.g., common light chain with knobs-into-holes, etc.), CrossMab, CrossFab, (SEED)body, leucine zipper, Duobody, IgG1/IgG2, dual acting Fab (DAF)-IgG, and Mab² bispecific formats (see, e.g., Klein et at. 2012, mAbs 4:6, 1-11, and references cited therein, for a review of the foregoing formats). Bispecific antibodies can also be constructed using peptide/nucleic acid conjugation, e.g., wherein unnatural amino acids with orthogonal chemical reactivity are used to generate site-specific antibody-oligonucleotide conjugates which then self-assemble into multimeric complexes with defined composition, valency and geometry. (See, e.g., Kazane et ai, J. Am. Chem. Soc. [Epub: Dec. 4, 2012]).

### Therapeutic Administration and Formulations

In certain embodiments of the invention, therapeutic compositions comprising an anti-PD-1 antibody, or antigen-binding fragments thereof, may be administered. Such therapeutic compositions may be administered with suitable carriers, excipients, and other agents that are incorporated into formulations to provide improved transfer, delivery, tolerance, and the like. A multitude of appropriate formulations can be found in the formulary known to pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-31 1.

The dose of antibody may vary depending upon the age and the size of a subject to be administered, target disease, conditions, route of administration, and the like. When an antibody is used for treating an HBV infection in an adult patient, or for preventing such a disease, it is advantageous to administer the antibody of the present invention normally at a single dose of about 0.1 to about 50 mg/kg body weight, specifically about 0.1 to about 25 mg/kg body weight, specifically about 0.1 to about 15 mg/kg body weight, or specifically about 0.1 to about 5 mg/kg body weight. Depending on the severity of the condition, the frequency and the duration of the treatment can be adjusted. In certain embodiments, the antibody or antigen-binding fragment thereof can be administered as an initial dose of at least about 0.1 mg to about 50 mg, about 0.1 mg to about 25 mg, about 0.1 to about 15 mg, or about 0.1 mg to about 5 mg. In certain embodiments, the initial dose may be followed by administration of a second or a plurality of subsequent doses of the antibody or antigen-binding fragment thereof in an amount that can be approximately the same or less than that of the initial dose, wherein the subsequent doses are separated by at least 1 day to 3 days; at least one week, at least 2 weeks; at least 3 weeks; at least 4 weeks; at least 5 weeks; at least 6 weeks; at least 7 weeks; at least 8 weeks; at least 9 weeks; at least 10 weeks; at least 12 weeks; or at least 14 weeks.

Various delivery systems are known and can be used to administer the pharmaceutical composition, e.g., encapsulation in liposomes, microparticles, microcapsules, recombinant cells capable of expressing mutant viruses, receptor mediated endocytosis (see, e.g., Wu et al. (1987) J. Biol. Chem. 262:4429-4432). Methods of introduction include, but are not limited to, intradermal, transdermal, intramuscular, intraperitoneal, intravenous, subcutaneous, intranasal, epidural and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. The pharmaceutical composition can be also delivered in a vesicle, in particular a liposome (see, for example, Langer (1990) Science 249:1527-1533).

The use of nanoparticles to deliver the antibodies of the present invention is also contemplated herein. Antibody-conjugated nanoparticles may be used both for therapeutic and diagnostic applications. Antibody-conjugated nanoparticles and methods of preparation and use are described in detail by Arruebo, M., et al. 2009 ("Antibody-conjugated nanoparticles for biomedical applications" in J. Nanomat. Volume 2009, Article ID 439389, 24 pages, doi: 10.1 155/2009/439389), incorporated herein by reference. Nanoparticles may be developed and conjugated to antibodies contained in pharmaceutical compositions to target virally infected cells. Nanoparticles for drug delivery have also been described in, for example, US 8257740, or US 8246995, each incorporated herein in its entirety.

In certain situations, the pharmaceutical composition can be delivered in a controlled release system. In one embodiment, a pump may be used. In another embodiment, polymeric materials can be used. In yet another embodiment, a controlled release system can be placed in proximity of the composition's target, thus requiring only a fraction of the systemic dose.

The injectable preparations may include dosage forms for intravenous, subcutaneous, intracutaneous, intracranial, intraperitoneal and intramuscular injections, drip infusions, etc. These injectable preparations may be prepared by methods publicly known. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant (e.g., polysorbate 80, HCO-50 (polyoxyethylene (50 mol) adduct of hydrogenated castor oil)), etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

A pharmaceutical composition comprising an anti-PD-1 antibody, or fragment thereof, can be delivered subcutaneously or intravenously with a standard needle and syringe. In addition, with respect to subcutaneous delivery, a pen delivery device readily has applications in delivering such a pharmaceutical composition. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded. Numerous reusable pen and autoinjector delivery devices have applications in the subcutaneous delivery of a pharmaceutical composition. Examples include, but are not limited to AUTOPEN^{™} (Owen Mumford, Inc., Woodstock, UK), DISETRONIC^{™} pen (Disetronic Medical Systems, Burghdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name a few. Examples of disposable pen delivery devices having applications in subcutaneous delivery of a pharmaceutical composition, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.) and the HUMIRA^{™} Pen (Abbott Labs, Abbott Park, IL), to name a few.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. The amount of the antibody contained is generally about 5 to about 500 mg per dosage form in a unit dose; especially in the form of injection, it is preferred that the antibody is contained in about 5 to about 100 mg and in about 10 to about 250 mg for the other dosage forms.

### Administrative Regimens

According to certain embodiments of the present invention, multiple doses of an anti-PD-1 antibody, or fragment thereof (or a pharmaceutical composition comprising a combination of an anti-PD-1 antibody and any of the additional therapeutic agents mentioned herein), may be administered to a subject over a defined time course. The methods according to this aspect of the invention comprise sequentially administering to a subject multiple doses of an anti-PD-1 antibody of the invention. As used herein, "sequentially administering" means that each dose of anti-PD-1 antibody is administered to the subject at a different point in time, e.g., on different days separated by a predetermined interval (e.g., hours, days, weeks or months). The present invention includes methods which comprise sequentially administering to the patient a single initial dose of an anti-PD-1 antibody, followed by one or more secondary doses of the anti-PD-1 antibody, and optionally followed by one or more tertiary doses of the anti-PD-1 antibody. The anti-PD-1 antibody may be administered at a dose between 0.1 mg/kg to 50 mg/kg.

The terms "initial dose," "secondary doses," and "tertiary doses," refer to the temporal sequence of administration of the anti-PD-1 antibody, or fragment thereof. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of anti-PD-1 antibody, but generally may differ from one another in terms of frequency of administration. In certain embodiments, however, the amount of anti-PD-1 antibody contained in the initial, secondary and/or tertiary doses varies from one another (e.g., adjusted up or down as appropriate) during the course of treatment. In certain embodiments, two or more (e.g., 2, 3, 4, or 5) doses are administered at the beginning of the treatment regimen as "loading doses" followed by subsequent doses that are administered on a less frequent basis (e.g., "maintenance doses").

In certain exemplary embodiments of the present invention, each secondary and/or tertiary dose is administered 1 to 26 (e.g. , 1 , 1 ½, 2, 2½, 3, 3½, 4, 4½, 5, 5½, 6, 6½ , 7, 7/₂, 8, 8½, 9, 9½, 10, 10½, 1 1 , 1 1½, 12, 12½, 13, 13½, 14, 14½, 15, 15½, 16, 16½, 17, 17½, 18, 18½, 19, 19½, 20, 20½, 21 , 21 ½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of anti-PD-1 antibody which is administered to a patient prior to the administration of the very next dose in the sequence with no intervening doses.

The methods according to this aspect of the invention may comprise administering to a patient any number of secondary and/or tertiary doses of an anti-PD-1 antibody. For example, in certain embodiments, only a single secondary dose is administered to the patient. In other embodiments, two or more (e.g., 2, 3, 4, 5, 6, 7, 8, or more) secondary doses are administered to the patient. Likewise, in certain embodiments, only a single tertiary dose is administered to the patient. In other embodiments, two or more (e.g., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2 weeks or 1 to 2 months after the immediately preceding dose. Similarly, in embodiments involving multiple tertiary doses, each tertiary dose may be administered at the same frequency as the other tertiary doses. For example, each tertiary dose may be administered to the patient 2 to 12 weeks after the immediately preceding dose. In certain embodiments of the invention, the frequency at which the secondary and/or tertiary doses are administered to a patient can vary over the course of the treatment regimen. The frequency of administration may also be adjusted during the course of treatment by a physician depending on the needs of the individual patient following clinical examination.

The present invention includes administration regimens in which 2 to 6 loading doses are administered to a patient at a first frequency (e.g., once a week, once every two weeks, once every three weeks, once a month, once every two months, etc.), followed by administration of two or more maintenance doses to the patient on a less frequent basis. For example, according to this aspect of the invention, if the loading doses are administered at a frequency of, e.g., once a month (e.g., two, three, four, or more loading doses administered once a month), then the maintenance doses may be administered to the patient once every five weeks, once every six weeks, once every seven weeks, once every eight weeks, once every ten weeks, once every twelve weeks, etc.).

### Combination Therapies and Formulations

In the context of the methods of treatment described herein, an anti-PD-1 antibody may be administered as a monotherapy (i.e., as the only therapeutic agent) or in combination with at least one additional therapeutic agent (i.e., one or more). Combination therapies may include an anti-PD-1 antibody, or fragment thereof, and any additional therapeutic agent that may be advantageously combined with an anti-PD-1 antibody, or fragment thereof. In certain embodiments, the additional therapeutic agent is useful for treating an HBV infection. In certain embodiments, the at least one additional therapeutic agent is selected from the group consisting of:
a) reverse transcriptase inhibitors;
b) capsid inhibitors;
c) cccDNA formation inhibitors;
d) sAg secretion inhibitors;
e) oligomeric nucleotides targeted to the Hepatitis B genome; and
f) immunostimulators.

### Reverse Transcriptase Inhibitors

In certain embodiments, the reverse transcriptase inhibitor is a nucleoside analog.

In certain embodiments, the reverse transcriptase inhibitor is a nucleoside analog reverse-transcriptase inhibitor (NARTI or NRTI).

In certain embodiments, the reverse transcriptase inhibitor is a nucleotide analog reverse-transcriptase inhibitor (NtARTI or NtRTI).

The term reverse transcriptase inhibitor includes, but is not limited to: entecavir, clevudine, telbivudine, lamivudine, adefovir, and tenofovir, tenofovir disoproxil, tenofovir alafenamide, adefovir dipovoxil, (1R,2R,3R,5R)-3-(6-amino-9H-9-purinyl)-2-fluoro-5-(hydroxymethyl)-4-methylenecyclopentan-1-ol (described in U.S. Patent No. 8,816,074), emtricitabine, abacavir, elvucitabine, ganciclovir, lobucavir, famciclovir, penciclovir, and amdoxovir.

The term reverse transcriptase inhibitor includes, but is not limited to, entecavir, lamivudine, and (1R,2R,3R,5R)-3-(6-amino-9H-9-purinyl)-2-fluoro-5-(hydroxymethyl)-4-methylenecyclopentan-1-ol.

The term reverse transcriptase inhibitor includes, but is not limited to a covalently bound phosphoramidate or phosphonamidate moiety of the above-mentioned reverse transcriptase inhibitors, or as described in, for example, U.S. Patent No. 8,816,074, US 2011/0245484 A1, and US 2008/0286230A1.

The term reverse transcriptase inhibitor includes, but is not limited to, nucleotide analogs that comprise a phosphoramidate moiety, such as, methyl ((((1R,3R,4R,5R)-3-(6-amino-9H-purin-9-yl)-4-fluoro-5-hydroxy-2-methylenecyclopentyl)methoxy)(phenoxy)phosphoryl)-(D or L)-alaninate and methyl ((((1R,2R,3R,4R)-3-fluoro-2-hydroxy-5-methylene-4-(6-oxo-1,6-dihydro-9H-purin-9-yl)cyclopentyl)methoxy)(phenoxy)phosphoryl)-(D or L)-alaninate. Also included are the individual diastereomers thereof, which includes, for example, methyl ((R)-(((1R,3R,4R,5R)-3-(6-amino-9H-purin-9-yl)-4-fluoro-5-hydroxy-2-methylenecyclopentyl)methoxy)(phenoxy)phosphoryl)-(D or L)-alaninate and methyl ((S)-(((1R,3R,4R,5R)-3-(6-amino-9H-purin-9-yl)-4-fluoro-5-hydroxy-2-methylenecyclopentyl)methoxy)(phenoxy)phosphoryl)-(D or L)-alaninate.

The term reverse transcriptase inhibitor includes, but is not limited to a phosphonamidate moiety, such as, tenofovir alafenamide, as well as those described in US 2008/0286230 A1. Methods for preparing stereoselective phosphoramidate or phosphonamidate containing actives are described in, for example, U.S. Patent No. 8,816,074, as well as US 2011/0245484 A1 and US 2008/0286230 A1.

### Capsid Inhibitors

As described herein the term "capsid inhibitor" includes compounds that are capable of inhibiting the expression and/or function of a capsid protein either directly or indirectly. For example, a capsid inhibitor may include, but is not limited to, any compound that inhibits capsid assembly, induces formation of non-capsid polymers, promotes excess capsid assembly or misdirected capsid assembly, affects capsid stabilization, and/or inhibits encapsidation of RNA. Capsid inhibitors also include any compound that inhibits capsid function in a downstream event(s) within the replication process (e.g., viral DNA synthesis, transport of relaxed circular DNA (rcDNA) into the nucleus, covalently closed circular DNA (cccDNA) formation, virus maturation, budding and/or release, and the like). For example, in certain embodiments, the inhibitor detectably inhibits the expression level or biological activity of the capsid protein as measured. In certain embodiments, the inhibitor inhibits the level of rcDNA and downstream products of viral life cycle by at least 5%, at least 10%, at least 20%, at least 50%, at least 75%, or at least 90%.

The term capsid inhibitor includes compounds described in International Patent Applications Publication Numbers WO2013006394, WO2014106019, and WO2014089296, including the following compounds:

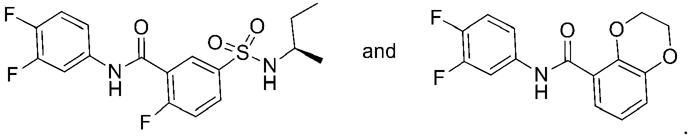

The term capsid inhibitor also includes the compounds **Bay-41-4109** (see International Patent Application Publication Number WO/2013/144129), **AT-61** (see International Patent Application Publication Number WO/1998/33501; and King, RW, et al., Antimicrob Agents Chemother., 1998, 42, 12, 3179-3186), **DVR-01** and **DVR-23** (see International Patent Application Publication Number WO 2013/006394; and Campagna, MR, et al., J. of Virology, 2013, 87, 12, 6931), and pharmaceutically acceptable salts thereof:

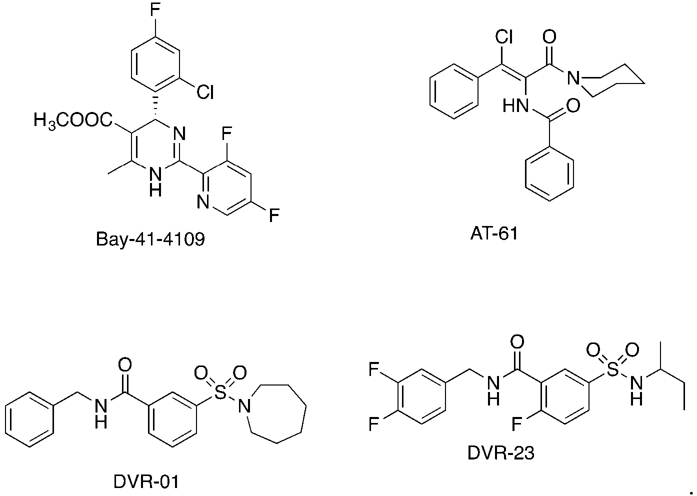

### cccDNA Formation Inhibitors

Covalently closed circular DNA (cccDNA) is generated in the cell nucleus from viral rcDNA and serves as the transcription template for viral mRNAs. As described herein, the term "cccDNA formation inhibitor" includes compounds that are capable of inhibiting the formation and/or stability of cccDNA either directly or indirectly. For example, a cccDNA formation inhibitor may include, but is not limited to, any compound that inhibits capsid disassembly, rcDNA entry into the nucleus, and/or the conversion of rcDNA into cccDNA. For example, in certain embodiments, the inhibitor detectably inhibits the formation and/or stability of the cccDNA as measured. In certain embodiments, the inhibitor inhibits the formation and/or stability of cccDNA by at least 5%, at least 10%, at least 20%, at least 50%, at least 75%, or at least 90%.

The term cccDNA formation inhibitor includes compounds described in International Patent Application Publication Number WO2013130703, including the following compounds:

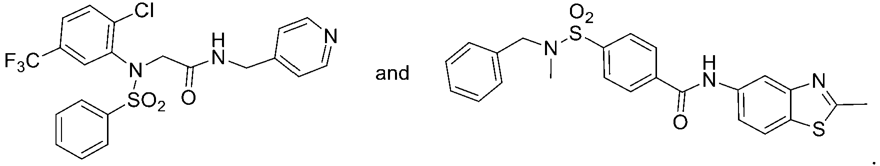

The term cccDNA formation inhibitor includes, but is not limited to those generally and specifically described in United States Patent Application Publication Number US 2015/0038515 A1. The term cccDNA formation inhibitor includes, but is not limited to, 1-(phenylsulfonyl)-N-(pyridin-4-ylmethyl)-1H-indole-2-carboxamide; 1-Benzenesulfonyl-pyrrolidine-2-carboxylic acid (pyridin-4-ylmethyl)-amide; 2-(2-chloro-N-(2-chloro-5-(trifluoromethyl)phenyl)-4-(trifluoromethyl)phenylsulfonamido)-N-(pyridin-4-ylmethyl)acetamide; 2-(4-chloro-N-(2-chloro-5-(trifluoromethyl)phenyl)phenylsulfonamido)-N-(pyridin-4-ylmethyl)acetamide; 2-(N-(2-chloro-5-(trifluoromethyl)phenyl)-4-(trifluoromethyl)phenylsulfonamido)-N-(pyridin-4-ylmethyl)acetamide; 2-(N-(2-chloro-5-(trifluoromethyl)phenyl)-4-methoxyphenylsulfonamido)-N-(pyridin-4-ylmethyl)acetamide; 2-(N-(2-chloro-5-(trifluoromethyl)phenyl)phenylsulfonamido)-N-((1-methylpiperidin-4-yl)methyl)acetamide; 2-(N-(2-chloro-5-(trifluoromethyl)phenyl)phenylsulfonamido)-N-(piperidin-4-ylmethyl)acetamide; 2-(N-(2-chloro-5-(trifluoromethyl)phenyl)phenylsulfonamido)-N-(pyridin-4-ylmethyl)propanamide; 2-(N-(2-chloro-5-(trifluoromethyl)phenyl)phenylsulfonamido)-N-(pyridin-3-ylmethyl)acetamide; 2-(N-(2-chloro-5-(trifluoromethyl)phenyl)phenylsulfonamido)-N-(pyrimidin-5-ylmethyl)acetamide; 2-(N-(2-chloro-5-(trifluoromethyl)phenyl)phenylsulfonamido)-N-(pyrimidin-4-ylmethyl)acetamide; 2-(N-(5-chloro-2-fluorophenyl)phenylsulfonamido)-N-(pyridin-4-ylmethyl)acetamide; 2-[(2-chloro-5-trifluoromethyl-phenyl)-(4-fluoro-benzenesulfonyl)-amino]-N-pyridin-4-ylmethyl-acetamide; 2-[(2-chloro-5-trifluoromethyl-phenyl)-(toluene-4-sulfonyl)-amino]-N-pyridin-4-ylmethyl-acetamide; 2-[benzenesulfonyl-(2-bromo-5-trifluoromethylphenyl)-amino]-N-pyridin-4-ylmethyl-acetamide; 2-[benzenesulfonyl-(2-chloro-5-trifluoromethyl-phenyl)-amino]-N-(2-methyl-benzothiazol-5-yl)-acetamide; 2-[benzenesulfonyl-(2-chloro-5-trifluoromethyl-phenyl)-amino]-N-[4-(4-methyl-piperazin-1-yl)-benzyl]-acetamide; 2-[benzenesulfonyl-(2-chloro-5-trifluoromethyl-phenyl)-amino]-N-[3-(4-methyl-piperazin-1-yl)-benzyl]-acetamide; 2-[benzenesulfonyl-(2-chloro-5-trifluoromethyl-phenyl)-amino]-N-benzyl-acetamide; 2-[benzenesulfonyl-(2-chloro-5-trifluoromethyl-phenyl)-amino]-N-pyridin-4-ylmethyl-acetamide; 2-[benzenesulfonyl-(2-chloro-5-trifluoromethyl-phenyl)-amino]-N-pyridin-4-ylmethyl-propionamide; 2-[benzenesulfonyl-(2-fluoro-5-trifluoromethyl-phenyl)-amino]-N-pyridin-4-ylmethyl-acetamide; 4 (N-(2-chloro-5-(trifluoromethyl)phenyl)phenylsulfonamido)-N-(pyridin-4-yl- methyl)butanamide; 4-((2-(N-(2-chloro-5-(trifluoromethyl)phenyl)phenylsulfonamido)-acetamido)-methyl)-1,1-dimethylpiperidin-1-ium chloride; 4-(benzyl-methyl-sulfamoyl)-N-(2-chloro-5-trifluoromethyl-phenyl)-benzamide; 4-(benzyl-methyl-sulfamoyl)-N-(2-methyl-1H-indol-5-yl)-benzamide; 4-(benzyl-methylsulfamoyl)-N-(2-methyl-1H-indol-5-yl)-benzamide; 4-(benzyl-methyl-sulfamoyl)-N-(2-methyl-benzothiazol-5-yl)-benzamide; 4-(benzyl-methyl-sulfamoyl)-N-(2-methyl-benzothiazol-6-yl)-benzamide; 4-(benzyl-methyl-sulfamoyl)-N-(2-methyl-benzothiazol-6-yl)-benzamide; 4-(benzyl-methyl-sulfamoyl)-N-pyridin-4-ylmethyl-benzamide; N-(2-aminoethyl)-2-(N-(2-chloro-5-(trifluoromethyl)phenyl)phenylsulfonamido)-acetamide; N-(2-chloro-5-(trifluoromethyl)phenyl)-N-(2-(3,4-dihydro-2,6-naphthyridin-2(1H)-yl)-2-oxoethyl)benzenesulfonamide; N-benzothiazol-6-yl-4-(benzyl-methyl-sulfamoyl)-benzamide; N-benzothiazol-6-yl-4-(benzyl-methyl-sulfamoyl)-benzamide; tert-butyl (2-(2-(N-(2-chloro-5-(trifluoromethyl)phenyl)phenylsulfonamido)acetamido)-ethyl)carbamate; and tert-butyl 4-((2-(N-(2-chloro-5-(trifluoromethyl)phenyl)phenylsulfonamido)- acetamido)-methyl)piperidine-1-carboxylate, and optionally, combinations thereof.

### sAg Secretion Inhibitors

As described herein the term "sAg secretion inhibitor" includes compounds that are capable of inhibiting, either directly or indirectly, the secretion of sAg (S, M and/or L surface antigens) bearing subviral particles and/or DNA containing viral particles from HBV-infected cells. For example, in certain embodiments, the inhibitor detectably inhibits the secretion of sAg as measured, e.g., using assays known in the art or described herein, e.g., ELISA assay or by Western Blot. In certain embodiments, the inhibitor inhibits the secretion of sAg by at least 5%, at least 10%, at least 20%, at least 50%, at least 75%, or at least 90%. In certain embodiments, the inhibitor reduces serum levels of sAg in a patient by at least 5%, at least 10%, at least 20%, at least 50%, at least 75%, or at least 90%.

The term sAg secretion inhibitor includes compounds described in United States Patent Number 8,921,381, as well as compounds described in United States Patent Application Publication Numbers 2015/0087659 and 2013/0303552. For example, the term includes the compounds PBHBV-001 and PBHBV-2-15, and pharmaceutically acceptable salts thereof:

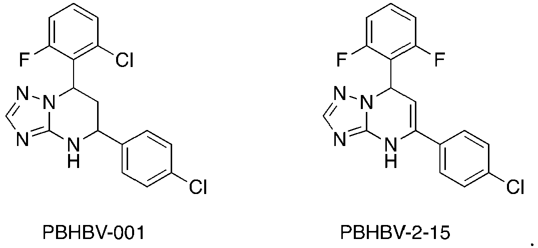

### Immunostimulators

The term "immunostimulator" includes compounds that are capable of modulating an immune response (e.g., stimulate an immune response (e.g., an adjuvant)). The term immunostimulators includes polyinosinic:polycytidylic acid (poly I:C) and interferons.

The term immunostimulators includes agonists of stimulator of IFN genes (STING) and interleukins. The term also includes HBsAg release inhibitors, TLR-7 agonists (GS-9620, RG-7795), T-cell stimulators (GS-4774, TG1050), RIG-1 inhibitors (SB-9200), and SMAC-mimetics (Birinapant).

### Oligomeric Nucleotides

The oligomeric nucleotides can be designed to target one or more genes and/or transcripts of the HBV genome.

The term oligomeric nucleotide targeted to the Hepatitis B genome also includes isolated, double stranded, siRNA molecules, that each include a sense strand and an antisense strand that is hybridized to the sense strand. The siRNA target one or more genes and/or transcripts of the HBV genome.

The term "small-interfering RNA" or "siRNA" as used herein refers to double stranded RNA (*i.e.,* duplex RNA) that is capable of reducing or inhibiting the expression of a target gene or sequence (*e.g.,* by mediating the degradation or inhibiting the translation of mRNAs which are complementary to the siRNA sequence) when the siRNA is in the same cell as the target gene or sequence. The siRNA may have substantial or complete identity to the target gene or sequence, or may comprise a region of mismatch *(i.e.,* a mismatch motif). In certain embodiments, the siRNAs may be about 19-25 (duplex) nucleotides in length, and is preferably about 20-24, 21-22, or 21-23 (duplex) nucleotides in length. siRNA duplexes may comprise 3' overhangs of about 1 to about 4 nucleotides or about 2 to about 3 nucleotides and 5' phosphate termini. Examples of siRNA include, without limitation, a double-stranded polynucleotide molecule assembled from two separate stranded molecules, wherein one strand is the sense strand and the other is the complementary antisense strand.

Preferably, siRNA are chemically synthesized. siRNA can also be generated by cleavage of longer dsRNA (*e.g.,* dsRNA greater than about 25 nucleotides in length) with the *E. coli* RNase III or Dicer. These enzymes process the dsRNA into biologically active siRNA (*see, e.g.,* Yang et al., Proc. Natl. Acad. Sci. USA, 99:9942-9947 (2002); Calegari et al., Proc. Natl. Acad. Sci. USA, 99:14236 (2002); Byrom et al., Ambion TechNotes, 10(1):4-6 (2003); Kawasaki et al., Nucleic Acids Res., 31:981-987 (2003); Knight et al., Science, 293:2269-2271 (2001); and Robertson et al., J. Biol. Chem., 243:82 (1968)). Preferably, dsRNA are at least 50 nucleotides to about 100, 200, 300, 400, or 500 nucleotides in length. A dsRNA may be as long as 1000, 1500, 2000, 5000 nucleotides in length, or longer. The dsRNA can encode for an entire gene transcript or a partial gene transcript. In certain instances, siRNA may be encoded by a plasmid (*e.g.,* transcribed as sequences that automatically fold into duplexes with hairpin loops).

The phrase "inhibiting expression of a target gene" refers to the ability of a siRNA to silence, reduce, or inhibit expression of a target gene *(e.g.,* a gene within the HBV genome). To examine the extent of gene silencing, a test sample (*e.g.,* a biological sample from an organism of interest expressing the target gene or a sample of cells in culture expressing the target gene) is contacted with a siRNA that silences, reduces, or inhibits expression of the target gene. Expression of the target gene in the test sample is compared to expression of the target gene in a control sample (*e.g.,* a biological sample from an organism of interest expressing the target gene or a sample of cells in culture expressing the target gene) that is not contacted with the siRNA. Control samples (*e.g.,* samples expressing the target gene) may be assigned a value of 100%. In particular embodiments, silencing, inhibition, or reduction of expression of a target gene is achieved when the value of the test sample relative to the control sample (*e.g.,* buffer only, an siRNA sequence that targets a different gene, a scrambled siRNA sequence, *etc.)* is about 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 70%, 65%, 60%, 55%, 50%, 45%, 40%, 35%, 30%, 25%, 20%, 15%, 10%, 5%, or 0%. Suitable assays include, without limitation, examination of protein or mRNA levels using techniques known to those of skill in the art, such as, *e.g.,* dot blots, Northern blots, *in situ* hybridization, ELISA, immunoprecipitation, enzyme function, as well as phenotypic assays known to those of skill in the art. An "effective amount" or "therapeutically effective amount" of a therapeutic nucleic acid such as a siRNA is an amount sufficient to produce the desired effect, *e.g.,* an inhibition of expression of a target sequence in comparison to the normal expression level detected in the absence of a siRNA. In particular embodiments, inhibition of expression of a target gene or target sequence is achieved when the value obtained with a siRNA relative to the control (*e.g.,* buffer only, an siRNA sequence that targets a different gene, a scrambled siRNA sequence, *etc*.) is about 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 70%, 65%, 60%, 55%, 50%, 45%, 40%, 35%, 30%, 25%, 20%, 15%, 10%, 5%, or 0%. Suitable assays for measuring the expression of a target gene or target sequence include, but are not limited to, examination of protein or mRNA levels using techniques known to those of skill in the art, such as, *e.g.,* dot blots, Northern blots, *in situ* hybridization, ELISA, immunoprecipitation, enzyme function, as well as phenotypic assays known to those of skill in the art.

The term "nucleic acid" as used herein refers to a polymer containing at least two nucleotides (i.e., deoxyribonucleotides or ribonucleotides) in either single- or double-stranded form and includes DNA and RNA. "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs and/or modified residues include, without limitation, phosphorothioates, phosphoramidates, methyl phosphonates, chiral-methyl phosphonates, 2'-O-methyl ribonucleotides, and peptide-nucleic acids (PNAs). Additionally, nucleic acids can include one or more UNA moieties.

The term "nucleic acid" includes any oligonucleotide or polynucleotide, with fragments containing up to 60 nucleotides generally termed oligonucleotides, and longer fragments termed polynucleotides. A deoxyribooligonucleotide consists of a 5-carbon sugar called deoxyribose joined covalently to phosphate at the 5' and 3' carbons of this sugar to form an alternating, unbranched polymer. DNA may be in the form of, *e.g.,* antisense molecules, plasmid DNA, precondensed DNA, a PCR product, vectors, expression cassettes, chimeric sequences, chromosomal DNA, or derivatives and combinations of these groups. A ribooligonucleotide consists of a similar repeating structure where the 5-carbon sugar is ribose. RNA may be in the form, for example, of small interfering RNA (siRNA), Dicer-substrate dsRNA, small hairpin RNA (shRNA), asymmetrical interfering RNA (aiRNA), microRNA (miRNA), mRNA, tRNA, rRNA, tRNA, viral RNA (vRNA), and combinations thereof. Accordingly, the terms "polynucleotide" and "oligonucleotide" refer to a polymer or oligomer of nucleotide or nucleoside monomers consisting of naturally-occurring bases, sugars and intersugar (backbone) linkages. The terms "polynucleotide" and "oligonucleotide" also include polymers or oligomers comprising non-naturally occurring monomers, or portions thereof, which function similarly. Such modified or substituted oligonucleotides are often preferred over native forms because of properties such as, for example, enhanced cellular uptake, reduced immunogenicity, and increased stability in the presence of nucleases.

Unless otherwise indicated, a particular nucleic acid sequence also implicitly encompasses conservatively modified variants thereof *(*e*.g.,* degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)).

An "isolated" or "purified" DNA molecule or RNA molecule is a DNA molecule or RNA molecule that exists apart from its native environment. An isolated DNA molecule or RNA molecule may exist in a purified form or may exist in a non-native environment such as, for example, a transgenic host cell. For example, an "isolated" or "purified" nucleic acid molecule or biologically active portion thereof, is substantially free of other cellular material, or culture medium when produced by recombinant techniques, or substantially free of chemical precursors or other chemicals when chemically synthesized. In one embodiment, an "isolated" nucleic acid is free of sequences that naturally flank the nucleic acid *(i.e.,* sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived.

The term "gene" refers to a nucleic acid (*e.g.,* DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "unlocked nucleobase analogue" (abbreviated as "UNA") refers to an acyclic nucleobase in which the C2' and C3' atoms of the ribose ring are not covalently linked. The term "unlocked nucleobase analogue" includes nucleobase analogues having the following structure identified as Structure A: 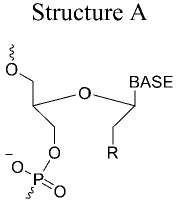 wherein R is hydroxyl, and Base is any natural or unnatural base such as, for example, adenine (A), cytosine (C), guanine (G) and thymine (T). UNA include the molecules identified as acyclic 2'-3'-seco-nucleotide monomers in U.S. patent serial number 8,314,227.

siRNA can be provided in several forms including, e.g., as one or more isolated small-interfering RNA (siRNA) duplexes, as longer double-stranded RNA (dsRNA), or as siRNA or dsRNA transcribed from a transcriptional cassette in a DNA plasmid. In some embodiments, siRNA may be produced enzymatically or by partial/total organic synthesis, and modified ribonucleotides can be introduced by *in vitro* enzymatic or organic synthesis. In certain instances, each strand is prepared chemically. Methods of synthesizing RNA molecules are known in the art, *e.g.,* the chemical synthesis methods as described in Verma and Eckstein (1998) or as described herein.

Methods for isolating RNA, synthesizing RNA, hybridizing nucleic acids, making and screening cDNA libraries, and performing PCR are well known in the art *(see, e.g.,* Gubler and Hoffman, Gene, 25:263-269 (1983); Sambrook *et al., supra;* Ausubel *et al., supra*), as are PCR methods (*see,* U.S. Patent Nos. 4,683,195 and 4,683,202; PCR Protocols: A Guide to Methods and Applications (Innis et al., eds, 1990)). Expression libraries are also well known to those of skill in the art. Additional basic texts disclosing the general methods include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994). The disclosures of these references are herein incorporated by reference in their entirety for all purposes.

Typically, siRNA are chemically synthesized. The oligonucleotides that comprise the siRNA molecules can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). The synthesis of oligonucleotides makes use of common nucleic acid protecting and coupling groups, such as dimethoxytrityl at the 5'-end and phosphoramidites at the 3'-end. As a non-limiting example, small scale syntheses can be conducted on an Applied Biosystems synthesizer using a 0.2 µmol scale protocol. Alternatively, syntheses at the 0.2 µmol scale can be performed on a 96-well plate synthesizer from Protogene (Palo Alto, CA). However, a larger or smaller scale of synthesis is also within the scope. Suitable reagents for oligonucleotide synthesis, methods for RNA deprotection, and methods for RNA purification are known to those of skill in the art.

siRNA molecules can be assembled from two distinct oligonucleotides, wherein one oligonucleotide comprises the sense strand and the other comprises the antisense strand of the siRNA. For example, each strand can be synthesized separately and joined together by hybridization or ligation following synthesis and/or deprotection.

**Formulation and Administration of the at Least One Additional Therapeutic Agent**

The agents can be formulated for and administered using any acceptable route of administration depending on the agent selected. For example, suitable routes include, but are not limited to, oral, sublingual, buccal, topical, transdermal, parenteral, subcutaneous, intraperitoneal, intrapulmonary, and intranasal, and, if desired for local treatment, intralesional administration. In one embodiment, the small molecule agents identified herein can be administered orally. In another embodiment, the oligomeric nucleotides can be administered by injection (e.g., into a blood vessel, such as a vein), or subcutaneously.

Typically, the oligomeric nucleotides targeted to the Hepatitis B genome are administered intravenously, for example in a lipid nanoparticle formulation, however, the present invention is not limited to intravenous formulations comprising the oligomeric nucleotides or to treatment methods wherein an oligomeric nucleotides is administered intravenously.

The agent(s) can be individually formulated by mixing at ambient temperature at the appropriate pH, and at the desired degree of purity, with physiologically acceptable carriers, *i.e.,* carriers that are non-toxic to recipients at the dosages and concentrations employed. The pH of the formulation depends mainly on the particular use and the concentration of compound, but may typically range anywhere from about 3 to about 8. The agents ordinarily will be stored as a solid composition, although lyophilized formulations or aqueous solutions are acceptable.

Compositions comprising the agent(s) can be formulated, dosed, and administered in a fashion consistent with good medical practice. Factors for consideration in this context include the particular disorder being treated, the particular mammal being treated, the clinical condition of the individual patient, the cause of the disorder, the site of administration, the method of administration, the scheduling of administration, and other factors known to medical practitioners.

The agent(s) may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, *e.g.,* diluents, carriers, pH modifiers, sweeteners, bulking agents, and further active agents. If parenteral administration is desired, the compositions will be sterile and in a solution or suspension form suitable for injection or infusion.

Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug or aid in the manufacturing of the pharmaceutical product (*i.e.,* medicament).

The agent(s) is typically dosed at least at a level to reach the desired biological effect. Thus, an effective dosing regimen will dose at least a minimum amount that reaches the desired biological effect, or biologically effective dose, however, the dose should not be so high as to outweigh the benefit of the biological effect with unacceptable side effects. Therefore, an effective dosing regimen will dose no more than the maximum tolerated dose ("MTD"). The maximum tolerated dose is defined as the highest dose that produces an acceptable incidence of dose-limiting toxicities ("DLT"). Doses that cause an unacceptable rate of DLT are considered non-tolerated. Typically, the MTD for a particular schedule is established in phase 1 clinical trials. These are usually conducted in patients by starting at a safe starting dose of 1/10 the severe toxic dose ("STD10") in rodents (on a mg/m² basis) and accruing patients in cohorts of three, escalating the dose according to a modified Fibonacci sequence in which ever higher escalation steps have ever decreasing relative increments (e.g., dose increases of 100%, 65%, 50%, 40%, and 30% to 35% thereafter). The dose escalation is continued in cohorts of three patients until a non-tolerated dose is reached. The next lower dose level that produces an acceptable rate of DLT is considered to be the MTD.

The amount of the agent(s) administered will depend upon the particular agent used, the strain of HBV being treated, the age, weight, and condition of the patient, and the judgment of the clinician, but will generally be between about 0.2 to 2.0 grams per day.

### Administration Regimen of Combination Therapies

It will be understood that the anti-PD-1 antibody, or fragment thereof, and the at least one additional therapeutic agent can be formulated together in a single preparation or that they can be formulated separately and, thus, administered separately, either simultaneously or sequentially (the agent(s) may be administered prior to or after the administration of the anti-PD-1 antibody or fragment thereof). In one embodiment, when the agents are administered sequentially (e.g. at different times), the agents may be administered so that their biological effects overlap (i.e. each agent is producing a biological effect at a single given time). For purposes of the present disclosure, such administration regimens are considered the administration of an anti-PD-1 antibody "in combination with" at least one additional therapeutic agent or active component.

The at least one additional therapeutic agent (additional component) may be administered to a subject prior to administration of an anti-PD-1 antibody. For example, a first component may be deemed to be administered "prior to" a second component if the first component is administered 1 week before, 72 hours before, 60 hours before, 48 hours before, 36 hours before, 24 hours before, 12 hours before, 6 hours before, 5 hours before, 4 hours before, 3 hours before, 2 hours before, 1 hour before, 30 minutes before, 15 minutes before, 10 minutes before, 5 minutes before, or less than 1 minute before administration of the second component. In other embodiments, the at least one additional therapeutic agent/component may be administered to a subject after administration of an anti-PD-1 antibody. For example, a first component may be deemed to be administered "after" a second component if the first component is administered 1 minute after, 5 minutes after, 10 minutes after, 15 minutes after, 30 minutes after, 1 hour after, 2 hours after, 3 hours after, 4 hours after, 5 hours after, 6 hours after, 12 hours after, 24 hours after, 36 hours after, 48 hours after, 60 hours after, 72 hours after administration of the second component. In yet other embodiments, the at least one additional therapeutic agent may be administered to a subject concurrent with administration of an anti-PD-1 antibody. "Concurrent" administration, for purposes of the present invention, includes, e.g., administration of an anti-PD-1 antibody and the at least one additional therapeutic agent to a subject in a single dosage form (e.g., co-formulated), or in separate dosage forms administered to the subject within about 30 minutes or less of each other. If administered in separate dosage forms, each dosage form may be administered via the same route (e.g., both the anti-PD-1 antibody and the additional therapeutically active component may be administered orally, intravenously, subcutaneously, etc.); alternatively, each dosage form may be administered via a different route (e.g., the anti-PD-1 antibody may be administered orally or intravenously, and the additional therapeutically active component may be administered subcutaneously). In any event, administering the components in a single dosage form, in separate dosage forms by the same route, or in separate dosage forms by different routes are all considered "concurrent administration," for purposes of the present disclosure. For purposes of the present disclosure, administration of an anti-PD-1 antibody "prior to", "concurrent with," or "after" (as those terms are defined herein above) administration of an additional therapeutic agent is considered administration of an anti-PD-1 antibody "in combination with" an additional therapeutic agent.

As described herein, pharmaceutical compositions in which an anti-PD-1 antibody is co-formulated with at least one additional therapeutic agent using a variety of dosage combinations may also be used. Accordingly, certain embodiments of the invention provide a composition comprising an anti-PD-1 antibody, or a fragment thereof, and at least one additional therapeutic agent, and a carrier. Certain embodiments of the invention also provide a pharmaceutical composition comprising an anti-PD-1 antibody, or a fragment thereof, and at least one additional therapeutic agent, and a pharmaceutically acceptable carrier.

### Kits Comprising Combination Therapies

One embodiment also provides a kit comprising an anti-PD-1 antibody, or a fragment thereof, and at least one additional therapeutic agent. The kit may comprise a container comprising the combination. Suitable containers include, for example, bottles, vials, syringes, blister pack, etc. The container may be formed from a variety of materials such as glass or plastic. The container may hold the combination which is effective for treating the condition and may have a sterile access port (for example, the container may be an intravenous solution bag or a vial having a stopper pierceable by a hypodermic injection needle).

The kit may further comprise a label or package insert on or associated with the container. The term "package insert" is used to refer to instructions customarily included in commercial packages of therapeutic products, that contain information about the indications, usage, dosage, administration, contraindications and/or warnings concerning the use of such therapeutic products. In one embodiment, the label or package inserts indicates that the composition comprising an anti-PD-1 antibody, or a fragment thereof, and the at least one additional therapeutic agent can be used to treat a viral infection, such as Hepatitis B.

According to another embodiment, a kit may comprise (a) a first container with an anti-PD-1 antibody, or a fragment thereof, contained therein; and (b) a second container with the at least one additional therapeutic agent contained therein. Alternatively, or additionally, the kit may further comprise a third container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of the an anti-PD-1 antibody, or a fragment thereof, and the at least one additional therapeutic agent. For example, the kit may further comprise directions for the simultaneous, sequential or separate administration of the anti-PD-1 antibody, or a fragment thereof, and the at least one additional therapeutic agent to a patient in need thereof.

In certain other embodiments, the kit may comprise a container for containing the separate compositions such as a divided bottle or a divided foil packet, however, the separate compositions may also be contained within a single, undivided container. In certain embodiments, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

Another aspect of the present invention provides a kit comprising separate containers of a an anti-PD-1 antibody, or a fragment thereof, and at least one additional therapeutic agent for use in combination to treat or prevent a viral infection, such as Hepatitis B.

Another aspect of the present invention provides a kit comprising separate containers in a single package pharmaceutical composition for use in combination to treat or prevent a viral infection, such as Hepatitis B, which comprises in one container a pharmaceutical composition comprising an effective amount of an anti-PD-1 antibody, or a fragment thereof, and in a second container a pharmaceutical composition comprising at least one additional therapeutic agent.

Another embodiment provides a kit comprising:
(a) an anti-PD-1 antibody, or a fragment thereof, and
(b) at least one additional therapeutic agent, for use in combination to treat or prevent a viral infection, such as Hepatitis B.

Another embodiment provides a kit comprising:
(a) a pharmaceutical composition comprising an anti-PD-1 antibody, or a fragment thereof, and a pharmaceutically acceptable diluent or carrier, and
(b) a pharmaceutical composition comprising a at least one additional therapeutic agent and a pharmaceutically acceptable diluent or carrier, for use in combination to treat or prevent a viral infection, such as Hepatitis B.

The ability of a composition described herein to treat Hepatitis B may be determined using pharmacological models which are well known to the art.

### Carrier Systems Containing Oligomeric Nucleotide(s)

As discussed herein, oligomeric nucleotides targeted to the Hepatitis B genome (i.e., nucleic acid molecules, such as siRNA molecules) may be administered intravenously, for example in a lipid nanoparticle formulation. In certain embodiments, the at least one oligomeric nucleotide is administered via a nucleic acid-lipid particle, wherein the nucleic acid lipid particle comprises: (a) the at least one oligomeric nucleotide; (b) a cationic lipid; and (c) a non-cationic lipid.

The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

The term "lipid particle" includes a lipid formulation that can be used to deliver an oligomeric nucleotide (*i.e.,* a nucleic acid, such as a siRNA) to a target site of interest (*e.g.,* cell, tissue, organ, and the like). In preferred embodiments, the lipid particle of the invention is typically formed from a cationic lipid, a non-cationic lipid, and optionally a conjugated lipid that prevents aggregation of the particle. A lipid particle that includes an oligomeric nucleotide (*e.g.,* siRNA molecule) is referred to as a nucleic acid-lipid particle. Typically, the oligomeric nucleotide is fully encapsulated within the lipid particle, thereby protecting the nucleic acid from enzymatic degradation.

In certain instances, nucleic acid-lipid particles are extremely useful for systemic applications, as they can exhibit extended circulation lifetimes following intravenous (i.v.) injection, they can accumulate at distal sites *(*e*.g.,* sites physically separated from the administration site), and they can mediate silencing of target gene expression at these distal sites. The nucleic acid may be complexed with a condensing agent and encapsulated within a lipid particle as set forth in PCT Publication No. WO 00/03683, the disclosure of which is herein incorporated by reference in its entirety for all purposes.

The lipid particles can comprise one or more oligomeric nucleotides (e.g., one or more siRNA), a cationic lipid, a non-cationic lipid, and a conjugated lipid that inhibits aggregation of particles. In some embodiments, the oligomeric nucleotide (e.g., siRNA molecule) is fully encapsulated within the lipid portion of the lipid particle such that the oligomeric nucleotide in the lipid particle is resistant in aqueous solution to nuclease degradation. In other embodiments, the lipid particles described herein are substantially non-toxic to mammals such as humans. The lipid particles typically have a mean diameter of from about 30 nm to about 150 nm, from about 40 nm to about 150 nm, from about 50 nm to about 150 nm, from about 60 nm to about 130 nm, from about 70 nm to about 110 nm, or from about 70 to about 90 nm. In certain embodiments, the lipid particles have a median diameter of from about 30 nm to about 150 nm. The lipid particles also typically have a lipid:nucleic acid ratio (*e.g.,* a lipid:siRNA ratio) (mass/mass ratio) of from about 1:1 to about 100:1, from about 1:1 to about 50:1, from about 2:1 to about 25:1, from about 3:1 to about 20:1, from about 5:1 to about 15:1, or from about 5:1 to about 10:1. In certain embodiments, the nucleic acid-lipid particle has a lipid:siRNA mass ratio of from about 5:1 to about 15:1.

The lipid particles include serum-stable nucleic acid-lipid particles which comprise one or more oligomeric nucleotides (e.g., one or more siRNA), a cationic lipid (*e.g.,* one or more cationic lipids of Formula I-III or salts thereof as set forth herein), a non-cationic lipid (*e.g.,* mixtures of one or more phospholipids and cholesterol), and a conjugated lipid that inhibits aggregation of the particles (*e.g.,* one or more PEG-lipid conjugates). The lipid particle may comprise at least 1, 2, 3, 4, 5, 6, 7, 8, 9, 10, or more siRNA molecules that target one or more of the genes described herein. Nucleic acid-lipid particles and their method of preparation are described in, *e.g.,* U.S. Patent Nos. 5,753,613; 5,785,992; 5,705,385; 5,976,567; 5,981,501; 6,110,745; and 6,320,017; and PCT Publication No. WO 96/40964, the disclosures of which are each herein incorporated by reference in their entirety for all purposes.

In the nucleic acid-lipid particles, the one or more oligomeric nucleotides (e.g., one or more siRNA) may be fully encapsulated within the lipid portion of the particle, thereby protecting the oligomeric nucleotide from nuclease degradation. In certain instances, the nucleic acid in the nucleic acid-lipid particle is not substantially degraded after exposure of the particle to a nuclease at 37°C for at least about 20, 30, 45, or 60 minutes. In certain other instances, the nucleic acid in the nucleic acid-lipid particle is not substantially degraded after incubation of the particle in serum at 37°C for at least about 30, 45, or 60 minutes or at least about 2, 3, 4, 5, 6, 7, 8, 9, 10, 12, 14, 16, 18, 20, 22, 24, 26, 28, 30, 32, 34, or 36 hours. In other embodiments, the nucleic acid is complexed with the lipid portion of the particle. One of the benefits of the formulations is that the nucleic acid-lipid particle compositions are substantially non-toxic to mammals such as humans.

The lipid particles typically have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, and are substantially non-toxic. In addition, nucleic acids, when present in the lipid particles of the present invention, are resistant in aqueous solution to degradation with a nuclease. Nucleic acid-lipid particles and their method of preparation are disclosed in, *e.g.,* U.S. Patent Publication Nos. 20040142025 and 20070042031, the disclosures of which are herein incorporated by reference in their entirety for all purposes.

As used herein, "lipid encapsulated" can refer to a lipid particle that provides a oligomeric nucleotide such as a siRNA, with full encapsulation, partial encapsulation, or both. In a preferred embodiment, the oligomeric nucleotide (*e.g.,* siRNA) is fully encapsulated in the lipid particle (*e.g.,* to form a nucleic acid-lipid particle).

The term "fully encapsulated" indicates that the oligomeric nucleotide (*e.g.,* a siRNA molecule) in the nucleic acid-lipid particle is not significantly degraded after exposure to serum or a nuclease assay that would significantly degrade free DNA or RNA. In a fully encapsulated system, preferably less than about 25% of the nucleic acid in the particle is degraded in a treatment that would normally degrade 100% of free nucleic acid, more preferably less than about 10%, and most preferably less than about 5% of the nucleic acid in the particle is degraded. "Fully encapsulated" also indicates that the nucleic acid-lipid particles are serum-stable, that is, that they do not rapidly decompose into their component parts upon *in vivo* administration.

In the context of nucleic acids, full encapsulation may be determined by performing a membrane-impermeable fluorescent dye exclusion assay, which uses a dye that has enhanced fluorescence when associated with nucleic acid. Specific dyes such as OliGreen^{®} and RiboGreen^{®} (Invitrogen Corp.; Carlsbad, CA) are available for the quantitative determination of plasmid DNA, single-stranded deoxyribonucleotides, and/or single- or double-stranded ribonucleotides. Encapsulation is determined by adding the dye to a liposomal formulation, measuring the resulting fluorescence, and comparing it to the fluorescence observed upon addition of a small amount of nonionic detergent. Detergent-mediated disruption of the liposomal bilayer releases the encapsulated nucleic acid, allowing it to interact with the membrane-impermeable dye. Nucleic acid encapsulation may be calculated as *E* = *(Iₒ - I)*/*Iₒ,* where *I and Iₒ* refer to the fluorescence intensities before and after the addition of detergent (*see,* Wheeler et al., Gene Ther., 6:271-281 (1999)).

In some instances, the nucleic acid-lipid particle composition comprises an oligomeric nucleotide (e.g., a siRNA molecule) that is fully encapsulated within the lipid portion of the particles, such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the particles have the oligomeric nucleotide (e.g., siRNA) encapsulated therein.

In other instances, the nucleic acid-lipid particle composition comprises an oligomeric nucleotide (e.g., siRNA) that is fully encapsulated within the lipid portion of the particles, such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the input oligomeric nucleotide (e.g., siRNA) is encapsulated in the particles.

Depending on the intended use of the lipid particles, the proportions of the components can be varied and the delivery efficiency of a particular formulation can be measured using, *e.g.,* an endosomal release parameter (ERP) assay.

The term "lipid conjugate" refers to a conjugated lipid that inhibits aggregation of lipid particles. Such lipid conjugates include, but are not limited to, PEG-lipid conjugates such as, *e.g.,* PEG coupled to dialkyloxypropyls *(e.g.,* PEG-DAA conjugates), PEG coupled to diacylglycerols (*e.g.,* PEG-DAG conjugates), PEG coupled to cholesterol, PEG coupled to phosphatidylethanolamines, and PEG conjugated to ceramides (*see, e.g.,* U.S. Patent No. 5,885,613), cationic PEG lipids, polyoxazoline (POZ)-lipid conjugates (*e.g.,* POZ-DAA conjugates), polyamide oligomers (*e.g.,* ATTA-lipid conjugates), and mixtures thereof. Additional examples of POZ-lipid conjugates are described in PCT Publication No. WO 2010/006282. PEG or POZ can be conjugated directly to the lipid or may be linked to the lipid via a linker moiety. Any linker moiety suitable for coupling the PEG or the POZ to a lipid can be used including, *e.g.,* non-ester containing linker moieties and ester-containing linker moieties. In certain preferred embodiments, non-ester containing linker moieties, such as amides or carbamates, are used.

The term "amphipathic lipid" refers, in part, to any suitable material wherein the hydrophobic portion of the lipid material orients into a hydrophobic phase, while the hydrophilic portion orients toward the aqueous phase. Hydrophilic characteristics derive from the presence of polar or charged groups such as carbohydrates, phosphate, carboxylic, sulfato, amino, sulfhydryl, nitro, hydroxyl, and other like groups. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). Examples of amphipathic compounds include, but are not limited to, phospholipids, aminolipids, and sphingolipids.

Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine. Other compounds lacking in phosphorus, such as sphingolipid, glycosphingolipid families, diacylglycerols, and β-acyloxyacids, are also within the group designated as amphipathic lipids. Additionally, the amphipathic lipids described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides, and diacylglycerols.

The term "non-cationic lipid" refers to any amphipathic lipid as well as any other neutral lipid or anionic lipid.

The term "anionic lipid" refers to any lipid that is negatively charged at physiological pH. These lipids include, but are not limited to, phosphatidylglycerols, cardiolipins, diacylphosphatidylserines, diacylphosphatidic acids, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

The term "hydrophobic lipid" refers to compounds having apolar groups that include, but are not limited to, long-chain saturated and unsaturated aliphatic hydrocarbon groups and such groups optionally substituted by one or more aromatic, cycloaliphatic, or heterocyclic group(s). Suitable examples include, but are not limited to, diacylglycerol, dialkylglycerol, N-N-dialkylamino, 1,2-diacyloxy-3-aminopropane, and 1,2-dialkyl-3-aminopropane.

The terms "cationic lipid" and "amino lipid" are used interchangeably herein to include those lipids and salts thereof having one, two, three, or more fatty acid or fatty alkyl chains and a pH-titratable amino head group (*e.g.,* an alkylamino or dialkylamino head group). The cationic lipid is typically protonated (*i.e.,* positively charged) at a pH below the pKₐ of the cationic lipid and is substantially neutral at a pH above the pKₐ. The cationic lipids of the invention may also be termed titratable cationic lipids. In some embodiments, the cationic lipids comprise: a protonatable tertiary amine (*e.g.,* pH-titratable) head group; C₁₈ alkyl chains, wherein each alkyl chain independently has 0 to 3 (*e.g.,* 0, 1, 2, or 3) double bonds; and ether, ester, or ketal linkages between the head group and alkyl chains. Such cationic lipids include, but are not limited to, DSDMA, DODMA, DLinDMA, DLenDMA, γ-DLenDMA, DLin-K-DMA, DLin-K-C2-DMA (also known as DLin-C2K-DMA, XTC2, and C2K), DLin-K-C3-DMA, DLin-K-C4-DMA, DLen-C2K-DMA, γ-DLen-C2K-DMA, DLin-M-C2-DMA (also known as MC2), and DLin-M-C3-DMA (also known as MC3).

The term "salts" includes any anionic and cationic complex, such as the complex formed between a cationic lipid and one or more anions. Non-limiting examples of anions include inorganic and organic anions, *e.g.,* hydride, fluoride, chloride, bromide, iodide, oxalate (*e.g.,* hemioxalate), phosphate, phosphonate, hydrogen phosphate, dihydrogen phosphate, oxide, carbonate, bicarbonate, nitrate, nitrite, nitride, bisulfite, sulfide, sulfite, bisulfate, sulfate, thiosulfate, hydrogen sulfate, borate, formate, acetate, benzoate, citrate, tartrate, lactate, acrylate, polyacrylate, fumarate, maleate, itaconate, glycolate, gluconate, malate, mandelate, tiglate, ascorbate, salicylate, polymethacrylate, perchlorate, chlorate, chlorite, hypochlorite, bromate, hypobromite, iodate, an alkylsulfonate, an aryl sulfonate, arsenate, arsenite, chromate, dichromate, cyanide, cyanate, thiocyanate, hydroxide, peroxide, permanganate, and mixtures thereof. In particular embodiments, the salts of the cationic lipids disclosed herein are crystalline salts.

The term "alkyl" includes a straight chain or branched, noncyclic or cyclic, saturated aliphatic hydrocarbon containing from 1 to 24 carbon atoms. Representative saturated straight chain alkyls include, but are not limited to, methyl, ethyl, *n*-propyl, *n*-butyl, *n*-pentyl, *n*-hexyl, and the like, while saturated branched alkyls include, without limitation, isopropyl, *sec*-butyl, isobutyl, *tert*-butyl, isopentyl, and the like. Representative saturated cyclic alkyls include, but are not limited to, cyclopropyl, cyclobutyl, cyclopentyl, cyclohexyl, and the like, while unsaturated cyclic alkyls include, without limitation, cyclopentenyl, cyclohexenyl, and the like.

The term "alkenyl" includes an alkyl, as defined above, containing at least one double bond between adjacent carbon atoms. Alkenyls include both *cis* and *trans* isomers. Representative straight chain and branched alkenyls include, but are not limited to, ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

The term "alkynyl" includes any alkyl or alkenyl, as defined above, which additionally contains at least one triple bond between adjacent carbons. Representative straight chain and branched alkynyls include, without limitation, acetylenyl, propynyl, 1-butynyl, 2-butynyl, 1-pentynyl, 2-pentynyl, 3-methyl-1 butynyl, and the like.

The term "acyl" includes any alkyl, alkenyl, or alkynyl wherein the carbon at the point of attachment is substituted with an oxo group, as defined below. The following are non-limiting examples of acyl groups: -C(=O)alkyl, -C(=O)alkenyl, and -C(=O)alkynyl.

The term "heterocycle" includes a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include, but are not limited to, heteroaryls as defined below, as well as morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" mean that, when substituted, at least one hydrogen atom is replaced with a substituent. In the case of an oxo substituent (=O), two hydrogen atoms are replaced. In this regard, substituents include, but are not limited to, oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, R^{x} and R^{y} are the same or different and are independently hydrogen, alkyl, or heterocycle, and each of the alkyl and heterocycle substituents may be further substituted with one or more of oxo, halogen, -OH, -CN, alkyl, -OR^{x}, heterocycle, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}. The term "optionally substituted," when used before a list of substituents, means that each of the substituents in the list may be optionally substituted as described herein.

The term "halogen" includes fluoro, chloro, bromo, and iodo.

The term "fusogenic" refers to the ability of a lipid particle to fuse with the membranes of a cell. The membranes can be either the plasma membrane or membranes surrounding organelles, *e.g.,* endosome, nucleus, *etc.*

As used herein, the term "aqueous solution" refers to a composition comprising in whole, or in part, water.

As used herein, the term "organic lipid solution" refers to a composition comprising in whole, or in part, an organic solvent having a lipid.

The term "electron dense core", when used to describe a lipid particle of the present invention, refers to the dark appearance of the interior portion of a lipid particle when visualized using cryo transmission electron microscopy ("cyroTEM"). Some lipid particles of the present invention have an electron dense core and lack a lipid bilayer structure. Some lipid particles of the present invention have an electron dense core, lack a lipid bilayer structure, and have an inverse Hexagonal or Cubic phase structure. While not wishing to be bound by theory, it is thought that the non-bilayer lipid packing provides a 3-dimensional network of lipid cylinders with water and nucleic acid on the inside, i.e., essentially a lipid droplet interpenetrated with aqueous channels containing the nucleic acid.

"Distal site," as used herein, refers to a physically separated site, which is not limited to an adjacent capillary bed, but includes sites broadly distributed throughout an organism.

"Serum-stable" in relation to nucleic acid-lipid particles means that the particle is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of lipid particles that leads to a broad biodistribution of an active agent such as an oligomeric nucleotide (e.g., a siRNA) within an organism. Some techniques of administration can lead to the systemic delivery of certain agents, but not others. Systemic delivery means that a useful, preferably therapeutic, amount of an agent is exposed to most parts of the body. To obtain broad biodistribution generally requires a blood lifetime such that the agent is not rapidly degraded or cleared (such as by first pass organs (liver, lung, *etc.)* or by rapid, nonspecific cell binding) before reaching a disease site distal to the site of administration. Systemic delivery of lipid particles can be by any means known in the art including, for example, intravenous, subcutaneous, and intraperitoneal. In a preferred embodiment, systemic delivery of lipid particles is by intravenous delivery.

"Local delivery," as used herein, refers to delivery of an active agent such as an oligomeric nucleotide (e.g., a siRNA) directly to a target site within an organism. For example, an agent can be locally delivered by direct injection into a disease site, other target site, or a target organ such as the liver, heart, pancreas, kidney, and the like.

### Cationic Lipids

Any of a variety of cationic lipids or salts thereof may be used in the lipid particles of the present invention either alone or in combination with one or more other cationic lipid species or non-cationic lipid species. The cationic lipids include the (R) and/or (S) enantiomers thereof.

In one aspect of the invention, the cationic lipid is a dialkyl lipid. For example, dialkyl lipids may include lipids that comprise two saturated or unsaturated alkyl chains, wherein each of the alkyl chains may be substituted or unsubstituted. In certain embodiments, each of the two alkyl chains comprise at least, e.g., 8 carbon atoms, 10 carbon atoms, 12 carbon atoms, 14 carbon atoms, 16 carbon atoms, 18 carbon atoms, 20 carbon atoms, 22 carbon atoms or 24 carbon atoms.

In one aspect of the invention, the cationic lipid is a trialkyl lipid. For example, trialkyl lipids may include lipids that comprise three saturated or unsaturated alkyl chains, wherein each of the alkyl chains may be substituted or unsubstituted. In certain embodiments, each of the three alkyl chains comprise at least, e.g., 8 carbon atoms, 10 carbon atoms, 12 carbon atoms, 14 carbon atoms, 16 carbon atoms, 18 carbon atoms, 20 carbon atoms, 22 carbon atoms or 24 carbon atoms.

In one aspect, cationic lipids of Formula I having the following structure are useful in the present invention: 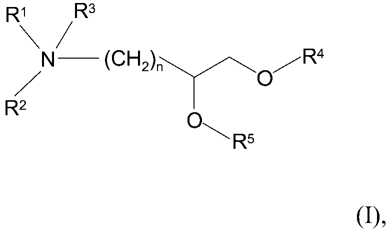 or salts thereof, wherein:
R¹ and R² are either the same or different and are independently hydrogen (H) or an optionally substituted C₁-C₆ alkyl, C₂-C₆ alkenyl, or C₂-C₆ alkynyl, or R¹ and R² may join to form an optionally substituted heterocyclic ring of 4 to 6 carbon atoms and 1 or 2 heteroatoms selected from the group consisting of nitrogen (N), oxygen (O), and mixtures thereof;
R³ is either absent or is hydrogen (H) or a C₁-C₆ alkyl to provide a quaternary amine;
R⁴ and R⁵ are either the same or different and are independently an optionally substituted C₁₀-C₂₄ alkyl, C₁₀-C₂₄ alkenyl, C₁₀-C₂₄ alkynyl, or C₁₀-C₂₄ acyl, wherein at least one of R⁴ and R⁵ comprises at least two sites of unsaturation; and
n is 0, 1, 2, 3, or 4.

In some embodiments, R¹ and R² are independently an optionally substituted C₁-C₄ alkyl, C₂-C₄ alkenyl, or C₂-C₄ alkynyl. In one preferred embodiment, R¹ and R² are both methyl groups. In other preferred embodiments, n is 1 or 2. In other embodiments, R³ is absent when the pH is above the pKₐ of the cationic lipid and R³ is hydrogen when the pH is below the pKₐ of the cationic lipid such that the amino head group is protonated. In an alternative embodiment, R³ is an optionally substituted C₁-C₄ alkyl to provide a quaternary amine. In further embodiments, R⁴ and R⁵ are independently an optionally substituted C₁₂-C₂₀ or C₁₄-C₂₂ alkyl, C₁₂-C₂₀ or C₁₄-C₂₂ alkenyl, C₁₂-C₂₀ or C₁₄-C₂₂ alkynyl, or C₁₂-C₂₀ or C₁₄-C₂₂ acyl, wherein at least one of R⁴ and R³ comprises at least two sites of unsaturation.

In certain embodiments, R⁴ and R⁵ are independently selected from the group consisting of a dodecadienyl moiety, a tetradecadienyl moiety, a hexadecadienyl moiety, an octadecadienyl moiety, an icosadienyl moiety, a dodecatrienyl moiety, a tetradectrienyl moiety, a hexadecatrienyl moiety, an octadecatrienyl moiety, an icosatrienyl moiety, an arachidonyl moiety, and a docosahexaenoyl moiety, as well as acyl derivatives thereof (*e.g.,* linoleoyl, linolenoyl, γ-linolenoyl, *etc*.). In some instances, one of R⁴ and R⁵ comprises a branched alkyl group (*e.g.,* a phytanyl moiety) or an acyl derivative thereof (*e.g.,* a phytanoyl moiety). In certain instances, the octadecadienyl moiety is a linoleyl moiety. In certain other instances, the octadecatrienyl moiety is a linolenyl moiety or a γ-linolenyl moiety. In certain embodiments, R⁴ and R⁵ are both linoleyl moieties, linolenyl moieties, or γ-linolenyl moieties. In particular embodiments, the cationic lipid of Formula I is 1,2-dilinoleyloxy-N,N-dimethylaminopropane (DLinDMA), 1,2-dilinolenyloxy-N,N-dimethylaminopropane (DLenDMA), 1,2-dilinoleyloxy-(N,N-dimethyl)-butyl-4-amine (C2-DLinDMA), 1,2-dilinoleoyloxy-(N,N-dimethyl)-butyl-4-amine (C2-DLinDAP), or mixtures thereof.

In some embodiments, the cationic lipid of Formula I forms a salt (preferably a crystalline salt) with one or more anions. In one particular embodiment, the cationic lipid of Formula I is the oxalate (*e*.*g*., hemioxalate) salt thereof, which is preferably a crystalline salt.

The synthesis of cationic lipids such as DLinDMA and DLenDMA, as well as additional cationic lipids, is described in U.S. Patent Publication No. 20060083780, the disclosure of which is herein incorporated by reference in its entirety for all purposes. The synthesis of cationic lipids such as C2-DLinDMA and C2-DLinDAP, as well as additional cationic lipids, is described in international patent application number WO2011/000106 the disclosure of which is herein incorporated by reference in its entirety for all purposes.

In another aspect, cationic lipids of Formula II having the following structure (or salts thereof) are useful in the present invention: 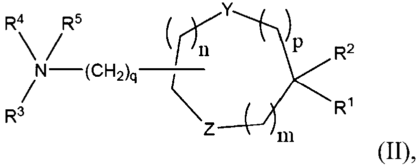 wherein R¹ and R² are either the same or different and are independently an optionally substituted C₁₂-C₂₄ alkyl, C₁₂-C₂₄ alkenyl, C₁₂-C₂₄ alkynyl, or C₁₂-C₂₄ acyl; R³ and R⁴ are either the same or different and are independently an optionally substituted C₁-C₆ alkyl, C₂-C₆ alkenyl, or C₂-C₆ alkynyl, or R³ and R⁴ may join to form an optionally substituted heterocyclic ring of 4 to 6 carbon atoms and 1 or 2 heteroatoms chosen from nitrogen and oxygen; R⁵ is either absent or is hydrogen (H) or a C₁-C₆ alkyl to provide a quaternary amine; m, n, and p are either the same or different and are independently either 0, 1, or 2, with the proviso that m, n, and p are not simultaneously 0; q is 0, 1, 2, 3, or 4; and Y and Z are either the same or different and are independently O, S, or NH. In a preferred embodiment, q is 2.

In some embodiments, the cationic lipid of Formula II is 2,2-dilinoleyl-4-(2-dimethylaminoethyl)-[1,3]-dioxolane (DLin-K-C2-DMA; "XTC2" or "C2K"), 2,2-dilinoleyl-4-(3-dimethylaminopropyl)-[1,3]-dioxolane (DLin-K-C3-DMA; "C3K"), 2,2-dilinoleyl-4-(4-dimethylaminobutyl)-[1,3]-dioxolane (DLin-K-C4-DMA; "C4K"), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dilinoleyl-4-dimethylaminomethyl-[1,3]-dioxolane (DLin-K-DMA), 2,2-dioleoyl-4-dimethylaminomethyl-[1,3]-dioxolane (DO-K-DMA), 2,2-distearoyl-4-dimethylaminomethyl-[1,3]-dioxolane (DS-K-DMA), 2,2-dilinoleyl-4-N-morpholino-[1,3]-dioxolane (DLin-K-MA), 2,2-Dilinoleyl-4-trimethylamino-[1,3]-dioxolane chloride (DLin-K-TMA.Cl), 2,2-dilinoleyl-4,5-bis(dimethylaminomethyl)-[1,3]-dioxolane (DLin-K²-DMA), 2,2-dilinoleyl-4-methylpiperzine-[1,3]-dioxolane (D-Lin-K-N-methylpiperzine), or mixtures thereof. In preferred embodiments, the cationic lipid of Formula II is DLin-K-C2-DMA.

In some embodiments, the cationic lipid of Formula II forms a salt (preferably a crystalline salt) with one or more anions. In one particular embodiment, the cationic lipid of Formula II is the oxalate (*e*.*g*., hemioxalate) salt thereof, which is preferably a crystalline salt.

The synthesis of cationic lipids such as DLin-K-DMA, as well as additional cationic lipids, is described in PCT Publication No. WO 09/086558, the disclosure of which is herein incorporated by reference in its entirety for all purposes. The synthesis of cationic lipids such as DLin-K-C2-DMA, DLin-K-C3-DMA, DLin-K-C4-DMA, DLin-K6-DMA, DLin-K-MPZ, DO-K-DMA, DS-K-DMA, DLin-K-MA, DLin-K-TMA.Cl, DLin-K²-DMA, and D-Lin-K-N-methylpiperzine, as well as additional cationic lipids, is described in PCT Application No. PCT/US2009/060251, entitled "Improved Amino Lipids and Methods for the Delivery of Nucleic Acids," filed October 9, 2009, the disclosure of which is incorporated herein by reference in its entirety for all purposes.

In a further aspect, cationic lipids of Formula III having the following structure are useful in the present invention: 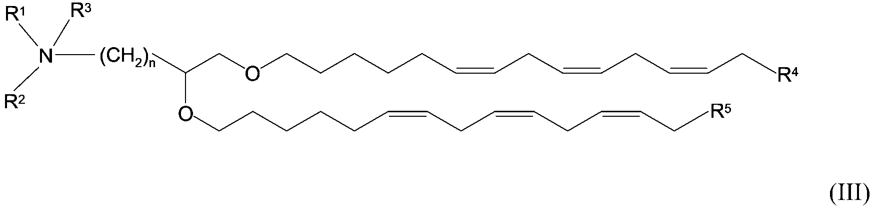 or salts thereof, wherein: R¹ and R² are either the same or different and are independently an optionally substituted C₁-C₆ alkyl, C₂-C₆ alkenyl, or C₂-C₆ alkynyl, or R¹ and R² may join to form an optionally substituted heterocyclic ring of 4 to 6 carbon atoms and 1 or 2 heteroatoms selected from the group consisting of nitrogen (N), oxygen (O), and mixtures thereof; R³ is either absent or is hydrogen (H) or a C₁-C₆ alkyl to provide a quaternary amine; R⁴ and R⁵ are either absent or present and when present are either the same or different and are independently an optionally substituted C₁-C₁₀ alkyl or C₂-C₁₀ alkenyl; and n is 0, 1, 2, 3, or 4.

In some embodiments, R¹ and R² are independently an optionally substituted C₁-C₄ alkyl, C₂-C₄ alkenyl, or C₂-C₄ alkynyl. In a preferred embodiment, R¹ and R² are both methyl groups. In another preferred embodiment, R⁴ and R⁵ are both butyl groups. In yet another preferred embodiment, n is 1. In other embodiments, R³ is absent when the pH is above the pKₐ of the cationic lipid and R³ is hydrogen when the pH is below the pKₐ of the cationic lipid such that the amino head group is protonated. In an alternative embodiment, R³ is an optionally substituted C₁-C₄ alkyl to provide a quaternary amine. In further embodiments, R⁴ and R⁵ are independently an optionally substituted C₂-C₆ or C₂-C₄ alkyl or C₂-C₆ or C₂-C₄ alkenyl.

In an alternative embodiment, the cationic lipid of Formula III comprises ester linkages between the amino head group and one or both of the alkyl chains. In some embodiments, the cationic lipid of Formula III forms a salt (preferably a crystalline salt) with one or more anions. In one particular embodiment, the cationic lipid of Formula III is the oxalate (*e*.*g*., hemioxalate) salt thereof, which is preferably a crystalline salt.

Although each of the alkyl chains in Formula III contains *cis* double bonds at positions 6, 9, and 12 (*i.e., cis,cis,cis*-Δ⁶*,*Δ⁹*,*Δ¹²)*,* in an alternative embodiment, one, two, or three of these double bonds in one or both alkyl chains may be in the *trans* configuration.

In a particularly preferred embodiment, the cationic lipid of Formula III has the structure:

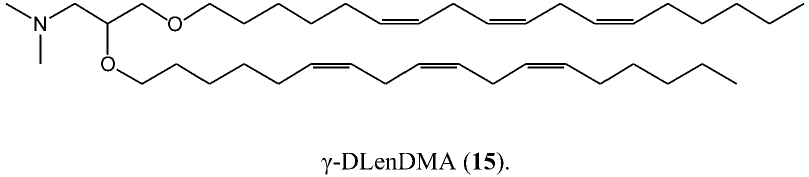

The synthesis of cationic lipids such as γ-DLenDMA **(15),** as well as additional cationic lipids, is described in U.S. Provisional Application No. 61/222,462, entitled "Improved Cationic Lipids and Methods for the Delivery of Nucleic Acids," filed July 1, 2009, the disclosure of which is herein incorporated by reference in its entirety for all purposes.

The synthesis of cationic lipids such as DLin-M-C3-DMA ("MC3"), as well as additional cationic lipids (*e*.*g*., certain analogs of MC3), is described in U.S. Provisional Application No. 61/185,800, entitled "Novel Lipids and Compositions for the Delivery of Therapeutics," filed June 10, 2009, and U.S. Provisional Application No. 61/287,995, entitled "Methods and Compositions for Delivery of Nucleic Acids," filed December 18, 2009, the disclosures of which are herein incorporated by reference in their entirety for all purposes.

Examples of other cationic lipids or salts thereof which may be included in the lipid particles of the present invention include, but are not limited to, cationic lipids such as those described in WO2011/000106, the disclosure of which is herein incorporated by reference in its entirety for all purposes, as well as cationic lipids such as N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3 -(N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethy-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), 1,2-dioeylcarbamoyloxy-3-dimethylaminopropane (DO-C-DAP), 1,2-dimyristoleoyl-3-dimethylaminopropane (DMDAP), 1,2-dioleoyl-3-trimethylaminopropane chloride (DOTAP.Cl), dilinoleylmethyl-3-dimethylaminopropionate (DLin-M-C2-DMA; also known as DLin-M-K-DMA or DLin-M-DMA), and mixtures thereof. Additional cationic lipids or salts thereof which may be included in the lipid particles of the present invention are described in U.S. Patent Publication No. 20090023673, the disclosure of which is herein incorporated by reference in its entirety for all purposes.

The synthesis of cationic lipids such as CLinDMA, as well as additional cationic lipids, is described in U.S. Patent Publication No. 20060240554, the disclosure of which is herein incorporated by reference in its entirety for all purposes. The synthesis of cationic lipids such as DLin-C-DAP, DLinDAC, DLinMA, DLinDAP, DLin-S-DMA, DLin-2-DMAP, DLinTMA.Cl, DLinTAP.Cl, DLinMPZ, DLinAP, DOAP, and DLin-EG-DMA, as well as additional cationic lipids, is described in PCT Publication No. WO 09/086558, the disclosure of which is herein incorporated by reference in its entirety for all purposes. The synthesis of cationic lipids such as DO-C-DAP, DMDAP, DOTAP.Cl, DLin-M-C2-DMA, as well as additional cationic lipids, is described in PCT Application No. PCT/US2009/060251, entitled "Improved Amino Lipids and Methods for the Delivery of Nucleic Acids," filed October 9, 2009, the disclosure of which is incorporated herein by reference in its entirety for all purposes. The synthesis of a number of other cationic lipids and related analogs has been described in U.S. Patent Nos. 5,208,036; 5,264,618; 5,279,833; 5,283,185; 5,753,613; and 5,785,992; and PCT Publication No. WO 96/10390, the disclosures of which are each herein incorporated by reference in their entirety for all purposes. Additionally, a number of commercial preparations of cationic lipids can be used, such as, *e.g*., LIPOFECTIN^{®} (including DOTMA and DOPE, available from Invitrogen); LIPOFECTAMINE^{®} (including DOSPA and DOPE, available from Invitrogen); and TRANSFECTAM^{®} (including DOGS, available from Promega Corp.).

In some embodiments, the cationic lipid comprises from about 50 mol % to about 90 mol %, from about 50 mol % to about 85 mol %, from about 50 mol % to about 80 mol %, from about 50 mol % to about 75 mol %, from about 50 mol % to about 70 mol %, from about 50 mol % to about 65 mol %, from about 50 mol % to about 60 mol %, from about 55 mol % to about 65 mol %, or from about 55 mol % to about 70 mol % (or any fraction thereof or range therein) of the total lipid present in the particle. In particular embodiments, the cationic lipid comprises about 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, or 65 mol % (or any fraction thereof) of the total lipid present in the particle.

In other embodiments, the cationic lipid comprises from about 2 mol % to about 60 mol %, from about 5 mol % to about 50 mol %, from about 10 mol % to about 50 mol %, from about 20 mol % to about 50 mol %, from about 20 mol % to about 40 mol %, from about 30 mol % to about 40 mol %, or about 40 mol % (or any fraction thereof or range therein) of the total lipid present in the particle.

Additional percentages and ranges of cationic lipids suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335, the disclosures of which are herein incorporated by reference in their entirety for all purposes.

It should be understood that the percentage of cationic lipid present in the lipid particles of the invention is a target amount, and that the actual amount of cationic lipid present in the formulation may vary, for example, by ± 5 mol %. For example, in one exemplary lipid particle formulation, the target amount of cationic lipid is 57.1 mol %, but the actual amount of cationic lipid may be ± 5 mol %, ± 4 mol %, ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle; however, one skilled in the art will understand that the total mol % may deviate slightly from 100% due to rounding, for example, 99.9 mol % or 100.1 mol %.).

Further examples of cationic lipids useful for inclusion in lipid particles used in the present invention are shown below: 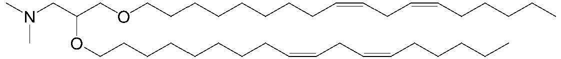
N,N-dimethyl-2,3-bis((9Z,12Z)-octadeca-9,12-dienyloxy)propan-1-amine **(5)** 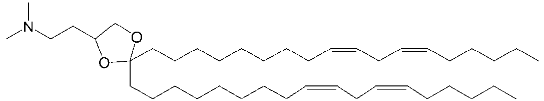
2-(2,2-di((9Z,12Z)-octadeca-9,12-dienyl)-1,3-dioxolan-4-yl)-N,N-dimethylethanamine **(6)** 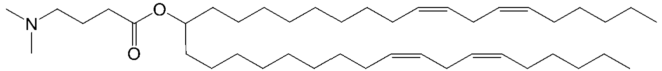
(6Z,9Z,28Z,31Z)-heptatriaconta-6,9,28,31-tetraen-19-yl 4-(dimethylamino)butanoate **(7)** 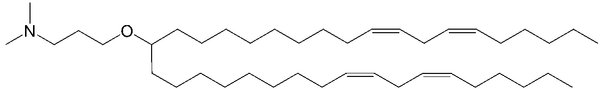
3-((6Z,9Z,28Z,31Z)-heptatriaconta-6,9,28,31-tetraen-19-yloxy)-N,N-dimethylpropan-1-amine **(8)** 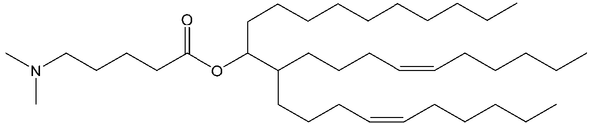
(Z)-12-((Z)-dec-4-enyl)docos-16-en-11-yl 5-(dimethylamino)pentanoate **(53)** 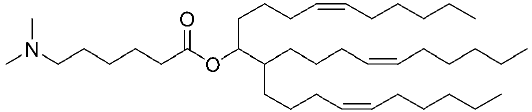
(6Z,16Z)-12-((Z)-dec-4-enyl)docosa-6,16-dien-11-yl 6-(dimethylamino)hexanoate (**11**) 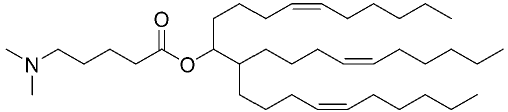
(6Z,16Z)-12-((Z)-dec-4-enyl)docosa-6,16-dien-11-yl 5-(dimethylamino)pentanoate **(13)** 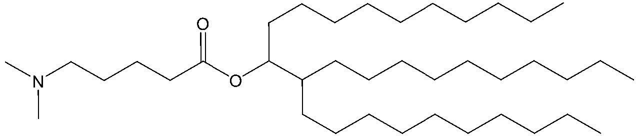
12-decyldocosan-11-yl 5-(dimethylamino)pentanoate **(14).**

### Non-cationic Lipids

The non-cationic lipids used in the lipid particles of the invention can be any of a variety of neutral uncharged, zwitterionic, or anionic lipids capable of producing a stable complex.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having C₁₀-C₂₄ carbon chains, *e.g*., lauroyl, myristoyl, palmitoyl, stearoyl, or oleoyl.

Additional examples of non-cationic lipids include sterols such as cholesterol and derivatives thereof. Non-limiting examples of cholesterol derivatives include polar analogues such as 5α-cholestanol, 5β-coprostanol, cholesteryl-(2'-hydroxy)-ethyl ether, cholesteryl-(4'-hydroxy)-butyl ether, and 6-ketocholestanol; non-polar analogues such as 5α-cholestane, cholestenone, 5α-cholestanone, 5β-cholestanone, and cholesteryl decanoate; and mixtures thereof. In preferred embodiments, the cholesterol derivative is a polar analogue such as cholesteryl-(4'-hydroxy)-butyl ether. The synthesis of cholesteryl-(2'-hydroxy)-ethyl ether is described in PCT Publication No. WO 09/127060, the disclosure of which is herein incorporated by reference in its entirety for all purposes.

In some embodiments, the non-cationic lipid present in the lipid particles comprises or consists of a mixture of one or more phospholipids and cholesterol or a derivative thereof. In other embodiments, the non-cationic lipid present in the lipid particles comprises or consists of one or more phospholipids, *e.g.,* a cholesterol-free lipid particle formulation. In yet other embodiments, the non-cationic lipid present in the lipid particles comprises or consists of cholesterol or a derivative thereof, *e.g.,* a phospholipid-free lipid particle formulation.

Other examples of non-cationic lipids suitable for use in the present invention include nonphosphorous containing lipids such as, *e.g*., stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide, ceramide, sphingomyelin, and the like.

In some embodiments, the non-cationic lipid comprises from about 10 mol % to about 60 mol %, from about 20 mol % to about 55 mol %, from about 20 mol % to about 45 mol %, from about 20 mol % to about 40 mol %, from about 25 mol % to about 50 mol %, from about 25 mol % to about 45 mol %, from about 30 mol % to about 50 mol %, from about 30 mol % to about 45 mol %, from about 30 mol % to about 40 mol %, from about 35 mol % to about 45 mol %, from about 37 mol % to about 45 mol %, or about 35 mol %, 36 mol %, 37 mol %, 38 mol %, 39 mol %, 40 mol %, 41 mol %, 42 mol %, 43 mol %, 44 mol %, or 45 mol % (or any fraction thereof or range therein) of the total lipid present in the particle.

In embodiments where the lipid particles contain a mixture of phospholipid and cholesterol or a cholesterol derivative, the mixture may comprise up to about 40 mol %, 45 mol %, 50 mol %, 55 mol %, or 60 mol % of the total lipid present in the particle.

In some embodiments, the phospholipid component in the mixture may comprise from about 2 mol % to about 20 mol %, from about 2 mol % to about 15 mol %, from about 2 mol % to about 12 mol %, from about 4 mol % to about 15 mol %, or from about 4 mol % to about 10 mol % (or any fraction thereof or range therein) of the total lipid present in the particle. In an certain embodiments, the phospholipid component in the mixture comprises from about 5 mol % to about 17 mol %, from about 7 mol % to about 17 mol %, from about 7 mol % to about 15 mol %, from about 8 mol % to about 15 mol %, or about 8 mol %, 9 mol %, 10 mol %, 11 mol %, 12 mol %, 13 mol %, 14 mol %, or 15 mol % (or any fraction thereof or range therein) of the total lipid present in the particle. As a non-limiting example, a lipid particle formulation comprising a mixture of phospholipid and cholesterol may comprise a phospholipid such as DPPC or DSPC at about 7 mol % (or any fraction thereof), *e.g.,* in a mixture with cholesterol or a cholesterol derivative at about 34 mol % (or any fraction thereof) of the total lipid present in the particle. As another non-limiting example, a lipid particle formulation comprising a mixture of phospholipid and cholesterol may comprise a phospholipid such as DPPC or DSPC at about 7 mol % (or any fraction thereof), *e.g*., in a mixture with cholesterol or a cholesterol derivative at about 32 mol % (or any fraction thereof) of the total lipid present in the particle.

By way of further example, a lipid formulation useful in the practice of the invention has a lipid to drug *(e.g.,* siRNA) ratio of about 10:1 (*e.g.,* a lipid:drug ratio of from 9.5:1 to 11:1, or from 9.9:1 to 11:1, or from 10:1 to 10.9:1). In certain other embodiments, a lipid formulation useful in the practice of the invention has a lipid to drug (*e.g.,* siRNA) ratio of about 9:1 (*e.g.,* a lipid:drug ratio of from 8.5:1 to 10:1, or from 8.9:1 to 10:1, or from 9:1 to 9.9:1, including 9.1:1, 9.2:1, 9.3:1, 9.4:1, 9.5:1, 9.6:1, 9.7:1, and 9.8:1).

In other embodiments, the cholesterol component in the mixture may comprise from about 25 mol % to about 45 mol %, from about 25 mol % to about 40 mol %, from about 30 mol % to about 45 mol %, from about 30 mol % to about 40 mol %, from about 27 mol % to about 37 mol %, from about 25 mol % to about 30 mol %, or from about 35 mol % to about 40 mol % (or any fraction thereof or range therein) of the total lipid present in the particle. In certain preferred embodiments, the cholesterol component in the mixture comprises from about 25 mol % to about 35 mol %, from about 27 mol % to about 35 mol %, from about 29 mol % to about 35 mol %, from about 30 mol % to about 35 mol %, from about 30 mol % to about 34 mol %, from about 31 mol % to about 33 mol %, or about 30 mol %, 31 mol %, 32 mol %, 33 mol %, 34 mol %, or 35 mol % (or any fraction thereof or range therein) of the total lipid present in the particle.

In embodiments where the lipid particles are phospholipid-free, the cholesterol or derivative thereof may comprise up to about 25 mol %, 30 mol %, 35 mol %, 40 mol %, 45 mol %, 50 mol %, 55 mol %, or 60 mol % of the total lipid present in the particle.

In some embodiments, the cholesterol or derivative thereof in the phospholipid-free lipid particle formulation may comprise from about 25 mol % to about 45 mol %, from about 25 mol % to about 40 mol %, from about 30 mol % to about 45 mol %, from about 30 mol % to about 40 mol %, from about 31 mol % to about 39 mol %, from about 32 mol % to about 38 mol %, from about 33 mol % to about 37 mol %, from about 35 mol % to about 45 mol %, from about 30 mol % to about 35 mol %, from about 35 mol % to about 40 mol %, or about 30 mol %, 31 mol %, 32 mol %, 33 mol %, 34 mol %, 35 mol %, 36 mol %, 37 mol %, 38 mol %, 39 mol %, or 40 mol % (or any fraction thereof or range therein) of the total lipid present in the particle. As a non-limiting example, a lipid particle formulation may comprise cholesterol at about 37 mol % (or any fraction thereof) of the total lipid present in the particle. As another non-limiting example, a lipid particle formulation may comprise cholesterol at about 35 mol % (or any fraction thereof) of the total lipid present in the particle.

In other embodiments, the non-cationic lipid comprises from about 5 mol % to about 90 mol %, from about 10 mol % to about 85 mol %, from about 20 mol % to about 80 mol %, about 10 mol % (*e.g.,* phospholipid only), or about 60 mol % (*e.g.,* phospholipid and cholesterol or derivative thereof) (or any fraction thereof or range therein) of the total lipid present in the particle.

Additional percentages and ranges of non-cationic lipids suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335, the disclosures of which are herein incorporated by reference in their entirety for all purposes.

It should be understood that the percentage of non-cationic lipid present in the lipid particles of the invention is a target amount, and that the actual amount of non-cationic lipid present in the formulation may vary, for example, by ± 5 mol %, ± 4 mol %, ± 3 mol %, ± 2 mol %, ± 1 mol %, ± 0.75 mol %, ± 0.5 mol %, ± 0.25 mol %, or ± 0.1 mol %.

### Lipid Conjugates

In addition to cationic and non-cationic lipids, the lipid particles of the invention may further comprise a lipid conjugate. The conjugated lipid is useful in that it prevents the aggregation of particles. Suitable conjugated lipids include, but are not limited to, PEG-lipid conjugates, POZ-lipid conjugates, ATTA-lipid conjugates, cationic-polymer-lipid conjugates (CPLs), and mixtures thereof. In certain embodiments, the particles comprise either a PEG-lipid conjugate or an ATTA-lipid conjugate together with a CPL.

In a preferred embodiment, the lipid conjugate is a PEG-lipid. Examples of PEG-lipids include, but are not limited to, PEG coupled to dialkyloxypropyls (PEG-DAA) as described in, *e.g.,* PCT Publication No. WO 05/026372, PEG coupled to diacylglycerol (PEG-DAG) as described in, *e.g.,* U.S. Patent Publication Nos. 20030077829 and 2005008689, PEG coupled to phospholipids such as phosphatidylethanolamine (PEG-PE), PEG conjugated to ceramides as described in, *e.g.,* U.S. Patent No. 5,885,613, PEG conjugated to cholesterol or a derivative thereof, and mixtures thereof. The disclosures of these patent documents are herein incorporated by reference in their entirety for all purposes.

Additional PEG-lipids suitable for use in the invention include, without limitation, mPEG2000-1,2-di-O-alkyl-*sn*3-carbomoylglyceride (PEG-C-DOMG). The synthesis of PEG-C-DOMG is described in PCT Publication No. WO 09/086558, the disclosure of which is herein incorporated by reference in its entirety for all purposes. Yet additional suitable PEG-lipid conjugates include, without limitation, 1-[8'-(1,2-dimyristoyl-3-propanoxy)-carboxamido-3',6'-dioxaoctanyl]carbamoyl-ω-methyl-poly(ethylene glycol) (2KPEG-DMG). The synthesis of 2KPEG-DMG is described in U.S. Patent No. 7,404,969, the disclosure of which is herein incorporated by reference in its entirety for all purposes.

PEG is a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, but are not limited to, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), monomethoxypolyethylene glycol-imidazolyl-carbonyl (MePEG-IM), as well as such compounds containing a terminal hydroxyl group instead of a terminal methoxy group (*e.g.,* HO-PEG-S, HO-PEG-S-NHS, HO-PEG-NH₂, *etc*.). Other PEGs such as those described in U.S. Patent Nos. 6,774,180 and 7,053,150 (*e.g.,* mPEG (20 KDa) amine) are also useful for preparing the PEG-lipid conjugates of the present invention. The disclosures of these patents are herein incorporated by reference in their entirety for all purposes. In addition, monomethoxypolyethyleneglycol-acetic acid (MePEG-CH₂COOH) is particularly useful for preparing PEG-lipid conjugates including, *e*.*g*., PEG-DAA conjugates.

The PEG moiety of the PEG-lipid conjugates described herein may comprise an average molecular weight ranging from about 550 daltons to about 10,000 daltons. In certain instances, the PEG moiety has an average molecular weight of from about 750 daltons to about 5,000 daltons (*e.g*., from about 1,000 daltons to about 5,000 daltons, from about 1,500 daltons to about 3,000 daltons, from about 750 daltons to about 3,000 daltons, from about 750 daltons to about 2,000 daltons, *etc*.). In preferred embodiments, the PEG moiety has an average molecular weight of about 2,000 daltons or about 750 daltons.

In certain instances, the PEG can be optionally substituted by an alkyl, alkoxy, acyl, or aryl group. The PEG can be conjugated directly to the lipid or may be linked to the lipid via a linker moiety. Any linker moiety suitable for coupling the PEG to a lipid can be used including, *e*.*g*., non-ester containing linker moieties and ester-containing linker moieties. In a preferred embodiment, the linker moiety is a non-ester containing linker moiety. As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, as well as combinations thereof (such as a linker containing both a carbamate linker moiety and an amido linker moiety). In a preferred embodiment, a carbamate linker is used to couple the PEG to the lipid.

In other embodiments, an ester containing linker moiety is used to couple the PEG to the lipid. Suitable ester containing linker moieties include, *e*.*g*., carbonate (-OC(O)O-), succinoyl, phosphate esters (-O-(O)POH-O-), sulfonate esters, and combinations thereof.

Phosphatidylethanolamines having a variety of acyl chain groups of varying chain lengths and degrees of saturation can be conjugated to PEG to form the lipid conjugate. Such phosphatidylethanolamines are commercially available, or can be isolated or synthesized using conventional techniques known to those of skill in the art. Phosphatidyl-ethanolamines containing saturated or unsaturated fatty acids with carbon chain lengths in the range of C₁₀ to C₂₀ are preferred. Phosphatidylethanolamines with mono- or diunsaturated fatty acids and mixtures of saturated and unsaturated fatty acids can also be used. Suitable phosphatidylethanolamines include, but are not limited to, dimyristoyl-phosphatidylethanolamine (DMPE), dipalmitoyl-phosphatidylethanolamine (DPPE), dioleoylphosphatidylethanolamine (DOPE), and distearoyl-phosphatidylethanolamine (DSPE).

The term "ATTA" or "polyamide" includes, without limitation, compounds described in U.S. Patent Nos. 6,320,017 and 6,586,559, the disclosures of which are herein incorporated by reference in their entirety for all purposes. These compounds include a compound having the formula: 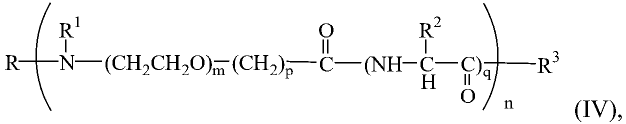 wherein R is a member selected from the group consisting of hydrogen, alkyl and acyl; R¹ is a member selected from the group consisting of hydrogen and alkyl; or optionally, R and R¹ and the nitrogen to which they are bound form an azido moiety; R² is a member of the group selected from hydrogen, optionally substituted alkyl, optionally substituted aryl and a side chain of an amino acid; R³ is a member selected from the group consisting of hydrogen, halogen, hydroxy, alkoxy, mercapto, hydrazino, amino and NR⁴R⁵, wherein R⁴ and R⁵ are independently hydrogen or alkyl; n is 4 to 80; m is 2 to 6; p is 1 to 4; and q is 0 or 1. It will be apparent to those of skill in the art that other polyamides can be used in the compounds of the present invention.

The term "diacylglycerol" or "DAG" includes a compound having 2 fatty acyl chains, R¹ and R², both of which have independently between 2 and 30 carbons bonded to the 1- and 2-position of glycerol by ester linkages. The acyl groups can be saturated or have varying degrees of unsaturation. Suitable acyl groups include, but are not limited to, lauroyl (C₁₂), myristoyl (C₁₄), palmitoyl (C₁₆), stearoyl (C₁₈), and icosoyl (C₂₀). In preferred embodiments, R¹ and R² are the same, *i.e.,* R¹ and R² are both myristoyl (*i.e.,* dimyristoyl), R¹ and R² are both stearoyl *(i.e.,* distearoyl), *etc.* Diacylglycerols have the following general formula:

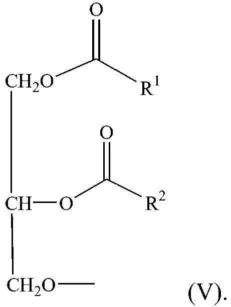

The term "dialkyloxypropyl" or "DAA" includes a compound having 2 alkyl chains, R¹ and R², both of which have independently between 2 and 30 carbons. The alkyl groups can be saturated or have varying degrees of unsaturation. Dialkyloxypropyls have the following general formula:

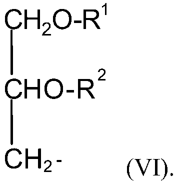

In a preferred embodiment, the PEG-lipid is a PEG-DAA conjugate having the following formula: 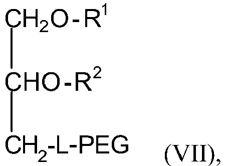 wherein R¹ and R² are independently selected and are long-chain alkyl groups having from about 10 to about 22 carbon atoms; PEG is a polyethyleneglycol; and L is a non-ester containing linker moiety or an ester containing linker moiety as described above. The long-chain alkyl groups can be saturated or unsaturated. Suitable alkyl groups include, but are not limited to, decyl (C₁₀), lauryl (C₁₂), myristyl (C₁₄), palmityl (C₁₆), stearyl (C₁₈), and icosyl (C₂₀). In preferred embodiments, R¹ and R² are the same, *i.e.,* R¹ and R² are both myristyl (*i.e*., dimyristyl), R¹ and R² are both stearyl (*i.e.,* distearyl), *etc.*

In Formula VII above, the PEG has an average molecular weight ranging from about 550 daltons to about 10,000 daltons. In certain instances, the PEG has an average molecular weight of from about 750 daltons to about 5,000 daltons (*e.g*., from about 1,000 daltons to about 5,000 daltons, from about 1,500 daltons to about 3,000 daltons, from about 750 daltons to about 3,000 daltons, from about 750 daltons to about 2,000 daltons, *etc.).* In preferred embodiments, the PEG has an average molecular weight of about 2,000 daltons or about 750 daltons. The PEG can be optionally substituted with alkyl, alkoxy, acyl, or aryl groups. In certain embodiments, the terminal hydroxyl group is substituted with a methoxy or methyl group.

In a preferred embodiment, "L" is a non-ester containing linker moiety. Suitable non-ester containing linkers include, but are not limited to, an amido linker moiety, an amino linker moiety, a carbonyl linker moiety, a carbamate linker moiety, a urea linker moiety, an ether linker moiety, a disulphide linker moiety, a succinamidyl linker moiety, and combinations thereof. In a preferred embodiment, the non-ester containing linker moiety is a carbamate linker moiety (*i.e*., a PEG-C-DAA conjugate). In another preferred embodiment, the non-ester containing linker moiety is an amido linker moiety (*i.e.,* a PEG-A-DAA conjugate). In yet another preferred embodiment, the non-ester containing linker moiety is a succinamidyl linker moiety (*i.e.,* a PEG-S-DAA conjugate).

In particular embodiments, the PEG-lipid conjugate is selected from: 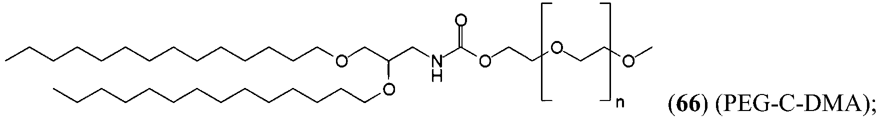 and

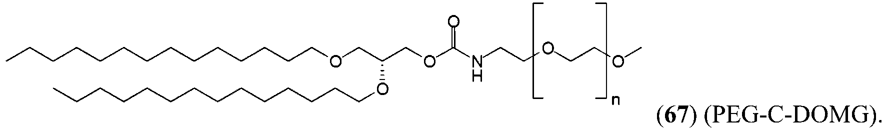

The PEG-DAA conjugates are synthesized using standard techniques and reagents known to those of skill in the art. It will be recognized that the PEG-DAA conjugates will contain various amide, amine, ether, thio, carbamate, and urea linkages. Those of skill in the art will recognize that methods and reagents for forming these bonds are well known and readily available. *See, e.g.,* March, ADVANCED ORGANIC CHEMISTRY (Wiley 1992); Larock, COMPREHENSIVE ORGANIC TRANSFORMATIONS (VCH 1989); and Furniss, VOGEL'S TEXTBOOK OF PRACTICAL ORGANIC CHEMISTRY, 5th ed. (Longman 1989). It will also be appreciated that any functional groups present may require protection and deprotection at different points in the synthesis of the PEG-DAA conjugates. Those of skill in the art will recognize that such techniques are well known. *See, e.g.,* Green and Wuts, PROTECTIVE GROUPS IN ORGANIC SYNTHESIS (Wiley 1991).

Preferably, the PEG-DAA conjugate is a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, or a PEG-distearyloxypropyl (C₁₈) conjugate. In these embodiments, the PEG preferably has an average molecular weight of about 750 or about 2,000 daltons. In one particularly preferred embodiment, the PEG-lipid conjugate comprises PEG2000-C-DMA, wherein the "2000" denotes the average molecular weight of the PEG, the "C" denotes a carbamate linker moiety, and the "DMA" denotes dimyristyloxypropyl. In another particularly preferred embodiment, the PEG-lipid conjugate comprises PEG750-C-DMA, wherein the "750" denotes the average molecular weight of the PEG, the "C" denotes a carbamate linker moiety, and the "DMA" denotes dimyristyloxypropyl. In particular embodiments, the terminal hydroxyl group of the PEG is substituted with a methyl group. Those of skill in the art will readily appreciate that other dialkyloxypropyls can be used in the PEG-DAA conjugates of the present invention.

In addition to the foregoing, it will be readily apparent to those of skill in the art that other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses such as hydroxymethylcellulose or hydroxyethylcellulose.

In addition to the foregoing components, the lipid particles of the present invention can further comprise cationic poly(ethylene glycol) (PEG) lipids or CPLs (*see, e.g.,* Chen et al., Bioconj. Chem., 11:433-437 (2000); U.S. Patent No. 6,852,334; PCT Publication No. WO 00/62813, the disclosures of which are herein incorporated by reference in their entirety for all purposes).

Suitable CPLs include compounds of Formula VIII:

A-W-Y (VIII),

wherein A, W, and Y are as described below.

With reference to Formula VIII, "A" is a lipid moiety such as an amphipathic lipid, a neutral lipid, or a hydrophobic lipid that acts as a lipid anchor. Suitable lipid examples include, but are not limited to, diacylglycerolyls, dialkylglycerolyls, N-N-dialkylaminos, 1,2-diacyloxy-3-aminopropanes, and 1,2-dialkyl-3-aminopropanes.

"W" is a polymer or an oligomer such as a hydrophilic polymer or oligomer. Preferably, the hydrophilic polymer is a biocompatable polymer that is nonimmunogenic or possesses low inherent immunogenicity. Alternatively, the hydrophilic polymer can be weakly antigenic if used with appropriate adjuvants. Suitable nonimmunogenic polymers include, but are not limited to, PEG, polyamides, polylactic acid, polyglycolic acid, polylactic acid/polyglycolic acid copolymers, and combinations thereof. In a preferred embodiment, the polymer has a molecular weight of from about 250 to about 7,000 daltons.

"Y" is a polycationic moiety. The term polycationic moiety refers to a compound, derivative, or functional group having a positive charge, preferably at least 2 positive charges at a selected pH, preferably physiological pH. Suitable polycationic moieties include basic amino acids and their derivatives such as arginine, asparagine, glutamine, lysine, and histidine; spermine; spermidine; cationic dendrimers; polyamines; polyamine sugars; and amino polysaccharides. The polycationic moieties can be linear, such as linear tetralysine, branched or dendrimeric in structure. Polycationic moieties have between about 2 to about 15 positive charges, preferably between about 2 to about 12 positive charges, and more preferably between about 2 to about 8 positive charges at selected pH values. The selection of which polycationic moiety to employ may be determined by the type of particle application which is desired.

The charges on the polycationic moieties can be either distributed around the entire particle moiety, or alternatively, they can be a discrete concentration of charge density in one particular area of the particle moiety *e.g.,* a charge spike. If the charge density is distributed on the particle, the charge density can be equally distributed or unequally distributed. All variations of charge distribution of the polycationic moiety are encompassed by the present invention.

The lipid "A" and the nonimmunogenic polymer "W" can be attached by various methods and preferably by covalent attachment. Methods known to those of skill in the art can be used for the covalent attachment of "A" and "W." Suitable linkages include, but are not limited to, amide, amine, carboxyl, carbonate, carbamate, ester, and hydrazone linkages. It will be apparent to those skilled in the art that "A" and "W" must have complementary functional groups to effectuate the linkage. The reaction of these two groups, one on the lipid and the other on the polymer, will provide the desired linkage. For example, when the lipid is a diacylglycerol and the terminal hydroxyl is activated, for instance with NHS and DCC, to form an active ester, and is then reacted with a polymer which contains an amino group, such as with a polyamide (*see, e.g.,* U.S. Patent Nos. 6,320,017 and 6,586,559, the disclosures of which are herein incorporated by reference in their entirety for all purposes), an amide bond will form between the two groups.

In certain instances, the polycationic moiety can have a ligand attached, such as a targeting ligand or a chelating moiety for complexing calcium. Preferably, after the ligand is attached, the cationic moiety maintains a positive charge. In certain instances, the ligand that is attached has a positive charge. Suitable ligands include, but are not limited to, a compound or device with a reactive functional group and include lipids, amphipathic lipids, carrier compounds, bioaffinity compounds, biomaterials, biopolymers, biomedical devices, analytically detectable compounds, therapeutically active compounds, enzymes, peptides, proteins, antibodies, immune stimulators, radiolabels, fluorogens, biotin, drugs, haptens, DNA, RNA, polysaccharides, liposomes, virosomes, micelles, immunoglobulins, functional groups, other targeting moieties, or toxins.

In some embodiments, the lipid conjugate (e.g., PEG-lipid) comprises from about 0.1 mol % to about 3 mol %, from about 0.5 mol % to about 3 mol %, or about 0.6 mol %, 0.7 mol %, 0.8 mol %, 0.9 mol %, 1.0 mol %, 1.1 mol %, 1.2 mol %, 1.3 mol %, 1.4 mol %, 1.5 mol %, 1.6 mol %, 1.7 mol %, 1.8 mol %, 1.9 mol %, 2.0 mol %, 2.1 mol%, 2.2 mol%, 2.3 mol %, 2.4 mol %, 2.5 mol %, 2.6 mol %, 2.7 mol %, 2.8 mol %, 2.9 mol % or 3 mol % (or any fraction thereof or range therein) of the total lipid present in the particle.

In other embodiments, the lipid conjugate (e.g., PEG-lipid) comprises from about 0 mol % to about 20 mol %, from about 0.5 mol % to about 20 mol %, from about 2 mol % to about 20 mol %, from about 1.5 mol % to about 18 mol %, from about 2 mol % to about 15 mol %, from about 4 mol % to about 15 mol %, from about 2 mol % to about 12 mol %, from about 5 mol % to about 12 mol %, or about 2 mol % (or any fraction thereof or range therein) of the total lipid present in the particle.

In further embodiments, the lipid conjugate (e.g., PEG-lipid) comprises from about 4 mol % to about 10 mol %, from about 5 mol % to about 10 mol %, from about 5 mol % to about 9 mol %, from about 5 mol % to about 8 mol %, from about 6 mol % to about 9 mol %, from about 6 mol % to about 8 mol %, or about 5 mol %, 6 mol %, 7 mol %, 8 mol %, 9 mol %, or 10 mol % (or any fraction thereof or range therein) of the total lipid present in the particle.

It should be understood that the percentage of lipid conjugate present in the lipid particles of the invention is a target amount, and that the actual amount of lipid conjugate present in the formulation may vary, for example, by ± 5 mol %, ± 4 mol %, ± 3 mol %, ± 2 mol %, ± 1 mol %, ± 0.75 mol %, ± 0.5 mol %, ± 0.25 mol %, or ± 0.1 mol %.

Additional percentages and ranges of lipid conjugates suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335, the disclosures of which are herein incorporated by reference in their entirety for all purposes.

One of ordinary skill in the art will appreciate that the concentration of the lipid conjugate can be varied depending on the lipid conjugate employed and the rate at which the lipid particle is to become fusogenic.

By controlling the composition and concentration of the lipid conjugate, one can control the rate at which the lipid conjugate exchanges out of the lipid particle and, in turn, the rate at which the lipid particle becomes fusogenic. For instance, when a PEG-DAA conjugate is used as the lipid conjugate, the rate at which the lipid particle becomes fusogenic can be varied, for example, by varying the concentration of the lipid conjugate, by varying the molecular weight of the PEG, or by varying the chain length and degree of saturation of the alkyl groups on the PEG-DAA conjugate. In addition, other variables including, for example, pH, temperature, ionic strength, *etc.* can be used to vary and/or control the rate at which the lipid particle becomes fusogenic. Other methods which can be used to control the rate at which the lipid particle becomes fusogenic will become apparent to those of skill in the art upon reading this disclosure. Also, by controlling the composition and concentration of the lipid conjugate, one can control the lipid particle size.

### Additional Carrier Systems

Non-limiting examples of additional lipid-based carrier systems suitable for use in the present invention include lipoplexes (*see, e.g.,* U.S. Patent Publication No. 20030203865; and Zhang et al., J. Control Release, 100:165-180 (2004)), pH-sensitive lipoplexes (*see, e.g.,* U.S. Patent Publication No. 20020192275), reversibly masked lipoplexes (*see, e.g.,* U.S. Patent Publication Nos. 20030180950), cationic lipid-based compositions (*see, e.g.,* U.S. Patent No. 6,756,054; and U.S. Patent Publication No. 20050234232), cationic liposomes (*see, e.g.,* U.S. Patent Publication Nos. 20030229040, 20020160038, and 20020012998; U.S. Patent No. 5,908,635; and PCT Publication No. WO 01/72283), anionic liposomes (*see, e.g.,* U.S. Patent Publication No. 20030026831), pH-sensitive liposomes (*see, e.g.,* U.S. Patent Publication No. 20020192274; and AU 2003210303), antibody-coated liposomes (*see, e.g.,* U.S. Patent Publication No. 20030108597; and PCT Publication No. WO 00/50008), cell-type specific liposomes (*see, e.g.,* U.S. Patent Publication No. 20030198664), liposomes containing nucleic acid and peptides (*see, e.g.,* U.S. Patent No. 6,207,456), liposomes containing lipids derivatized with releasable hydrophilic polymers (*see, e.g.,* U.S. Patent Publication No. 20030031704), lipid-entrapped nucleic acid (*see, e.g.,* PCT Publication Nos. WO 03/057190 and WO 03/059322), lipid-encapsulated nucleic acid (*see, e.g.,* U.S. Patent Publication No. 20030129221; and U.S. Patent No. 5,756,122), other liposomal compositions (*see, e.g.,* U.S. Patent Publication Nos. 20030035829 and 20030072794; and U.S. Patent No. 6,200,599), stabilized mixtures of liposomes and emulsions (*see, e.g.,* EP1304160), emulsion compositions (*see, e.g.,* U.S. Patent No. 6,747,014), and nucleic acid micro-emulsions (*see, e.g.,* U.S. Patent Publication No. 20050037086).

Examples of polymer-based carrier systems suitable for use in the present invention include, but are not limited to, cationic polymer-nucleic acid complexes (*i*.*e*., polyplexes). To form a polyplex, a nucleic acid (*e.g.,* an oligomeric nucleotide, such as a siRNA molecule) is typically complexed with a cationic polymer having a linear, branched, star, or dendritic polymeric structure that condenses the nucleic acid into positively charged particles capable of interacting with anionic proteoglycans at the cell surface and entering cells by endocytosis. In some embodiments, the polyplex comprises nucleic acid complexed with a cationic polymer such as polyethylenimine (PEI) *(see, e.g.,* U.S. Patent No. 6,013,240; commercially available from Qbiogene, Inc. (Carlsbad, CA) as *In vivo* jetPEI^{™}, a linear form of PEI), polypropylenimine (PPI), polyvinylpyrrolidone (PVP), poly-L-lysine (PLL), diethylaminoethyl (DEAE)-dextran, poly(β-amino ester) (PAE) polymers (*see*, *e.g.,* Lynn et al., J. Am. Chem. Soc., 123:8155-8156 (2001)), chitosan, polyamidoamine (PAMAM) dendrimers (*see, e.g.,* Kukowska-Latallo et al., Proc. Natl. Acad. Sci. USA, 93:4897-4902 (1996)), porphyrin (*see, e.g.,* U.S. Patent No. 6,620,805), polyvinylether *(see, e.g.,* U.S. Patent Publication No. 20040156909), polycyclic amidinium (*see, e.g.,* U.S. Patent Publication No. 20030220289), other polymers comprising primary amine, imine, guanidine, and/or imidazole groups (*see, e.g.,* U.S. Patent No. 6,013,240; PCT Publication No. WO/9602655; PCT Publication No. WO95/21931; Zhang et al., J. Control Release, 100:165-180 (2004); and Tiera et al., Curr. Gene Ther., 6:59-71 (2006)), and a mixture thereof. In other embodiments, the polyplex comprises cationic polymer-nucleic acid complexes as described in U.S. Patent Publication Nos. 20060211643, 20050222064, 20030125281, and 20030185890, and PCT Publication No. WO 03/066069; biodegradable poly(β-amino ester) polymer-nucleic acid complexes as described in U.S. Patent Publication No. 20040071654; microparticles containing polymeric matrices as described in U.S. Patent Publication No. 20040142475; other microparticle compositions as described in U.S. Patent Publication No. 20030157030; condensed nucleic acid complexes as described in U.S. Patent Publication No. 20050123600; and nanocapsule and microcapsule compositions as described in AU 2002358514 and PCT Publication No. WO 02/096551.

In certain instances, the oligomeric nucleotide may be complexed with cyclodextrin or a polymer thereof. Non-limiting examples of cyclodextrin-based carrier systems include the cyclodextrin-modified polymer-nucleic acid complexes described in U.S. Patent Publication No. 20040087024; the linear cyclodextrin copolymer-nucleic acid complexes described in U.S. Patent Nos. 6,509,323, 6,884,789, and 7,091,192; and the cyclodextrin polymer-complexing agent-nucleic acid complexes described in U.S. Patent No. 7,018,609. In certain other instances, the oligomeric nucleotide may be complexed with a peptide or polypeptide. An example of a protein-based carrier system includes, but is not limited to, the cationic oligopeptide-nucleic acid complex described in PCT Publication No. WO95/21931.

### Preparation of Lipid Particles

The nucleic acid-lipid particles of the present invention, in which an oligomeric nucleotide (i.e., nucleic acid molecule) is entrapped within the lipid portion of the particle and is protected from degradation, can be formed by any method known in the art including, but not limited to, a continuous mixing method, a direct dilution process, and an in-line dilution process.

In particular embodiments, the cationic lipids may comprise lipids of Formula I-III or salts thereof, alone or in combination with other cationic lipids. In other embodiments, the non-cationic lipids are egg sphingomyelin (ESM), distearoylphosphatidylcholine (DSPC), dioleoylphosphatidylcholine (DOPC), 1-palmitoyl-2-oleoyl-phosphatidylcholine (POPC), dipalmitoyl-phosphatidylcholine (DPPC), monomethyl-phosphatidylethanolamine, dimethyl-phosphatidylethanolamine, 14:0 PE (1,2-dimyristoyl-phosphatidylethanolamine (DMPE)), 16:0 PE (1,2-dipalmitoyl-phosphatidylethanolamine (DPPE)), 18:0 PE (1,2-distearoyl-phosphatidylethanolamine (DSPE)), 18:1 PE (1,2-dioleoyl-phosphatidylethanolamine (DOPE)), 18:1 trans PE (1,2-dielaidoyl-phosphatidylethanolamine (DEPE)), 18:0-18:1 PE (1-stearoyl-2-oleoyl-phosphatidylethanolamine (SOPE)), 16:0-18:1 PE (1-palmitoyl-2-oleoyl-phosphatidylethanolamine (POPE)), polyethylene glycol-based polymers (e.g., PEG 2000, PEG 5000, PEG-modified diacylglycerols, or PEG-modified dialkyloxypropyls), cholesterol, derivatives thereof, or combinations thereof.

In certain embodiments, the present invention provides nucleic acid-lipid particles produced via a continuous mixing method, *e.g.,* a process that includes providing an aqueous solution comprising an oligomeric nucleotide in a first reservoir, providing an organic lipid solution in a second reservoir (wherein the lipids present in the organic lipid solution are solubilized in an organic solvent, *e.g.,* a lower alkanol such as ethanol), and mixing the aqueous solution with the organic lipid solution such that the organic lipid solution mixes with the aqueous solution so as to substantially instantaneously produce a lipid vesicle (*e*.*g*., liposome) encapsulating the oligomeric nucleotide within the lipid vesicle. This process and the apparatus for carrying out this process are described in detail in U.S. Patent Publication No. 20040142025, the disclosure of which is herein incorporated by reference in its entirety for all purposes.

The action of continuously introducing lipid and buffer solutions into a mixing environment, such as in a mixing chamber, causes a continuous dilution of the lipid solution with the buffer solution, thereby producing a lipid vesicle substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (*i.e*., aqueous solution) to produce a nucleic acid-lipid particle.

The nucleic acid-lipid particles formed using the continuous mixing method typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

In another embodiment, the present invention provides nucleic acid-lipid particles produced via a direct dilution process that includes forming a lipid vesicle (e.g., liposome) solution and immediately and directly introducing the lipid vesicle solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of lipid vesicle solution introduced thereto. As a non-limiting example, a lipid vesicle solution in 45% ethanol when introduced into the collection vessel containing an equal volume of dilution buffer will advantageously yield smaller particles.

In yet another embodiment, the present invention provides nucleic acid-lipid particles produced via an in-line dilution process in which a third reservoir containing dilution buffer is fluidly coupled to a second mixing region. In this embodiment, the lipid vesicle (*e*.*g*., liposome) solution formed in a first mixing region is immediately and directly mixed with dilution buffer in the second mixing region. In preferred aspects, the second mixing region includes a T-connector arranged so that the lipid vesicle solution and the dilution buffer flows meet as opposing 180° flows; however, connectors providing shallower angles can be used, *e.g.,* from about 27° to about 180° (*e.g.,* about 90°). A pump mechanism delivers a controllable flow of buffer to the second mixing region. In one aspect, the flow rate of dilution buffer provided to the second mixing region is controlled to be substantially equal to the flow rate of lipid vesicle solution introduced thereto from the first mixing region. This embodiment advantageously allows for more control of the flow of dilution buffer mixing with the lipid vesicle solution in the second mixing region, and therefore also the concentration of lipid vesicle solution in buffer throughout the second mixing process. Such control of the dilution buffer flow rate advantageously allows for small particle size formation at reduced concentrations.

These processes and the apparatuses for carrying out these direct dilution and in-line dilution processes are described in detail in U.S. Patent Publication No. 20070042031, the disclosure of which is herein incorporated by reference in its entirety for all purposes.

The nucleic acid-lipid particles formed using the direct dilution and in-line dilution processes typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

If needed, the lipid particles of the invention can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Patent No. 4,737,323, the disclosure of which is herein incorporated by reference in its entirety for all purposes. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In some embodiments, the oligomeric nucleotides present in the particles (*e*.*g*., the siRNA molecules) are precondensed as described in, *e.g.,* U.S. Patent Application No. 09/744,103, the disclosure of which is herein incorporated by reference in its entirety for all purposes.

In other embodiments, the methods may further comprise adding non-lipid polycations which are useful to effect the lipofection of cells using the present compositions. Examples of suitable non-lipid polycations include, hexadimethrine bromide (sold under the brand name POLYBRENE^{®}, from Aldrich Chemical Co., Milwaukee, Wisconsin, USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

In some embodiments, the oligomeric nucleotide (*i.e.,* nucleic acid molecule, such as a siRNA molecule) to lipid ratios (mass/mass ratios) in a formed nucleic acid-lipid particle will range from about 0.01 to about 0.2, from about 0.05 to about 0.2, from about 0.02 to about 0.1, from about 0.03 to about 0.1, or from about 0.01 to about 0.08. The ratio of the starting materials (input) also falls within this range. In other embodiments, the particle preparation uses about 400 µg nucleic acid per 10 mg total lipid or a nucleic acid to lipid mass ratio of about 0.01 to about 0.08 and, more preferably, about 0.04, which corresponds to 1.25 mg of total lipid per 50 µg of nucleic acid. In other preferred embodiments, the particle has a nucleic acid:lipid mass ratio of about 0.08.

In other embodiments, the lipid to nucleic acid (e.g., siRNA) ratios (mass/mass ratios) in a formed nucleic acid-lipid particle will range from about 1 (1:1) to about 100 (100:1), from about 5 (5:1) to about 100 (100:1), from about 1 (1:1) to about 50 (50:1), from about 2 (2:1) to about 50 (50:1), from about 3 (3:1) to about 50 (50:1), from about 4 (4:1) to about 50 (50:1), from about 5 (5:1) to about 50 (50:1), from about 1 (1:1) to about 25 (25:1), from about 2 (2:1) to about 25 (25:1), from about 3 (3:1) to about 25 (25:1), from about 4 (4:1) to about 25 (25:1), from about 5 (5:1) to about 25 (25:1), from about 5 (5:1) to about 20 (20:1), from about 5 (5:1) to about 15 (15:1), from about 5 (5:1) to about 10 (10:1), or about 5 (5:1), 6 (6:1), 7 (7:1), 8 (8:1), 9 (9:1), 10 (10:1), 11 (11:1), 12 (12:1), 13 (13:1), 14 (14:1), 15 (15:1), 16 (16:1), 17 (17:1), 18 (18:1), 19 (19:1), 20 (20:1), 21 (21:1), 22 (22:1), 23 (23:1), 24 (24:1), or 25 (25:1), or any fraction thereof or range therein. The ratio of the starting materials (input) also falls within this range.

As previously discussed, the conjugated lipid may further include a CPL. A variety of general methods for making lipid particle-CPLs (CPL-containing lipid particles) are discussed herein. Two general techniques include the "post-insertion" technique, that is, insertion of a CPL into, for example, a pre-formed lipid particle, and the "standard" technique, wherein the CPL is included in the lipid mixture during, for example, the lipid particle formation steps. The post-insertion technique results in lipid particles having CPLs mainly in the external face of the lipid particle bilayer membrane, whereas standard techniques provide lipid particles having CPLs on both internal and external faces. The method is especially useful for vesicles made from phospholipids (which can contain cholesterol) and also for vesicles containing PEG-lipids (such as PEG-DAAs and PEG-DAGs). Methods of making lipid particle-CPLs are taught, for example, in U.S. Patent Nos. 5,705,385; 6,586,410; 5,981,501; 6,534,484; and 6,852,334; U.S. Patent Publication No. 20020072121; and PCT Publication No. WO 00/62813, the disclosures of which are herein incorporated by reference in their entirety for all purposes.

### Administration of Lipid Particles

Once formed, the lipid particles may be used for the introduction of an oligomeric nucleotide, such as a siRNA molecule, into cells. In particular embodiments, the oligomeric nucleotide (e.g., siRNA molecule) is introduced into an infected cell. The methods may be carried out *in vitro* or *in vivo* by first forming the particles as described above and then contacting the particles with the cells for a period of time sufficient for delivery of the oligomeric nucleotide to the cells to occur.

The lipid particles can be adsorbed to almost any cell type with which they are mixed or contacted. Once adsorbed, the particles can either be endocytosed by a portion of the cells, exchange lipids with cell membranes, or fuse with the cells. Transfer or incorporation of the oligomeric nucleotide portion of the particle can take place via any one of these pathways. In particular, when fusion takes place, the particle membrane is integrated into the cell membrane and the contents of the particle combine with the intracellular fluid.

The lipid particles can be administered either alone or in a mixture with a pharmaceutically acceptable carrier (e.g., physiological saline or phosphate buffer) selected in accordance with the route of administration and standard pharmaceutical practice. Generally, normal buffered saline (*e.g.,* 135-150 mM NaCl) will be employed as the pharmaceutically acceptable carrier. Other suitable carriers include, *e*.*g*., water, buffered water, 0.4% saline, 0.3% glycine, and the like, including glycoproteins for enhanced stability, such as albumin, lipoprotein, globulin, *etc.* Additional suitable carriers are described in, *e.g.,* REMINGTON'S PHARMACEUTICAL SCIENCES, Mack Publishing Company, Philadelphia, PA, 17th ed. (1985). As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The phrase "pharmaceutically acceptable" refers to molecular entities and compositions that do not produce an allergic or similar untoward reaction when administered to a human.

The pharmaceutically acceptable carrier is generally added following lipid particle formation. Thus, after the lipid particle is formed, the particle can be diluted into pharmaceutically acceptable carriers such as normal buffered saline.

The concentration of particles in the pharmaceutical formulations can vary widely, *i.e.,* from less than about 0.05%, usually at or at least about 2 to 5%, to as much as about 10 to 90% by weight, and will be selected primarily by fluid volumes, viscosities, *etc.,* in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well-known sterilization techniques. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol, and water-soluble iron-specific chelators, such as ferrioxamine, are suitable.

In some embodiments, the lipid particles may be useful in methods for the therapeutic delivery of one or more oligomeric nucleotides described herein.

### In vivo Administration of Lipid Particles

Systemic delivery for *in vivo* therapy, *e.g.,* delivery of an oligomeric nucleotide described herein, such as an siRNA, to a distal target cell via body systems such as the circulation, has been achieved using nucleic acid-lipid particles such as those described in PCT Publication Nos. WO 05/007196, WO 05/121348, WO 05/120152, and WO 04/002453, the disclosures of which are herein incorporated by reference in their entirety for all purposes. Described herein are fully encapsulated lipid particles that protect the oligomeric nucleotide from nuclease degradation in serum, are non-immunogenic, are small in size, and are suitable for repeat dosing. Additionally, the at least one oligomeric nucleotide may be administered alone in the lipid particles of the invention, or in combination (*e*.*g*., co-administered) with lipid particles comprising peptides, polypeptides, or small molecules such as conventional drugs.

For *in vivo* administration, administration can be in any manner known in the art, *e.g.,* by injection, oral administration, inhalation (*e*.*g*., intransal or intratracheal), transdermal application, or rectal administration. Administration can be accomplished via single or divided doses. The pharmaceutical compositions can be administered parenterally, *i.e.,* intraarticularly, intravenously, intraperitoneally, subcutaneously, or intramuscularly. In some embodiments, the pharmaceutical compositions are administered intravenously or intraperitoneally by a bolus injection (*see, e.g.,* U.S. Patent No. 5,286,634). Intracellular nucleic acid delivery has also been discussed in Straubringer et al., Methods Enzymol., 101:512 (1983); Mannino et al., Biotechniques, 6:682 (1988); Nicolau et al., Crit. Rev. Ther. Drug Carrier Syst., 6:239 (1989); and Behr, Acc. Chem. Res., 26:274 (1993). Still other methods of administering lipid-based therapeutics are described in, for example, U.S. Patent Nos. 3,993,754; 4,145,410; 4,235,871; 4,224,179; 4,522,803; and 4,588,578. The lipid particles can be administered by direct injection at the site of disease or by injection at a site distal from the site of disease (*see, e.g.,* Culver, HUMAN GENE THERAPY, MaryAnn Liebert, Inc., Publishers, New York. pp.70-71(1994)). The disclosures of the above-described references are herein incorporated by reference in their entirety for all purposes.

In embodiments where the lipid particles of the present invention are administered intravenously, at least about 5%, 10%, 15%, 20%, or 25% of the total injected dose of the particles is present in plasma about 8, 12, 24, 36, or 48 hours after injection. In other embodiments, more than about 20%, 30%, 40% and as much as about 60%, 70% or 80% of the total injected dose of the lipid particles is present in plasma about 8, 12, 24, 36, or 48 hours after injection. In certain instances, more than about 10% of a plurality of the particles is present in the plasma of a mammal about 1 hour after administration. In certain other instances, the presence of the lipid particles is detectable at least about 1 hour after administration of the particle. In some embodiments, the presence of a oligomeric nucleotide (e.g., an siRNA molecule) is detectable in cells at about 8, 12, 24, 36, 48, 60, 72 or 96 hours after administration. In other embodiments, downregulation of expression of a target sequence, such as a viral or host sequence, by a siRNA molecule is detectable at about 8, 12, 24, 36, 48, 60, 72 or 96 hours after administration. In yet other embodiments, downregulation of expression of a target sequence, such as a viral or host sequence, by a siRNA molecule occurs preferentially in infected cells and/or cells capable of being infected. In further embodiments, the presence or effect of a siRNA molecule in cells at a site proximal or distal to the site of administration is detectable at about 12, 24, 48, 72, or 96 hours, or at about 6, 8, 10, 12, 14, 16, 18, 19, 20, 22, 24, 26, or 28 days after administration. In additional embodiments, the lipid particles of the invention are administered parenterally or intraperitoneally.

The compositions described herien, either alone or in combination with other suitable components, can be made into aerosol formulations (*i.e.,* they can be "nebulized") to be administered via inhalation (*e.g.,* intranasally or intratracheally) (*see,* Brigham et al., Am. J. Sci., 298:278 (1989)). Aerosol formulations can be placed into pressurized acceptable propellants, such as dichlorodifluoromethane, propane, nitrogen, and the like.

In certain embodiments, the pharmaceutical compositions may be delivered by intranasal sprays, inhalation, and/or other aerosol delivery vehicles. Methods for delivering nucleic acid compositions directly to the lungs via nasal aerosol sprays have been described, *e*.*g*., in U.S. Patent Nos. 5,756,353 and 5,804,212. Likewise, the delivery of drugs using intranasal microparticle resins and lysophosphatidyl-glycerol compounds (U.S. Patent 5,725,871) are also well-known in the pharmaceutical arts. Similarly, transmucosal drug delivery in the form of a polytetrafluoroetheylene support matrix is described in U.S. Patent No. 5,780,045. The disclosures of the above-described patents are herein incorporated by reference in their entirety for all purposes.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In certain embodiments, compositions may be administered, for example, by intravenous infusion, orally, topically, intraperitoneally, intravesically, or intrathecally.

Generally, when administered intravenously, the lipid particle formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, PA, 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, *etc.* Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, *etc.* These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

In certain applications, the lipid particles disclosed herein may be delivered via oral administration to the individual. The particles may be incorporated with excipients and used in the form of ingestible tablets, buccal tablets, troches, capsules, pills, lozenges, elixirs, mouthwash, suspensions, oral sprays, syrups, wafers, and the like *(see, e.g.,* U.S. Patent Nos. 5,641,515, 5,580,579, and 5,792,451, the disclosures of which are herein incorporated by reference in their entirety for all purposes). These oral dosage forms may also contain the following: binders, gelatin; excipients, lubricants, and/or flavoring agents. When the unit dosage form is a capsule, it may contain, in addition to the materials described above, a liquid carrier. Various other materials may be present as coatings or to otherwise modify the physical form of the dosage unit. Of course, any material used in preparing any unit dosage form should be pharmaceutically pure and substantially non-toxic in the amounts employed.

Typically, these oral formulations may contain at least about 0.1% of the lipid particles or more, although the percentage of the particles may, of course, be varied and may conveniently be between about 1% or 2% and about 60% or 70% or more of the weight or volume of the total formulation. Naturally, the amount of particles in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Formulations suitable for oral administration can consist of: (a) liquid solutions, such as an effective amount of a packaged oligomeric nucleotide (*e*.*g*., a siRNA molecule) suspended in diluents such as water, saline, or PEG 400; (b) capsules, sachets, or tablets, each containing a predetermined amount of an oligomeric nucleotide, as liquids, solids, granules, or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise an oligomeric nucleotide in a flavor, *e.g.,* sucrose, as well as pastilles comprising the therapeutic nucleic acid in an inert base, such as gelatin and glycerin or sucrose and acacia emulsions, gels, and the like containing, in addition to the oligomeric nucleotide, carriers known in the art.

In another example of their use, lipid particles can be incorporated into a broad range of topical dosage forms. For instance, a suspension containing nucleic acid-lipid particles can be formulated and administered as gels, oils, emulsions, topical creams, pastes, ointments, lotions, foams, mousses, and the like.

When preparing pharmaceutical preparations of the lipid particles of the invention, it is preferable to use quantities of the particles which have been purified to reduce or eliminate empty particles or particles with therapeutic agents such as an oligomeric nucleotide associated with the external surface.

The methods described herein may be practiced in a variety of hosts. Preferred hosts include mammalian species, such as primates (*e*.*g*., humans and chimpanzees as well as other nonhuman primates), canines, felines, equines, bovines, ovines, caprines, rodents (e.g., rats and mice), lagomorphs, and swine.

The amount of particles administered will depend upon the ratio of the oligomeric nucleotide (e.g., siRNA molecule) to lipid, the particular oligomeric nucleotide used, the strain of HBV being treated, the age, weight, and condition of the patient, and the judgment of the clinician, but will generally be between about 0.01 and about 50 mg per kilogram of body weight, preferably between about 0.1 and about 5 mg/kg of body weight, or about 10⁸-10¹⁰ particles per administration (e.g., injection).

### In vitro Administration

For *in vitro* applications, the delivery of oligomeric nucleotides can be to any cell grown in culture. In preferred embodiments, the cells are animal cells, more preferably mammalian cells, and most preferably human cells.

Contact between the cells and the lipid particles, when carried out *in vitro,* takes place in a biologically compatible medium. The concentration of particles varies widely depending on the particular application, but is generally between about 1 µmol and about 10 mmol. Treatment of the cells with the lipid particles is generally carried out at physiological temperatures (about 37°C) for periods of time of from about 1 to 48 hours, preferably of from about 2 to 4 hours.

In one group of preferred embodiments, a lipid particle suspension is added to 60-80% confluent plated cells having a cell density of from about 10³ to about 10⁵ cells/ml, more preferably about 2 x 10⁴ cells/ml. The concentration of the suspension added to the cells is preferably of from about 0.01 to 0.2 µg/ml, more preferably about 0.1 µg/ml.

To the extent that tissue culture of cells may be required, it is well-known in the art. For example, Freshney, Culture of Animal Cells, a Manual of Basic Technique, 3rd Ed., Wiley-Liss, New York (1994), Kuchler et al., Biochemical Methods in Cell Culture and Virology, Dowden, Hutchinson and Ross, Inc. (1977), and the references cited therein provide a general guide to the culture of cells. Cultured cell systems often will be in the form of monolayers of cells, although cell suspensions are also used.

Using an Endosomal Release Parameter (ERP) assay, the delivery efficiency of a nucleic acid-lipid particle described herein can be optimized. An ERP assay is described in detail in U.S. Patent Publication No. 20030077829, the disclosure of which is herein incorporated by reference in its entirety for all purposes. More particularly, the purpose of an ERP assay is to distinguish the effect of various cationic lipids and helper lipid components of the lipid particle based on their relative effect on binding/uptake or fusion with/destabilization of the endosomal membrane. This assay allows one to determine quantitatively how each component of the lipid particle affects delivery efficiency, thereby optimizing the lipid particle. Usually, an ERP assay measures expression of a reporter protein (*e*.*g*., luciferase, β-galactosidase, green fluorescent protein (GFP), *etc.),* and in some instances, a lipid particle formulation optimized for an expression plasmid will also be appropriate for encapsulating an oligomeric nucleotide. In other instances, an ERP assay can be adapted to measure downregulation of transcription or translation of a target sequence in the presence or absence of an oligomeric nucleotide. By comparing the ERPs for each of the various lipid particles, one can readily determine the optimized system, *e*.*g*., the lipid particle that has the greatest uptake in the cell.

### Detection of Lipid Particles

In some embodiments, the lipid particles are detectable in the subject at about 1, 2, 3, 4, 5, 6, 7, 8 or more hours. In other embodiments, the lipid particles are detectable in the subject at about 8, 12, 24, 48, 60, 72, or 96 hours, or about 6, 8, 10, 12, 14, 16, 18, 19, 22, 24, 25, or 28 days after administration of the particles. The presence of the particles can be detected in the cells, tissues, or other biological samples from the subject. The particles may be detected, *e*.*g*., by direct detection of the particles, detection of an oligomeric nucleotide sequence, detection of the target sequence of interest (*i*.*e*., by detecting expression or reduced expression of the sequence of interest), detection of a compound modulated by an HBV protein, detection of viral load in the subject, or a combination thereof.

Lipid particles can be detected using any method known in the art. For example, a label can be coupled directly or indirectly to a component of the lipid particle using methods well-known in the art. A wide variety of labels can be used, with the choice of label depending on sensitivity required, ease of conjugation with the lipid particle component, stability requirements, and available instrumentation and disposal provisions. Suitable labels include, but are not limited to, spectral labels such as fluorescent dyes (*e*.*g*., fluorescein and derivatives, such as fluorescein isothiocyanate (FITC) and Oregon Green^{™}; rhodamine and derivatives such Texas red, tetrarhodimine isothiocynate (TRITC), *etc.,* digoxigenin, biotin, phycoerythrin, AMCA, CyDyes^{™}, and the like; radiolabels such as ³H, ¹²⁵I, ³⁵S, ¹⁴C, ³²P, ³³P, *etc*.; enzymes such as horse radish peroxidase, alkaline phosphatase, *etc*.; spectral colorimetric labels such as colloidal gold or colored glass or plastic beads such as polystyrene, polypropylene, latex, *etc.* The label can be detected using any means known in the art.

### Detection of Nucleic Acids

Oligomeric nucleotides (i.e., nucleic acids, such as siRNA molecules) are detected and quantified herein by any of a number of means well-known to those of skill in the art. The detection of nucleic acids may proceed by well-known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), and hyperdiffusion chromatography may also be employed.

The selection of a nucleic acid hybridization format is not critical. A variety of nucleic acid hybridization formats are known to those skilled in the art. For example, common formats include sandwich assays and competition or displacement assays. Hybridization techniques are generally described in, *e.g.,* "Nucleic Acid Hybridization, A Practical Approach," Eds. Hames and Higgins, IRL Press (1985).

The sensitivity of the hybridization assays may be enhanced through the use of a nucleic acid amplification system which multiplies the target nucleic acid being detected. *In vitro* amplification techniques suitable for amplifying sequences for use as molecular probes or for generating nucleic acid fragments for subsequent subcloning are known. Examples of techniques sufficient to direct persons of skill through such *in vitro* amplification methods, including the polymerase chain reaction (PCR), the ligase chain reaction (LCR), Qβ-replicase amplification, and other RNA polymerase mediated techniques (*e.g*., NASBA^{™}) are found in Sambrook et al., In Molecular Cloning: A Laboratory Manual, Cold Spring Harbor Laboratory Press (2000); and Ausubel et al., SHORT PROTOCOLS IN MOLECULAR BIOLOGY, eds., Current Protocols, Greene Publishing Associates, Inc. and John Wiley & Sons, Inc. (2002); as well as U.S. Patent No. 4,683,202; PCR Protocols, A Guide to Methods and Applications (Innis et al. eds.) Academic Press Inc. San Diego, CA (1990); Arnheim & Levinson (October 1, 1990), C&EN 36; The Journal Of NIH Research, 3:81 (1991); Kwoh et al., Proc. Natl. Acad. Sci. USA, 86:1173 (1989); Guatelli et al., Proc. Natl. Acad. Sci. USA, 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990); and Sooknanan and Malek, Biotechnology, 13:563 (1995). Improved methods of cloning *in vitro* amplified nucleic acids are described in U.S. Pat. No. 5,426,039. Other methods described in the art are the nucleic acid sequence based amplification (NASBA^{™}, Cangene, Mississauga, Ontario) and Qβ-replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation. The disclosures of the above-described references are herein incorporated by reference in their entirety for all purposes.

Nucleic acids for use as probes, *e.g.,* in *in vitro* amplification methods, for use as gene probes, or as inhibitor components are typically synthesized chemically according to the solid phase phosphoramidite triester method described by Beaucage et al., Tetrahedron Letts., 22:1859 1862 (1981), *e.g.,* using an automated synthesizer, as described in Needham VanDevanter et al., Nucleic Acids Res., 12:6159 (1984). Purification of polynucleotides, where necessary, is typically performed by either native acrylamide gel electrophoresis or by anion exchange HPLC as described in Pearson et al., J. Chrom., 255:137 149 (1983). The sequence of the synthetic polynucleotides can be verified using the chemical degradation method of Maxam and Gilbert (1980) in Grossman and Moldave (eds.) Academic Press, New York, Methods in Enzymology, 65:499.

An alternative means for determining the level of transcription is *in situ* hybridization. *In situ* hybridization assays are well-known and are generally described in Angerer et al., Methods Enzymol., 152:649 (1987). In an *in situ* hybridization assay, cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### Description of Certain Nucleic Acid-Lipid Particle Embodiments

A nucleic acid-lipid particle typically may comprise one or more oligomeric nucleotides (*e.g.,* a cocktail), a cationic lipid, and a non-cationic lipid. In certain instances, the nucleic acid-lipid particles further comprise a conjugated lipid that inhibits aggregation of particles.

In some embodiments, the at least one oligomeric nucleotide (e.g., an siRNA) is fully encapsulated in the nucleic acid-lipid particle. With respect to formulations comprising a cocktail of oligomeric nucleotides, the different types of species present in the cocktail (*e*.*g*., siRNA compounds with different sequences) may be co-encapsulated in the same particle, or each type of species present in the cocktail may be encapsulated in a separate particle. The cocktail may be formulated in the particles described herein using a mixture of two, three or more individual nucleotides (each having a unique sequence) at identical, similar, or different concentrations or molar ratios. In one embodiment, a cocktail of siRNAs (corresponding to a plurality of siRNAs with different sequences) is formulated using identical, similar, or different concentrations or molar ratios of each siRNA species, and the different types of siRNAs are co-encapsulated in the same particle. In another embodiment, each type of siRNA species present in the cocktail is encapsulated in different particles at identical, similar, or different siRNA concentrations or molar ratios, and the particles thus formed (each containing a different siRNA payload) are administered separately (*e*.*g*., at different times in accordance with a therapeutic regimen), or are combined and administered together as a single unit dose *(e.g.,* with a pharmaceutically acceptable carrier). The particles described herein are serum-stable, are resistant to nuclease degradation, and are substantially non-toxic to mammals such as humans.

The cationic lipid in the nucleic acid-lipid particles of the invention may comprise, e.g., one or more cationic lipids of Formula I-III described herein or any other cationic lipid species. In one embodiment, cationic lipid is a dialkyl lipid. In another embodiment, the cationic lipid is a trialkyl lipid. In one particular embodiment, the cationic lipid is selected from the group consisting of 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (γ-DLenDMA; Compound **(15)),** 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), dilinoleylmethyl-3-dimethylaminopropionate (DLin-M-C2-DMA), (6Z,9Z,28Z,31Z)-heptatriaconta-6,9,28,31-tetraen-19-yl 4-(dimethylamino)butanoate (DLin-M-C3-DMA; Compound **(7)),** salts thereof, and mixtures thereof.

In another particular embodiment, the cationic lipid is selected from the group consisting of 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane(γ-DLenDMA; Compound **(15)),** 3-((6Z,9Z,28Z,31Z)-heptatriaconta-6,9,28,31-tetraen-19-yloxy)-N,N-dimethylpropan-1-amine (DLin-MP-DMA; Compound **(8)),** (6Z,9Z,28Z,31Z)-heptatriaconta-6,9,28,31-tetraen-19-yl 4-(dimethylamino)butanoate) (Compound **(7)),** (6Z,16Z)-12-((Z)-dec-4-enyl)docosa-6,16-dien-11-yl 5-(dimethylamino)pentanoate (Compound **(13)),** a salt thereof, or a mixture thereof.

In certain embodiments, the cationic lipid comprises from about 48 mol % to about 62 mol % of the total lipid present in the particle.

The non-cationic lipid in the nucleic acid-lipid particles of the present invention may comprise, *e.g.,* one or more anionic lipids and/or neutral lipids. In some embodiments, the non-cationic lipid comprises one of the following neutral lipid components: (1) a mixture of a phospholipid and cholesterol or a derivative thereof; (2) cholesterol or a derivative thereof; or (3) a phospholipid. In certain preferred embodiments, the phospholipid comprises dipalmitoylphosphatidylcholine (DPPC), distearoylphosphatidylcholine (DSPC), or a mixture thereof. In a preferred embodiment, the non-cationic lipid is a mixture of DPPC and cholesterol. In a preferred embodiment, the non-cationic lipid is a mixture of DSPC and cholesterol.

In certain embodiments, the non-cationic lipid comprises a mixture of a phospholipid and cholesterol or a derivative thereof, wherein the phospholipid comprises from about 7 mol % to about 17 mol % of the total lipid present in the particle and the cholesterol or derivative thereof comprises from about 25 mol % to about 40 mol % of the total lipid present in the particle.

The lipid conjugate in the nucleic acid-lipid particles of the invention inhibits aggregation of particles and may comprise, *e*.*g*., one or more of the lipid conjugates described herein. In one particular embodiment, the lipid conjugate comprises a PEG-lipid conjugate. Examples of PEG-lipid conjugates include, but are not limited to, PEG-DAG conjugates, PEG-DAA conjugates, and mixtures thereof. In certain embodiments, the PEG-lipid conjugate is selected from the group consisting of a PEG-diacylglycerol (PEG-DAG) conjugate, a PEG-dialkyloxypropyl (PEG-DAA) conjugate, a PEG-phospholipid conjugate, a PEG-ceramide (PEG-Cer) conjugate, and a mixture thereof. In certain embodiments, the PEG-lipid conjugate is a PEG-DAA conjugate. In certain embodiments, the PEG-DAA conjugate in the lipid particle may comprise a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, a PEG-distearyloxypropyl (C₁₈) conjugate, or mixtures thereof. In certain embodiments, wherein the PEG-DAA conjugate is a PEG-dimyristyloxypropyl (C₁₄) conjugate. In another embodiment, the PEG-DAA conjugate is a compound **(66)** (PEG-C-DMA) conjugate. In another embodiment, the lipid conjugate comprises a POZ-lipid conjugate such as a POZ-DAA conjugate.

In certain embodiments, the conjugated lipid that inhibits aggregation of particles comprises from about 0.5 mol % to about 3 mol % of the total lipid present in the particle.

In certain embodiments, the nucleic acid-lipid particle has a total lipid:oligomeric nucleotide mass ratio of from about 5:1 to about 15:1.

In certain embodiments, the nucleic acid-lipid particle has a median diameter of from about 30 nm to about 150 nm.

In certain embodiments, the nucleic acid-lipid particle has an electron dense core.

In some embodiments, the present invention provides nucleic acid-lipid particles comprising: (a) at least one oligomeric nucleotide; (b) one or more cationic lipids or salts thereof comprising from about 50 mol % to about 85 mol % of the total lipid present in the particle; (c) one or more non-cationic lipids comprising from about 13 mol % to about 49.5 mol % of the total lipid present in the particle; and (d) one or more conjugated lipids that inhibit aggregation of particles comprising from about 0.5 mol % to about 2 mol % of the total lipid present in the particle.

In one aspect of this embodiment, the nucleic acid-lipid particle comprises: (a) at least one oligomeric nucleotide; (b) a cationic lipid or a salt thereof comprising from about 52 mol % to about 62 mol % of the total lipid present in the particle; (c) a mixture of a phospholipid and cholesterol or a derivative thereof comprising from about 36 mol % to about 47 mol % of the total lipid present in the particle; and (d) a PEG-lipid conjugate comprising from about 1 mol % to about 2 mol % of the total lipid present in the particle. In one particular embodiment, the formulation is a four-component system comprising about 1.4 mol % PEG-lipid conjugate (e.g., PEG2000-C-DMA), about 57.1 mol % cationic lipid (e.g., DLin-K-C2-DMA) or a salt thereof, about 7.1 mol % DPPC (or DSPC), and about 34.3 mol % cholesterol (or derivative thereof).

In another aspect of this embodiment, the nucleic acid-lipid particle comprises: (a) at least one oligomeric nucleotide; (b) a cationic lipid or a salt thereof comprising from about 56.5 mol % to about 66.5 mol % of the total lipid present in the particle; (c) cholesterol or a derivative thereof comprising from about 31.5 mol % to about 42.5 mol % of the total lipid present in the particle; and (d) a PEG-lipid conjugate comprising from about 1 mol % to about 2 mol % of the total lipid present in the particle. In one particular embodiment, the formulation is a three-component system which is phospholipid-free and comprises about 1.5 mol % PEG-lipid conjugate (*e.g.,* PEG2000-C-DMA), about 61.5 mol % cationic lipid (*e.g.,* DLin-K-C2-DMA) or a salt thereof, and about 36.9 mol % cholesterol (or derivative thereof).

Additional formulations are described in PCT Publication No. WO 09/127060 and published US patent application publication number US 2011/0071208 A1, the disclosures of which are herein incorporated by reference in their entirety for all purposes.

In other embodiments, the present invention provides nucleic acid-lipid particles comprising: (a) at least one oligomeric nucleotide; (b) one or more cationic lipids or salts thereof comprising from about 2 mol % to about 50 mol % of the total lipid present in the particle; (c) one or more non-cationic lipids comprising from about 5 mol % to about 90 mol % of the total lipid present in the particle; and (d) one or more conjugated lipids that inhibit aggregation of particles comprising from about 0.5 mol % to about 20 mol % of the total lipid present in the particle.

In one aspect of this embodiment, the nucleic acid-lipid particle comprises: (a) at least one oligomeric nucleotide; (b) a cationic lipid or a salt thereof comprising from about 30 mol % to about 50 mol % of the total lipid present in the particle; (c) a mixture of a phospholipid and cholesterol or a derivative thereof comprising from about 47 mol % to about 69 mol % of the total lipid present in the particle; and (d) a PEG-lipid conjugate comprising from about 1 mol % to about 3 mol % of the total lipid present in the particle. In one particular embodiment, the formulation is a four-component system which comprises about 2 mol % PEG-lipid conjugate (*e.g.,* PEG2000-C-DMA), about 40 mol % cationic lipid *(e.g.,* DLin-K-C2-DMA) or a salt thereof, about 10 mol % DPPC (or DSPC), and about 48 mol % cholesterol (or derivative thereof).

In further embodiments, the present invention provides nucleic acid-lipid particles comprising: (a) at least one oligomeric nucleotide; (b) one or more cationic lipids or salts thereof comprising from about 50 mol % to about 65 mol % of the total lipid present in the particle; (c) one or more non-cationic lipids comprising from about 25 mol % to about 45 mol % of the total lipid present in the particle; and (d) one or more conjugated lipids that inhibit aggregation of particles comprising from about 5 mol % to about 10 mol % of the total lipid present in the particle.

In one aspect of this embodiment, the nucleic acid-lipid particle comprises: (a) at least one oligomeric nucleotide; (b) a cationic lipid or a salt thereof comprising from about 50 mol % to about 60 mol % of the total lipid present in the particle; (c) a mixture of a phospholipid and cholesterol or a derivative thereof comprising from about 35 mol % to about 45 mol % of the total lipid present in the particle; and (d) a PEG-lipid conjugate comprising from about 5 mol % to about 10 mol % of the total lipid present in the particle. In certain instances, the non-cationic lipid mixture in the formulation comprises: (i) a phospholipid of from about 5 mol % to about 10 mol % of the total lipid present in the particle; and (ii) cholesterol or a derivative thereof of from about 25 mol % to about 35 mol % of the total lipid present in the particle. In one particular embodiment, the formulation is a four-component system which comprises about 7 mol % PEG-lipid conjugate (*e.g*., PEG750-C-DMA), about 54 mol % cationic lipid *(e.g.,* DLin-K-C2-DMA) or a salt thereof, about 7 mol % DPPC (or DSPC), and about 32 mol % cholesterol (or derivative thereof).

In another aspect of this embodiment, the nucleic acid-lipid particle comprises: (a) at least one oligomeric nucleotide; (b) a cationic lipid or a salt thereof comprising from about 55 mol % to about 65 mol % of the total lipid present in the particle; (c) cholesterol or a derivative thereof comprising from about 30 mol % to about 40 mol % of the total lipid present in the particle; and (d) a PEG-lipid conjugate comprising from about 5 mol % to about 10 mol % of the total lipid present in the particle. In one particular embodiment, the formulation is a three-component system which is phospholipid-free and comprises about 7 mol % PEG-lipid conjugate (*e.g.,* PEG750-C-DMA), about 58 mol % cationic lipid (*e.g.,* DLin-K-C2-DMA) or a salt thereof, and about 35 mol % cholesterol (or derivative thereof).

Additional embodiments of useful formulations are described in published US patent application publication number US 2011/0076335 A1, the disclosure of which is herein incorporated by reference in its entirety for all purposes.

In certain embodiments of the invention, the nucleic acid-lipid particle comprises: (a) at least one oligomeric nucleotide; (b) a cationic lipid or a salt thereof comprising from about 48 mol % to about 62 mol % of the total lipid present in the particle; (c) a mixture of a phospholipid and cholesterol or a derivative thereof, wherein the phospholipid comprises about 7 mol % to about 17 mol % of the total lipid present in the particle, and wherein the cholesterol or derivative thereof comprises about 25 mol % to about 40 mol % of the total lipid present in the particle; and (d) a PEG-lipid conjugate comprising from about 0.5 mol % to about 3.0 mol % of the total lipid present in the particle. Exemplary lipid formulations A-Z of this aspect of the invention are included below.

Exemplary lipid formulation A includes the following components (wherein the percentage values of the components are mole percent): PEG-lipid (1.2%), cationic lipid (53.2%), phospholipid (9.3%), cholesterol (36.4%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.2%), the cationic lipid is 1,2-dilinoleyloxy-N,N-dimethylaminopropane (DLinDMA) (53.2%), the phospholipid is DPPC (9.3%), and cholesterol is present at 36.4%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation A, which comprises at least one oligomeric nucleotide described herein (e.g., siRNA).

Exemplary lipid formulation B which includes the following components (wherein the percentage values of the components are mole percent): PEG-lipid (0.8%), cationic lipid (59.7%), phospholipid (14.2%), cholesterol (25.3%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound (67)) (0.8%), the cationic lipid is 1,2-dilinoleyloxy-N,N-dimethylaminopropane (DLinDMA) (59.7%), the phospholipid is DSPC (14.2%), and cholesterol is present at 25.3%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation B, which comprises at least one oligomeric nucleotide described herein (e.g., siRNA).

Exemplary lipid formulation C includes the following components (wherein the percentage values of the components are mole percent): PEG-lipid (1.9%), cationic lipid (52.5%), phospholipid (14.8%), cholesterol (30.8%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (1.9%), the cationic lipid is 1,2-di-γ-linolenyloxy-N,N-dimethylaminopropane (γ-DLenDMA; Compound **(15))** (52.5%), the phospholipid is DSPC (14.8%), and cholesterol is present at 30.8%, wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation C, which comprises at least one oligomeric nucleotide described herein (e.g., siRNA).

Exemplary lipid formulation D includes the following components (wherein the percentage values of the components are mole percent): PEG-lipid (0.7%), cationic lipid (60.3%), phospholipid (8.4%), cholesterol (30.5%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (0.7%), the cationic lipid is 3-((6Z,9Z,28Z,31Z)-heptatriaconta-6,9,28,31-tetraen-19-yloxy)-N,N-dimethylpropan-1-amine (DLin-MP-DMA; Compound **(8)** (60.3%), the phospholipid is DSPC (8.4%), and cholesterol is present at 30.5%, wherein the actual amounts of the lipids present may vary by, *e.g.,* ± 5 % (or e.g., ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation D, which comprises at least one oligomeric nucleotide described herein (e.g., siRNA).

Exemplary lipid formulation E includes the following components (wherein the percentage values of the components are mole percent): PEG-lipid (1.8%), cationic lipid (52.1%), phospholipid (7.5%), cholesterol (38.5%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.8%), the cationic lipid is (6Z,9Z,28Z,31Z)-heptatriaconta-6,9,28,31-tetraen-19-yl 4-(dimethylamino)butanoate) (Compound (7)) (52.1%), the phospholipid is DPPC (7.5%), and cholesterol is present at 38.5%, wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation E, which comprises at least one oligomeric nucleotide described herein (e.g., siRNA).

Exemplary formulation F includes the following components (wherein the percentage values of the components are mole percent): PEG-lipid (0.9%), cationic lipid (57.1%), phospholipid (8.1%), cholesterol (33.8%), wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (0.9%), the cationic lipid is 1,2-dilinolenyloxy-N,N-dimethylaminopropane (DLenDMA), 1,2-di-γ-linolenyloxy-N,N-dimethylaminopropane (γ-DLenDMA; Compound **(15))** (57.1%), the phospholipid is DSPC (8.1%), and cholesterol is present at 33.8%, wherein the actual amounts of the lipids present may vary by, *e.g.,* ± 5 % (or *e*.*g*., ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation F, which comprises at least one oligomeric nucleotide described herein (e.g., siRNA).

Exemplary lipid formulation G includes the following components (wherein the percentage values of the components are mole percent): PEG-lipid (1.7%), cationic lipid (61.6%), phospholipid (11.2%), cholesterol (25.5%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (1.7%), the cationic lipid is 1,2-dilinolenyloxy-N,N-dimethylaminopropane (DLenDMA), 1,2-di-γ-linolenyloxy-N,N-dimethylaminopropane (γ-DLenDMA; Compound (15)) (61.6%), the phospholipid is DPPC (11.2%), and cholesterol is present at 25.5%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation G, which comprises at least one oligomeric nucleotide described herein (e.g., siRNA).

Exemplary lipid formulation H includes the following components (wherein the percentage values of the components are mole percent): PEG-lipid (1.1%), cationic lipid (55.0%), phospholipid (11.0%), cholesterol (33.0%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.1%), the cationic lipid is (6Z,16Z)-12-((Z)-dec-4-enyl)docosa-6,16-dien-11-yl 5-(dimethylamino)pentanoate (Compound (13)) (55.0%), the phospholipid is DSPC (11.0%), and cholesterol is present at 33.0%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation H, which comprises at least one oligomeric nucleotide described herein (e.g., siRNA).

Exemplary lipid formulation I includes the following components (wherein the percentage values of the components are mole percent): PEG-lipid (2.6%), cationic lipid (53.1%), phospholipid (9.4%), cholesterol (35.0%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.6%), the cationic lipid is (6Z,16Z)-12-((Z)-dec-4-enyl)docosa-6,16-dien-11-yl 5-(dimethylamino)pentanoate (Compound **(13))** (53.1%), the phospholipid is DSPC (9.4%), and cholesterol is present at 35.0%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation I, which comprises at least one oligomeric nucleotide described herein (e.g., siRNA).

Exemplary lipid formulation J includes the following components (wherein the percentage values of the components are mole percent): PEG-lipid (0.6%), cationic lipid (59.4%), phospholipid (10.2%), cholesterol (29.8%), wherein the actual amounts of the lipids present may vary by by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (0.6%), the cationic lipid is 1,2-dilinoleyloxy-N,N-dimethylaminopropane (DLinDMA) (59.4%), the phospholipid is DPPC (10.2%), and cholesterol is present at 29.8%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation J, which comprises at least one oligomeric nucleotide described herein (e.g., siRNA).

Exemplary lipid formulation K includes the following components (wherein the percentage values of the components are mole percent): PEG-lipid (0.5%), cationic lipid (56.7%), phospholipid (13.1%), cholesterol (29.7%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (0.5%), the cationic lipid is (6Z,9Z,28Z,31Z)-heptatriaconta-6,9,28,31-tetraen-19-yl 4-(dimethylamino)butanoate) (Compound **(7))** (56.7%), the phospholipid is DSPC (13.1%), and cholesterol is present at 29.7%, wherein the actual amounts of the lipids present may vary by, *e.g.,* ± 5 % (or *e.g.,* ± *4* mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation K, which comprises at least one oligomeric nucleotide described herein (e.g., siRNA).

Exemplary lipid formulation L includes the following components (wherein the percentage values of the components are mole percent): PEG-lipid (2.2%), cationic lipid (52.0%), phospholipid (9.7%), cholesterol (36.2%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (2.2%), the cationic lipid is 1,2-di-γ-linolenyloxy-N,N-dimethylaminopropane (γ-DLenDMA; Compound **(15))** (52.0%), the phospholipid is DSPC (9.7%), and cholesterol is present at 36.2%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation L, which comprises at least one oligomeric nucleotide described herein (e.g., siRNA).

Exemplary lipid formulation M includes the following components (wherein the percentage values of the components are mole percent): PEG-lipid (2.7%), cationic lipid (58.4%), phospholipid (13.1%), cholesterol (25.7%), wherein the actual amounts of the lipids present may vary by by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.7%), the cationic lipid is 1,2-dilinoleyloxy-N,N-dimethylaminopropane (DLinDMA) (58.4%), the phospholipid is DPPC (13.1%), and cholesterol is present at 25.7%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation M, which comprises at least one oligomeric nucleotide described herein (e.g., siRNA).

Exemplary lipid formulation N includes the following components (wherein the percentage values of the components are mole percent): PEG-lipid (3.0%), cationic lipid (53.3%), phospholipid (12.1%), cholesterol (31.5%), wherein the actual amounts of the lipids present may vary by by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (3.0%), the cationic lipid is 1,2-dilinoleyloxy-N,N-dimethylaminopropane (DLinDMA) (53.3%), the phospholipid is DPPC (12.1%), and cholesterol is present at 31.5%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation N, which comprises at least one oligomeric nucleotide described herein (e.g., siRNA).

Exemplary lipid formulation O includes the following components (wherein the percentage values of the components are mole percent): PEG-lipid (1.5%), cationic lipid (56.2%), phospholipid (7.8%), cholesterol (34.7%), wherein the actual amounts of the lipids present may vary by by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.5%), the cationic lipid is 1,2-dilinoleyloxy-N,N-dimethylaminopropane (DLinDMA) (56.2%), the phospholipid is DPPC (7.8%), and cholesterol is present at 34.7%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation O, which comprises at least one oligomeric nucleotide described herein (e.g., siRNA).

Exemplary lipid formulation P includes the following components (wherein the percentage values of the components are mole percent): PEG-lipid (2.1%), cationic lipid (48.6%), phospholipid (15.5%), cholesterol (33.8%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (2.1%), the cationic lipid is 3-((6Z,9Z,28Z,31Z)-heptatriaconta-6,9,28,31-tetraen-19-yloxy)-N,N-dimethylpropan-1-amine (DLin-MP-DMA; Compound **(8))** (48.6%), the phospholipid is DSPC (15.5%), and cholesterol is present at 33.8%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation P, which comprises at least one oligomeric nucleotide described herein (e.g., siRNA).

Exemplary lipid formulation Q includes the following components (wherein the percentage values of the components are mole percent): PEG-lipid (2.5%), cationic lipid (57.9%), phospholipid (9.2%), cholesterol (30.3%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.5%), the cationic lipid is (6Z,16Z)-12-((Z)-dec-4-enyl)docosa-6,16-dien-11-yl 5-(dimethylamino)pentanoate (Compound **(13))** (57.9%), the phospholipid is DSPC (9.2%), and cholesterol is present at 30.3%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation Q, which comprises at least one oligomeric nucleotide described herein (e.g., siRNA).

Exemplary lipid formulation R includes the following components (wherein the percentage values of the components are mole percent): PEG-lipid (1.6%), cationic lipid (54.6%), phospholipid (10.9%), cholesterol (32.8%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.6%), the cationic lipid is 3-((6Z,9Z,28Z,31Z)-heptatriaconta-6,9,28,31-tetraen-19-yloxy)-N,N-dimethylpropan-1-amine (Compound **(8))** (54.6%), the phospholipid is DSPC (10.9%), and cholesterol is present at 32.8%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation R, which comprises at least one oligomeric nucleotide described herein (e.g., siRNA).

Exemplary lipid formulation S includes the following components (wherein the percentage values of the components are mole percent): PEG-lipid (2.9%), cationic lipid (49.6%), phospholipid (16.3%), cholesterol (31.3%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.9%), the cationic lipid is (6Z,16Z)-12-((Z)-dec-4-enyl)docosa-6,16-dien-11-yl 5-(dimethylamino)pentanoate (Compound (13)) (49.6%), the phospholipid is DPPC (16.3%), and cholesterol is present at 31.3%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation S, which comprises at least one oligomeric nucleotide described herein (e.g., siRNA).

Exemplary lipid formulation T includes the following components (wherein the percentage values of the components are mole percent): PEG-lipid (0.7%), cationic lipid (50.5%), phospholipid (8.9%), cholesterol (40.0%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (0.7%), the cationic lipid is 1,2-dilinoleyloxy-N,N-dimethylaminopropane (DLinDMA) (50.5%), the phospholipid is DPPC (8.9%), and cholesterol is present at 40.0%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation T, which comprises at least one oligomeric nucleotide described herein (e.g., siRNA).

Exemplary lipid formulation U includes the following components (wherein the percentage values of the components are mole percent): PEG-lipid (1.0%), cationic lipid (51.4%), phospholipid (15.0%), cholesterol (32.6%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (1.0%), the cationic lipid is 1,2-dilinoleyloxy-N,N-dimethylaminopropane (DLinDMA) (51.4%), the phospholipid is DSPC (15.0%), and cholesterol is present at 32.6%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation U, which comprises at least one oligomeric nucleotide described herein (e.g., siRNA).

Exemplary lipid formulation V includes the following components (wherein the percentage values of the components are mole percent): PEG-lipid (1.3%), cationic lipid (60.0%), phospholipid (7.2%), cholesterol (31.5%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound (67)) (1.3%), the cationic lipid is 1,2-dilinolenyloxy-N,N-dimethylaminopropane (DLenDMA) (60.0%), the phospholipid is DSPC (7.2%), and cholesterol is present at 31.5%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation V, which comprises at least one oligomeric nucleotide described herein (e.g., siRNA).

Exemplary lipid formulation W includes the following components (wherein the percentage values of the components are mole percent): PEG-lipid (1.8%), cationic lipid (51.6%), phospholipid (8.4%), cholesterol (38.3%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (1.8%), the cationic lipid is 1,2-dilinoleyloxy-N,N-dimethylaminopropane (DLinDMA) (51.6%), the phospholipid is DSPC (8.4%), and cholesterol is present at 38.3%, wherein the actual amounts of the lipids present may vary by, *e*.*g*., ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation W, which comprises at least one oligomeric nucleotide described herein (e.g., siRNA).

Exemplary lipid formulation X includes the following components (wherein the percentage values of the components are mole percent): PEG-lipid (2.4%), cationic lipid (48.5%), phospholipid (10.0%), cholesterol (39.2%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.4%), the cationic lipid is 1,2-di-γ-linolenyloxy-N,N-dimethylaminopropane (γ-DLenDMA; Compound **(15))** (48.5%), the phospholipid is DPPC (10.0%), and cholesterol is present at 39.2%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation X, which comprises at least one oligomeric nucleotide described herein (e.g., siRNA).

Exemplary lipid formulation Y includes the following components (wherein the percentage values of the components are mole percent): PEG-lipid (2.6%), cationic lipid (61.2%), phospholipid (7.1%), cholesterol (29.2%), wherein the actual amounts of the lipids present may vary by, *e.g.,* ± *5* % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DMA (compound **(66))** (2.6%), the cationic lipid is (6Z,16Z)-12-((Z)-dec-4-enyl)docosa-6,16-dien-11-yl 5-(dimethylamino)pentanoate (Compound **(13))** (61.2%), the phospholipid is DSPC (7.1%), and cholesterol is present at 29.2%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation Y, which comprises at least one oligomeric nucleotide described herein (e.g., siRNA).

Exemplary lipid formulation Z includes the following components (wherein the percentage values of the components are mole percent): PEG-lipid (2.2%), cationic lipid (49.7%), phospholipid (12.1%), cholesterol (36.0%), wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). For example, in one representative embodiment, the PEG-lipid is PEG-C-DOMG (compound **(67))** (2.2%), the cationic lipid is (6Z,9Z,28Z,31Z)-heptatriaconta-6,9,28,31-tetraen-19-yl 4-(dimethylamino)butanoate) (Compound (7)) (49.7%), the phospholipid is DPPC (12.1%), and cholesterol is present at 36.0%, wherein the actual amounts of the lipids present may vary by, *e.g.,* ± 5 % (or *e.g.,* ± 4 mol %, ± 3 mol %, ± 2 mol %, ± 1 mol %, ± 0.75 mol %, ± 0.5 mol %, ± 0.25 mol %, or ± 0.1 mol %). Thus, certain embodiments of the invention provide a nucleic acid-lipid particle based on formulation Z, which comprises at least one oligomeric nucleotide described herein (e.g., siRNA).

Accordingly, certain embodiments of the invention provide a nucleic acid-lipid particle described herein, wherein the lipids are formulated as described in any one of formulations A, B, C, D, E, F, G, H, I, J, K, L, M, N, O, P, Q, R, S, T, U, V, W, X, Y or Z.

The present invention also provides pharmaceutical compositions comprising a nucleic acid-lipid particle and a pharmaceutically acceptable carrier.

The nucleic acid-lipid particles of the present invention are useful, for example, for the therapeutic delivery of siRNAs that silence the expression of one or more HBV genes. In some embodiments, a cocktail of siRNAs that target different regions (*e*.*g*., overlapping and/or nonoverlapping sequences) of an HBV gene or transcript is formulated into the same or different nucleic acid-lipid particles, and the particles are administered to a mammal (*e.g*., a human) requiring such treatment. In certain instances, a therapeutically effective amount of the nucleic acid-lipid particles can be administered to the mammal, *e*.*g*., for treating HBV infection in a human.

In certain embodiments, one or more oligomeric nucleotides (e.g., siRNA molecules) may be introduced into a cell by contacting the cell with a nucleic acid-lipid particle described herein.

In certain embodiments, one or more siRNA molecules that silence expression of a Hepatitis B virus gene may be introduced into a cell by contacting the cell with a nucleic acid-lipid particle described herein under conditions whereby the siRNA enters the cell and silences the expression of the Hepatitis B virus gene within the cell. In certain embodiments, the cell is in a mammal, such as a human. In certain embodiments, the human has been diagnosed with a Hepatitis B virus infection. In certain embodiments, silencing of the Hepatitis B virus gene expression reduces Hepatitis B virus particle load in the mammal by at least about 50% (*e.g.,* about 55, 60, 65, 70, 75, 80, 85, 90, 95, 96, 97, 98, 99 or 100%) relative to Hepatitis B virus particle load in the absence of the nucleic acid-lipid particle.

In some embodiments, the nucleic acid-lipid particles or compositions (e.g., a pharmaceutical composition) described herein are administered by one of the following routes of administration: oral, intranasal, intravenous, intraperitoneal, intramuscular, intra-articular, intralesional, intratracheal, subcutaneous, and intradermal. In particular embodiments, the nucleic acid-lipid particles are administered systemically, *e*.*g*., via enteral or parenteral routes of administration.

### Examples

The present invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

The following methods were used in Examples 1-5 detailed below.

### Program Death 1 (PD-1) Study Goal.

To determine whether PD-1, checkpoint inhibitor breaks the tolerance in T cells induced by chronic HBV infection, a human anti-PD-1 monoclonal antibody (mAb) was evaluated using peripheral blood mononuclear cells (PBMC) from chronic Hepatitis B subjects and control healthy normal subjects in the presence or absence of Hepatitis B virus (HBV)-specific 15-mer overlapping peptides.

### Human Subjects Recruitment.

The chronic Hepatitis B subjects were more than 6 months after first HBV infection, HBsAg-positive and 18 years or older without evidence for hepatic decompensation, hepatocellular carcinoma, and liver transplant. All participants provided informed consent for the study and did not have the simultaneous presence of two chronic diseases or conditions that may cause serious health issue from blood draw. HBV-uninfected healthy subjects were used, which were without any known liver diseases and had tested negative for HBV, HCV and HIV. All participating subjects in the study had a 50ml blood draw, which was collected in EDTA-coated tubes, shipped overnight in ambient temperature to Arbutus Biopharma and processed within 24 hours.

### Anti-PD-1 antibody.

The anti-PD-1 antibody used in the experiments was a human anti-PD-1 monoclonal antibody (EH12.2H7), which was Mouse IgG1, κ isotype, commercially available from BioLegend (CA, USA). It has Cross-Reactivity with Chimpanzee, Common Marmoset, Rhesus, Squirrel Monkey, and Cynomolgus. It was purified by affinity chromatography and prepared as a Low Endotoxin, Azide-Free antibody.

### Viral Peptides and Controls.

The unique genotype-specific 15-mer overlapping peptides from HBV genotypes A, B, C, and D were synthesized from the conserved peptide sequences of HBV S, Core or Polymerase. In the same method, 15-mer overlapping peptides covering the matrix M1 protein of H1N1 virus were generated and used as a positive control. An additional control included a human anti-CD3 antibody for general T cell stimulation.

### Hepatitis B virus 15-mer overlapping peptides Generation Protocol.

The conserved peptide sequences were determined by aligning three published HBV sequences of each HBV genotype and choosing each sequence that are common among three sequences. The GenBank accession numbers of these genotypes are as follows:
- Genotype A (AF297624, AF536524, AF537372)
- Genotype B (AB033554, D50521, X97850)
- Genotype C (AB033553, AF241407, X04615)
- Genotype D (X02496, X72702, X85254)

These conserved sequences were used for synthesizing 15-mer overlapping peptides by 6 peptides offset. The number of 15-mer overlapping peptides that were generated are summarized in Table 1.

**Table 1. The numbers of unique 15-mer overlapping peptides of Hepatitis B genotype A, B, C, and D that are used for stimulation of HBV-specific T cells.**

| **HBV Region** | **HBV genotype** | | | | **Unique peptides pool** |
|---|---|---|---|---|---|
| | **A** | **B** | **C** | **D** | |
| preS | 29 | 29 | 29 | 29 | 90 |
| S | 35 | 35 | 35 | 35 | 92 |
| preC | 4 | 4 | 4 | 4 | 7 |
| Core | 31 | 31 | 31 | 31 | 61 |
| RT1 | 28 | 28 | 28 | 28 | 77 |
| RT2 | 28 | 28 | 28 | 28 | 59 |

### T cell proliferation by Flowcytometry analysis of cell division using carboxyfluorescein diacetate succinimidyl ester (CFSE) Experimental Protocol.

PBMC were isolated by density gradient centrifugation using Ficoll-Paque at 2200 rpm for 20 minutes at room temperature following layering blood on to Ficoll-Paque. The buffy coat (middle layer containing PBMC) was isolated to remove red blood cells. To remove platelets after density gradient separation, the cell pellet was re-suspended in RPMI and centrifuged at 1800 rpm for 8 minutes at room temperature. The red blood cells were further removed by 5 minutes incubation with ACK lysis buffer. PBMC were washed and re-suspended in 10% ABS or 10% FBS in RPMI following centrifugation at 1200 rpm for 5 minutes. PBMC (50x10⁶ cells/ml) were then re-suspended in RPMI medium and stained by 5µM CFSE as a final concentration. Specifically, the cells were mixed gently but quickly 4-5 times with a pipette; however, vigorous pipetting and bubbles were avoided. The tube was then covered with foil and incubated for 10 min at 37 °C in a water bath. 5 times of ice-cold 10% ABS or 10% FBS in RPMI medium was added and then put in ice for 5 minutes. After 5 min on ice, the tube was centrifuged at 1600 rpm for 5 minutes and the supernatant was aspirated. The cell pellet was then re-suspended with 2 ml of 10% ABS or 10% FBS in RPMI medium. The CFSE-stained cells were transferred at concentration of 0.2x10⁶ cells per well into a 96 well plate for incubation at 37 °C in a CO₂ incubator for 5 or 7 days. The CSFE-stained sample was taken on day 0 to determine the initial labeling. On Day 5 or 7, PBMC were stained with human α-CD3, α-CD4 and α-CD8 antibodies. The stained samples were acquired by LSRFortessa (BD Biosciences, San Jose, CA) and analyzed with FlowJo (Tree Star Inc., San Carlos, CA).

### Allogeneic presentation of foreign antigens Experimental Protocol.

Fresh healthy normal PBMC were plated in 10% FBS in RPMI medium at a concentration of 5x10⁶/ml in a 175 cm² cell culture flask for 30 minutes at 37 °C in a CO₂ incubator. The floating PBMC were removed and the flask was washed once with 20ml of RPMI. The PBMC that were stuck to the flask were cultured with 20ml 10% FBS in RPMI in the presence of the final concentration of 100U/ml GM-CSF and 500U/ml IL-4 for 7 days. Half of the culture medium was replaced with another half of fresh medium containing IL-4 and GM-CSF at 3 or 4 days from the initial culture. These cells were matured monocytes-derived Dendritic Cells. Additionally, CD4+ T cells were isolated from fresh PBMC from another donor using a CD4+ T cell isolation kit from Miltenyi Biotec and were re-suspended at a concentration of 40 µL of buffer per 10⁷ total cells. 10 µL of CD4+ T Cell Biotin-Antibody cocktail per 10⁷ total cells was added. It was mixed well and incubated for 5 minutes in the refrigerator (2-8 °C). 30 µL of buffer per 10⁷ total cells was added. 20 µL of CD4+ T Cell MicroBead Cocktail per 10⁷ cells then added. It was mixed well and incubated for an additional 10 minutes in the refrigerator. The column was then placed in the magnetic field of a suitable MACS Separator. The column was prepared by rinsing with 3 mL of buffer. The cell suspension was applied onto the column; the column was removed from the separator; and then placed on the collection tube. 5 mL of buffer was pipetted onto the column. The magnetically labeled CD4+ T cells were immediately flushed by pushing the plunger into the new collection tube. CD4+ T cells were stained with CFSE and a co-culture of the CFSE-stained CD4+ T cells (1x10⁵) and monocytes-derived DC (0.5x10⁴) per well was set up in a round 96 well plate in the presence of IgG, or α-PD-1 mAb for 5 days. The cells were stained with human α-CD3, α-CD4, and α-CD8; were acquired with LSRFortessa (BD Biosciences, San Jose, CA) and analyzed with FlowJo (Tree Star Inc., San Carlos, CA).

### Hepatitis B virus-specific T cell Proliferation Experimental Protocol.

Fresh PBMC from chronic Hepatitis B subjects were stained with CFSE according to the protocol that was mentioned above. 0.2x10⁶ CFSE-stained cells were added per well into the 96 well plate in the presence of medium, 15-mer overlapping peptides of HBV regions including S (pool of 92 unique peptides from 4 HBV genotype), Core (61), preS (92), preC (7), RT1 (77) and RT2 (59). Positive controls included 15-mer overlapping peptides of Influenza Matrix and anti-CD3 antibody. These PBMC were also co-cultured in the presence of Isotype control IgG or α-PD-1 mAb for 5 days.

### IFN-γ Cytokine Production Experimental Protocol.

IFN-γ production stimulation was analyzed by ELISA (BioLegend) assay. 0.2x10⁶ PBMC were stimulated with 15-mer overlapping peptides of HBV region or Influenza Virus Matrix. All reagents were brought to room temperature prior to use. The plate was washed 4 times with at least 300 µL of 1X Wash Buffer per well and any residual buffer was blotted by firmly tapping the plate upside down on absorbent paper. 50 µL of Assay Buffer A was added to each well that contained either standard dilutions or samples. 50 µL of standard dilutions or samples were added to the appropriate wells. The plate was incubated at room temperature for 2 hours while shaking at 200 rpm. The contents of the plate were discarded into a sink, then the plate was washed 4 times with Wash Buffer. 100 µL of Human IFN-γ Detection Antibody solution was added to each well and incubated at room temperature for 1 hour while shaking. The contents of the plate were discarded into a sink and then the plate was washed 4 times with Wash Buffer. 100 µL of Avidin-HRP solution was added to each well; the plate was sealed; and incubated at room temperature for 30 minutes while shaking. The contents of the plate were discarded into a sink and the plate was then washed 5 times with 1X Wash Buffer. 100 µL of Substrate Solution was added to each well and incubated for 15 minutes in the dark. Wells that contained human IFN-γ turned blue in color; the intensity of the color was proportional to its concentration. The reaction was stopped by adding 100 µL of Stop Solution to each well. The absorbance was read at 450 nm within 30 minutes.

### Example 1: Allogeneic co-culture of CD4+ T cells and monocytes-derived Dendritic Cells.

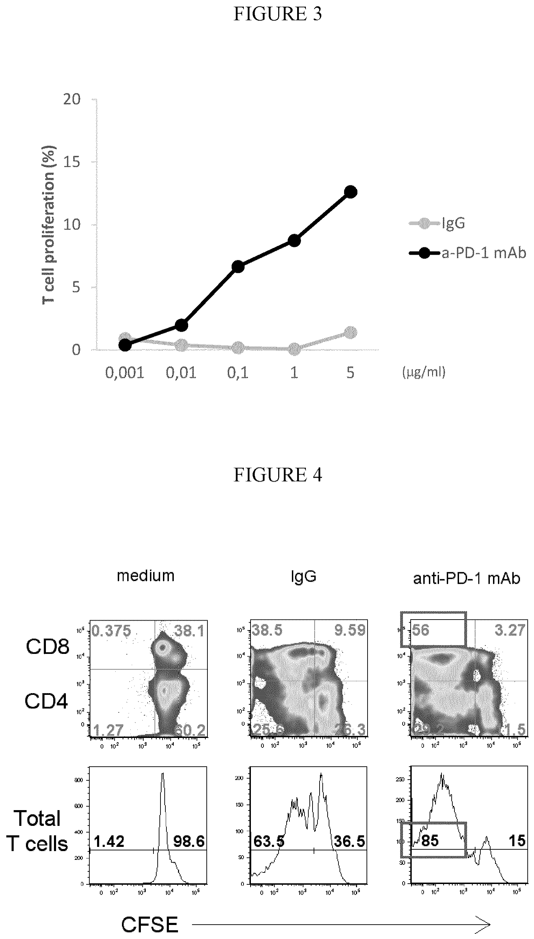

CD4+ T cells isolated from 3 healthy donors were labeled with CFSE and were co-cultured for 5 days with monocytes-derived DC from a different donor in the presence of titrated concentration (0, 0.001, 0.01, 0.1, 1, 5 µg/ml) of α-PD-1 mAb or control Isotype IgG. The T cell proliferation were subtracted from one at no antibody treatment background and shown in Figure 3 and Table 2. In this assay system, the α-PD-1 mAb enhanced T cell proliferation compared to Isotype control IgG.

**Table 2. T cells proliferation (%) relative to background up on allogeneic co-culture.**

| Conc. (mg/ml) | Isotype IgG | anti-PD-1 mAb |
|---|---|---|
| 0.001 | 0.9 | 0.4 |
| 0.01 | 0.4 | 2.0 |
| 0.1 | 0.2 | 6.6 |
| 1 | 0.1 | 8.7 |
| 5 | 1.4 | 12.6 |

### Example 2: General T cell proliferation in chronic Hepatitis B patients in the presence and absence of anti-PD-1 mAb.

Whole PBMC from a chronic Hepatitis B subject were labeled with CFSE and were stimulated with 0.1 µg/ml α-CD3 antibody with 10 U of human recombinant IL-2 in the presence of α-PD-1 mAb or control isotype IgG at concentration of 5 µg/ml for 5 days. The plots in Figure 4 were gated on CD3+ T live lymphocytes while the top left quadrant shows CD8+ T cell proliferation and the bottom left as CD4+ T cell proliferation. The left gate on histograms in the bottom indicates total CD3+ T cell proliferation. These proliferation frequencies of total CD3+ T, CD4+ T, and CD8+ T cells are shown in Figure 4 and Table 3. In this assay system, the α-PD-1 mAb enhanced CD3+ T, CD4+ T, and CD8+ T cell proliferation whereas control IgG did not increased T cell proliferation.

**Table 3. The proliferation of general T cells marked as CFSE-/CD3+, CFSE-/CD4+, and CFSE-/CD8+ T cells (%) in a chronic HBV patient.**

| | Medium | Control IgG | Anti-PD-1 mAb |
|---|---|---|---|
| CD3+ T cells(%) | 1.4 | 63.5 | 85 |
| CD8+ T cells (%) | 0.4 | 38.5 | 56 |
| CD4+ T cells (%) | 1.3 | 25.6 | 29.2 |

### Example 3: The comparison of general T cell division in chronic Hepatitis B patients in the presence and absence of anti-PD-1 mAb.

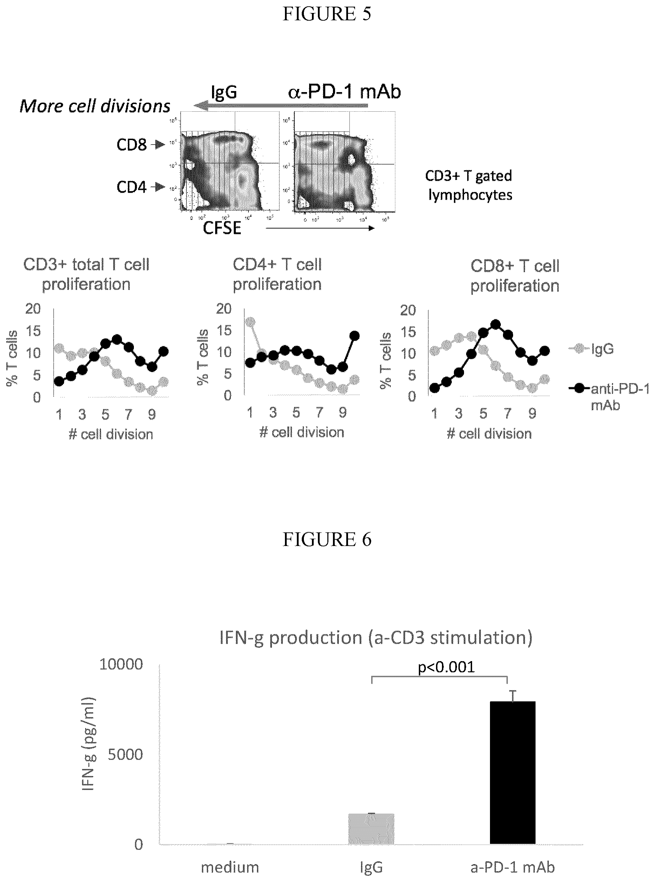

This assay was done as in Example 2 and compares the T cell divisions between α-PD-1 mAb and isotype control IgG. All top left and bottom left in the plot as proliferation was sliced into each cell division while leftward direction indicates more cell division (Figure 5). Total CD3+ T cells, CD4+ T cells, and CD8+ T cells were analyzed as shown in the graph in Figure 5 and Table 4 based on cell division number. In this analysis, α-PD-1 mAb stimulates the proliferation of total CD3+ T cells, CD4+ T cells, and CD8+ T cells more compared with that of isotype control IgG.

**Tables 4A-C. The cell division numbers of general T cell from chronic HBV patient up on treatment with α-PD-1 mAb or control IgG.**

**Table 4A. CD3 total T cell proliferation**

| Antibody | Cell division | | | | | | | | | |
|---|---|---|---|---|---|---|---|---|---|---|
| | #1 | #2 | #3 | #4 | #5 | #6 | #7 | #8 | #9 | #10 |
| IgG | 11 | 9.22 | 9.94 | 10.1 | 8.13 | 5.3 | 3.39 | 2.17 | 1.48 | 3.4 |
| anti-PD-1 mAb | 3.53 | 4.73 | 6.16 | 9.2 | 12.1 | 13 | 11.2 | 8.13 | 6.8 | 10.3 |

**Table 4B. CD4+ T cell proliferation**

| Antibody | Cell division | | | | | | | | | |
|---|---|---|---|---|---|---|---|---|---|---|
| | #1 | #2 | #3 | #4 | #5 | #6 | #7 | #8 | #9 | #10 |
| IgG | 16.9 | 9.6 | 8.11 | 6.84 | 5.74 | 3.83 | 2.74 | 2.01 | 1.39 | 3.47 |
| anti-PD-1 mAb | 7.43 | 8.73 | 9.09 | 10.3 | 10.2 | 9.51 | 7.91 | 5.94 | 6.47 | 13.6 |

**Table 4C. CD8+ T cell proliferation**

| Antibody | Cell division | | | | | | | | | |
|---|---|---|---|---|---|---|---|---|---|---|
| | #1 | #2 | #3 | #4 | #5 | #6 | #7 | #8 | #9 | #10 |
| IgG | 10.5 | 11.8 | 13.5 | 13.8 | 10.7 | 7.04 | 4.41 | 2.65 | 1.92 | 3.97 |
| anti-PD-1 mAb | 1.92 | 3.32 | 5.51 | 9.78 | 14.6 | 16.6 | 14.2 | 10.1 | 8.16 | 10.5 |

### Example 4: The IFN-rproduction by general T cell in chronic Hepatitis B patients in the presence and absence of anti-PD-1 mAb.

Whole PBMC from chronic Hepatitis B donors were stimulated with 0.1 µg/ml α-CD3 antibody, with α-PD-1 mAb or control isotype IgG at a concentration of 5 µg/ml for 5 days. The supernatants were collected from the culture and IFN-γ production was measured by ELISA (Figure 6 and Table 5). In this assay system, the anti-PD-1 mAb stimulated IFN-γ production more than Isotype control IgG.

**Table 5. Anti-PD-1 clones enhances IFN-γ production from general T cells in chronic HBV patients**

| | IFN-g (pg/ml) |
|---|---|
| medium | 14.4 |
| IgG | 1685.0 |
| a-PD-1 mAb | 7910.6 |

| | |
|---|---|
| *p value between IgG and a-PD-1 mAb is <0.001 | |

### Example 5: The HBV-specific T cell proliferation in chronic Hepatitis B patients in the presence and absence of anti-PD-1 mAb.

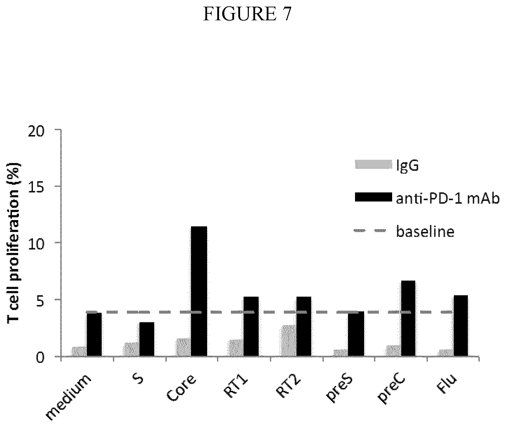

Whole PBMC from chronic Hepatitis B donors were labeled with CFSE and were stimulated with 15-mer overlapping peptides of HBV regions including S, Core, RT1, RT2, preS, preC; influenza at 1 µM of final concentration. These cells were co-cultured with a-PD-1 mAb or control isotype IgG at a concentration of 5 µg/ml for 5 days. In this assay system, the anti-PD-1 mAb enhanced the proliferation of T cells in response to HBV peptides including S, Core, RT1, RT2, preC as well as influenza peptides (Figure 7 and Table 6).

**Table 6. T cell proliferation (%) in response to HBV-specific 15-mer overlapping peptides and positive control Influenza 15-mer overlapping peptides**

| | **IgG** | **anti-PD-1 mAb** |
|---|---|---|
| medium | 0.906 | 3.88 |
| S | 1.18 | 3.08 |
| Core | 1.64 | 11.5 |
| RT1 | 1.54 | 5.27 |
| RT2 | 2.75 | 5.25 |
| preS | 0.536 | 4.01 |
| preC | 0.993 | 6.72 |
| Flu | 0.591 | 5.41 |

All publications, patents, and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

The present invention relates to the following items:
1. A method for ameliorating one or more symptoms associated with Hepatitis B virus infection in a mammal, the method comprising the step of administering to the mammal a therapeutically effective amount of an anti-PD-1 antibody, or fragment thereof.
2. A method for treating a Hepatitis B virus infection in mammal, the method comprising the step of administering to the mammal a therapeutically effective amount of an anti-PD-1 antibody, or fragment thereof.
3. The method of item 1 or 2, wherein the anti-PD-1 antibody, or fragment thereof, specifically binds to PD-1 and modulates its activity.
4. The method of any one of items 1-3, wherein a full-length anti-PD-1 antibody is administered.
5. The method of any one of items 1-3, wherein a fragment of an anti-PD-1 antibody is administered.
6. The method of item 5, wherein the fragment is a Fab fragment, a F(ab')2 fragment, a Fd fragment, a Fv fragment, a single-chain Fv (scFv) molecule or a dAb fragment.
7. The method of any one of items 1-6, wherein the anti-PD-1 antibody, or a fragment thereof, is a monoclonal antibody, or a fragment thereof.
8. The method of any one of items 1-7, wherein the anti-PD-1 antibody, or a fragment thereof, is a human antibody, or a fragment thereof.
9. The method of any one of items 1-8, wherein the anti-PD-1 antibody, or a fragment thereof, is a blocking antibody, or a fragment thereof, or a neutralizing antibody, a fragment thereof.
10. The method of item 1 or 2, wherein the anti-PD-1 antibody is Nivolumab, Pembrolizumab, TSR-042, REGN2810 or EH12.2H7.
11. The method of any one of items 1-10, wherein the anti-PD-1 antibody, or fragment thereof, is administered via systemic route.
12. The method of any one of items 1-10, wherein the anti-PD-1 antibody, or fragment thereof, is administered orally.
13. The method of any one of items 1-12, wherein the Hepatitis B virus particle load in the mammal is reduced by at least about 50% relative to Hepatitis B virus particle load in the absence of the anti-PD-1 antibody, or fragment thereof.
14. The method of any one of items 1-13, wherein the mammal is human.
15. The method of any one of items 1-14, further comprising administering at least one additional therapeutic agent.
16. The method of item 15, wherein the at least one additional therapeutic agent is selected from the group consisting of:
   a) reverse transcriptase inhibitors;
   b) capsid inhibitors;
   c) cccDNA formation inhibitors;
   d) sAg secretion inhibitors;
   e) oligomeric nucleotides targeted to the Hepatitis B genome; and
   f) immunostimulators.
17. The method of item 16, wherein at least one reverse transcriptase inhibitor is administered to the mammal.
18. The method of item 17, wherein the reverse transcriptase inhibitor is selected from the group consisting of lamivudine, adefovir, entecavir, telbivudine, and tenofovir.
19. The method of item 16, wherein at least one capsid inhibitor is administered to the mammal.
20. The method of item 19, wherein the capsid inhibitor is selected from the group consisting of Bay-41-4109, AT-61, DVR-01, and DVR-23f.
21. The method of item 16, wherein at least one cccDNA formation inhibitor is administered to the mammal.
22. The method of item 21, wherein the cccDNA formation inhibitor is selected from CCC-0975 and CCC-0346.
23. The method of item 16, wherein at least one sAg secretion inhibitor is administered to the mammal.
24. The method of item 23, wherein the sAg secretion inhibitor is selected from the group consisting of PBHBV-001 and PBHBV-2-15.
25. The method item 16, wherein at least one oligomeric nucleotide targeted to the Hepatitis B genome is administered to the mammal.
26. The method of item 25, wherein the at least one oligomeric nucleotide is an isolated, double stranded siRNA molecule.
27. The method of item 16, wherein at least one immunostimulator is administered to the mammal.
28. The method of item 27, wherein the immunostimulator is selected from the group consisting of agonists of stimulator of IFN genes (STING) and interleukins.
29. The method of any one of items 16-28, wherein the at least one additional therapeutic agent is administered orally.
30. The method of any one of items 16-28, wherein the at least one additional therapeutic agent is administered intravenously.
31. The method of item 25 or 26, wherein the at least one oligomeric nucleotide is administered via a nucleic acid-lipid particle, wherein the nucleic acid-lipid particle comprises:
   a) the at least one oligomeric nucleotide;
   b) a cationic lipid; and
   c) a non-cationic lipid.
32. The method of item 31, wherein the cationic lipid is selected from the group consisting of 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (γ-DLenDMA; Compound **(15)),** 3-((6Z,9Z,28Z,31Z)-heptatriaconta-6,9,28,31-tetraen-19-yloxy)-N,N-dimethylpropan-1-amine (DLin-MP-DMA; Compound **(8)),** (6Z,9Z,28Z,31Z)-heptatriaconta-6,9,28,31-tetraen-19-yl 4-(dimethylamino)butanoate) (Compound **(7)),** (6Z,16Z)-12-((Z)-dec-4-enyl)docosa-6,16-dien-11-yl 5-(dimethylamino)pentanoate (Compound **(13)),** a salt thereof, and a mixture thereof.
33. The method of item 31 or 32, wherein the non-cationic lipid is cholesterol or a derivative thereof.
34. The method of item 31 or 32, wherein the non-cationic lipid is a phospholipid.
35. The method of item 31 or 32, wherein the non-cationic lipid is a mixture of a phospholipid and cholesterol or a derivative thereof.
36. The method of item 34 or 35, wherein the phospholipid is selected from the group consisting of dipalmitoylphosphatidylcholine (DPPC), distearoylphosphatidylcholine (DSPC), and a mixture thereof.
37. The method of any one of items 31-36, further comprising a conjugated lipid that inhibits aggregation of particles.
38. The method of item 37, wherein the conjugated lipid that inhibits aggregation of particles is a polyethyleneglycol (PEG)-lipid conjugate.
39. The method of item 38, wherein the PEG-lipid conjugate is selected from the group consisting of a PEG-diacylglycerol (PEG-DAG) conjugate, a PEG-dialkyloxypropyl (PEG-DAA) conjugate, a PEG-phospholipid conjugate, a PEG-ceramide (PEG-Cer) conjugate, and a mixture thereof.
40. The method of item 39, wherein the PEG-lipid conjugate is a PEG-DAA conjugate.
41. The method of item 40, wherein the PEG-DAA conjugate is selected from the group consisting of a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, a PEG-distearyloxypropyl (C₁₃) conjugate, and a mixture thereof.
42. The method of any one of items 31-41, wherein the at least one oligomeric nucleotide is fully encapsulated in the particle.
43. The method of any one of items 31-42, wherein the particle has a total lipid:oligomeric nucleotide mass ratio of from about 5:1 to about 15:1.
44. The method of any one of items 31-43, wherein the particle has a median diameter of from about 30 nm to about 150 nm.
45. The method of any one of items 31-44, wherein the particle has an electron dense core.
46. The method of any one of items 31-45, wherein the cationic lipid comprises from about 48 mol % to about 62 mol % of the total lipid present in the particle.
47. The method of any one of items 35-46, comprising a phospholipid and cholesterol or cholesterol derivative, wherein the phospholipid comprises from about 7 mol % to about 17 mol % of the total lipid present in the particle and the cholesterol or derivative thereof comprises from about 25 mol % to about 40 mol % of the total lipid present in the particle.
48. The method of any one of items 37-47, wherein the conjugated lipid that inhibits aggregation of particles comprises from about 0.5 mol % to about 3 mol % of the total lipid present in the particle.
49. The method of any one of items 46-48, wherein the lipids are formulated as described in any one of formulations A, B, C, D, E, F, G, H, I, J, K, L, M, N, O, P, Q, R, S, T, U, V, W, X, Y or Z.
50. The method of any one of items 16-49, wherein the anti-PD-1 antibody, or fragment thereof, and the at least one additional therapeutic agent are administered separately.
51. The method of any one of items 16-50, wherein the anti-PD-1 antibody, or fragment thereof, and the at least one additional therapeutic agent are administered sequentially.
52. The method of any one of items 16-50, wherein the anti-PD-1 antibody, or fragment thereof, and the at least one additional therapeutic agent are administered simultaneously.
53. A kit comprising:
   (a) a pharmaceutical composition comprising an anti-PD-1 antibody, or a fragment thereof, and a pharmaceutically acceptable diluent or carrier;
   (b) a pharmaceutical composition comprising at least one additional therapeutic agent and a pharmaceutically acceptable diluent or carrier; and
   (c) instructions for the simultaneous, sequential or separate administration of the an anti-PD-1 antibody, or a fragment thereof, and the at least one additional therapeutic agent.
54. An anti-PD-1 antibody, or fragment thereof, for ameliorating one or more symptoms associated with Hepatitis B virus infection in a mammal.
55. The use of an anti-PD-1 antibody, or a fragment thereof, to prepare a medicament for ameliorating one or more symptoms associated with Hepatitis B virus infection in a mammal.
56. An anti-PD-1 antibody, or fragement thereof, for the prophylactic or therapeutic treatment of a Hepatitis B virus infection.
57. The use of an anti-PD-1 antibody, or fragment thereof, to prepare a medicament for the treatment of a Hepatitis B virus infection.
58. An anti-PD-1 antibody, or fragment thereof, for ameliorating one or more symptoms associated with Hepatitis B virus infection in a mammal, in combination with at least one additional therapeutic agent.
59. The use of an anti-PD-1 antibody, or a fragment thereof, to prepare a medicament for ameliorating one or more symptoms associated with Hepatitis B virus infection in a mammal, in combination with at least one additional therapeutic agent.
60. An anti-PD-1 antibody, or fragment thereof, for the prophylactic or therapeutic treatment of a Hepatitis B virus infection, in combination with at least one additional therapeutic agent.
61. The use of an anti-PD-1 antibody, or fragment thereof, to prepare a medicament for the treatment of a Hepatitis B virus infection, in combination with at least one additional therapeutic agent.
62. The antibody or use of any one of items 54-61, wherein the anti-PD-1 antibody, or fragment thereof, specifically binds to PD-1 and modulates its activity.
63. The antibody or use of any one of items 54-62, which is a full-length anti-PD-1 antibody.
64. The antibody or use of any one of items 54-62, which is a fragment of an anti-PD-1 antibody.
65. The antibody or use of item 64, wherein the fragment is a Fab fragment, a F(ab')2 fragment, a Fd fragment, a Fv fragment, a single-chain Fv (scFv) molecule or a dAb fragment.
66. The antibody or use of any one of items 54-65, wherein the anti-PD-1 antibody, or a fragment thereof, is a monoclonal antibody, or a fragment thereof.
67. The antibody or use of any one of items 54-66, wherein the anti-PD-1 antibody, or a fragment thereof, is a human antibody, or a fragment thereof.
68. The antibody or use of any one of items 54-61, wherein the anti-PD-1 antibody is Nivolumab, Pembrolizumab, TSR-042, REGN2810 or EH12.2H7.
69. The antibody or use of any one of items 58-68, wherein the at least one additional therapeutic agent is selected from the group consisting of:
   a) reverse transcriptase inhibitors;
   b) capsid inhibitors;
   c) cccDNA formation inhibitors;
   d) sAg secretion inhibitors;
   e) oligomeric nucleotides targeted to the Hepatitis B genome; and
   f) immunostimulators.
70. The antibody or use of item 69, wherein the at least one additional therapeutic agent is a reverse transcriptase inhibitor.
71. The antibody or use of item 70, wherein the reverse transcriptase inhibitor is selected from the group consisting of lamivudine, adefovir, entecavir, telbivudine, and tenofovir.
72. The antibody or use of item 69, wherein the at least one additional therapeutic agent is a capsid inhibitor.
73. The antibody or use of item 72, wherein the capsid inhibitor is selected from the group consisting of Bay-41-4109, AT-61, DVR-01, and DVR-23f.
74. The antibody or use of item 69, wherein the at least one additional therapeutic agent is a cccDNA formation inhibitor.
75. The antibody or use of item 74, wherein the cccDNA formation inhibitor is selected from CCC-0975 and CCC-0346.
76. The antibody or use of item 69, wherein the at least one additional therapeutic agent is a sAg secretion inhibitor.
77. The antibody or use of item 76, wherein the sAg secretion inhibitor is selected from the group consisting of PBHBV-001 and PBHBV-2-15.
78. The antibody or use of item 69, wherein the at least one additional therapeutic agent is a oligomeric nucleotide targeted to the Hepatitis B genome.
79. The antibody or use of item 78, wherein the oligomeric nucleotide is an isolated, double stranded siRNA molecule.
80. The antibody or use of item 69, wherein the at least one additional therapeutic agent is an immunostimulator.
81. The antibody or use of item 80, wherein the immunostimulator is selected from the group consisting of agonists of stimulator of IFN genes (STING) and interleukins.
82. The antibody or use of item 78 or 79, wherein the oligomeric nucleotide is formulated in a nucleic acid-lipid particle, wherein the nucleic acid-lipid particle comprises:
   a) at least one oligomeric nucleotide;
   b) a cationic lipid; and
   c) a non-cationic lipid.

## Claims

1. A combination comprising
(i) an anti-PD-1 antibody, or fragment thereof;
(ii) a reverse transcriptase inhibitor; and
(iii) an oligomeric nucleotide targeted to the Hepatitis B genome,
for use in ameliorating one or more symptoms associated with Hepatitis B virus infection in a mammal, or for use in the prophylactic or therapeutic treatment of a Hepatitis B virus infection.

2. The combination for use of claim 1, wherein the anti-PD-1 antibody fragment is a Fab fragment, a F(ab')2 fragment, a Fd fragment, a Fv fragment, a single-chain Fv (scFv) molecule or a dAb fragment.

3. The combination for use of claim 1 or claim 2, wherein the anti-PD-1 antibody, or a fragment thereof, is a monoclonal antibody, or a fragment thereof.

4. The combination for use of any one of claims 1 to 3, wherein the anti-PD-1 antibody, or a fragment thereof, is a human antibody, or a fragment thereof.

5. The combination for use of claim 1, wherein the anti-PD-1 antibody is Nivolumab, Pembrolizumab, TSR-042, REGN2810 or EG12.2H7.

6. The combination for use of claim 5, wherein the anti-PD-1 antibody is Nivolumab.

7. The combination for use of any one of claims 1 to 6, wherein the reverse transcriptase inhibitor is a nucleoside analog.

8. The combination for use of any one of claims 1 to 7, wherein the reverse transcriptase inhibitor is selected from the group consisting of lamivudine, adefovir, entecavir, telbivudine, and tenofovir.

9. The combination for use of any one of claims 1 to 7, wherein the reverse transcriptase inhibitor is selected from the group consisting of entecavir, tenofovir, tenofovir disoproxil, and tenofovir alafenamide.

10. The combination for use of any one of claims 1 to 9, wherein the oligomeric nucleotide targeted to the Hepatitis B genome is a double-stranded siRNA.

11. The combination for use of any one of claims 1 to 10, wherein the mammal is human.
